# EUROPEAN PATENT SPECIFICATION

(11) **EP 3 121 278 B1**
(45) Date of publication and mention of the grant of the patent: **18.07.2018**
(21) Application number: 16174066.7
(22) Date of filing: 02.05.2007
(51) Int. Cl.: C12N 15/113

(54) **SHORT INTERFERING RIBONUCLEIC ACID (SIRNA)**
KURZE INTERFERIERENDE RIBONUKLEINSÄURE (SIRNA)
ACIDE RIBONUCLÉIQUE INTERFÉRENT COURT (SIRNA)

(30) Priority: 04.05.2006 GB 0608838
(43) Date of publication of application: 25.01.2017
(62) Divisional of application: 14163854.4
(73) Proprietor: NOVARTIS AG, 4056 Basel (CH)
(72) Inventor: NATT, François Jean-Charles, 4002 Basel (CH); BILLY, Eric, 4002 Basel (CH); HUNZIKER, Juerg, 4002 Basel (CH); SCHNELL, Christian, 4002 Basel (CH)
(74) Representative: D Young & Co LLP

(56) References cited:
- US-A1- 2004 219 671
- EPA RUWAN W ET AL: "Downregulation of the p75 neurotrophin receptor in tissue culture and in vivo, using beta-cyclodextrin-adamantane-oligonucleoti de conjugates", ANTISENSE & NUCLEIC ACID DRUG DEVELOPMENT, MARY ANN LIEBERT, INC., NEW YORK, US, vol. 10, no. 6, 1 January 2000 (2000-01-01), pages 469-478, XP002967374, ISSN: 1087-2906
- MANOHARAN M ET AL: "Lipidic nucleic acids", TETRAHEDRON LETTERS, ELSEVIER, AMSTERDAM, NL, vol. 36, no. 21, 22 May 1995 (1995-05-22), pages 3651-3654, XP004028042, ISSN: 0040-4039, DOI: 10.1016/0040-4039(95)00597-6

## Description

### Background:

RNA interference initially discovered in plants as Post-Transcriptional Gene Silencing (PTGS), is a highly conserved mechanism triggered by double-stranded RNA (dsRNA) and able to down regulate transcript of genes homologous to the dsRNA¹. The dsRNA is first processed by Dicer into short duplexes of 21-23 nt, called short interfering RNAs (siRNAs)². Incorporated in RNA-induced silencing complex (RISC) they are able to mediate gene silencing through cleavage of the target mRNA in the center of the region of homology by Argonaute 2, a component of RISC³. In 2001, Elbashir et al⁴ demonstrated that the direct introduction of synthetic siRNAs would mediate RNA interference gene silencing in drosophila but also in mammalian cells. Since then, siRNA-mediated gene silencing has become a powerful and widely-used molecular biology tool in both target identification target validation studies. Use of siRNAs for gene silencing in animal studies has been described in a limited amount of animal models. Unmodified siRNAs were delivered locally in the eye⁵, intrathecally or intracerebellarly in the central nervous system⁶, and intranasally for the inhibition of respiratory viruses⁷. Intravenous hydrodynamic tail vein injection of unmodified siRNAs has also been studied. This approach allows a rapid delivery, mainly to the liver⁸. A very limited number of studies have been reported on the systemic administration of unmodified siRNAs. Duxbury et al⁹ administered intravenously unmodified siRNAs targeting Focal Adhesion Kinase to an orthotopic tumor xenograft mice model, and observed a tumor growth inhibition as well as a chemosensitization to gemcitabine. Soutscheck et al reported the systemic use of highly chemically modified siRNAs for the endogeneous silencing Apolipoprotein B. Intraperitoneal administration of most anti-ApoB siRNA at the high dose of 50 mg/kg reduced ApoB protein level and Lipoprotein concentration¹⁰. Despite these examples, *in vivo* use of siRNAs upon systemic delivery requires improvements in order to make this technology widely applicable for target validation or therapeutic applications. Indeed, unmodified siRNAs are subject to enzymatic digestion, mainly by nucleases abundant in the blood stream. In order to improve pharmacological properties of siRNAs several groups investigated chemical modification of these reagents. While the approaches described are very different among themselves and that no systematic study was yet performed, an overview of the results allows to determine the tolerance of siRNAs to chemical modifications. Several chemistries such as phosphorothioates¹¹ or boranophosphates¹², 2'-O-Methyl¹³, 2'-O-allyl¹⁴, 2'-methoxyethyl (MOE) and 2'-deoxyfluoronucleotides¹⁵ or Locked Nucleic Acids (LNA)¹⁶ have been investigated. These studies highlighted that tolerance for modification is not only chemistry-dependent, but also position-dependent.

The present invention provides a minimally modified siRNA with improved pharmacological properties. The minimally modified siRNAs are 19bp double-stranded RNA modified on the 3'-end of each strand in order to prevent 3'-exonuclease digestion: the 3'-dideoxynucleotide overhang of 21-nt siRNA has been replaced by a universal 3'-hydroxypropyl phosphodiester moiety and the modification of the two first base-pairing nucleotides on 3'-end of each strand further enhances serum stability. Applied intraperitoneally or orally to adult mice, the modified siRNAs displayed higher potency in a growth factor induce angiogenesis model which correlates with their increased serum stability.

### Summary:

In one aspect, the present invention provides a short interfering ribonucleic acid (siRNA) for oral administration, said siRNA comprising two separate RNA strands that are complementary to each other over at least 15 nucleotides, wherein each strand is 49 nucleotides or less, and wherein the 3'-terminus of at least one strand comprises a modification at the 3' carbon, wherein the modification is adamantane according to claim 1. In one embodiment, the siRNA comprises at least one modified nucleotide.

In another embodiment, said modified nucleotide is selected from among 2' alkoxyribonucleotide, 2' alkoxyalkoxy ribonucleotide, a locked nucleic acid ribonucleotide (LNA), 2'-fluoro ribonucleotide, morpholino nucleotide.

In another embodiment, said modified nucleotide is selected from among nucleotides having a modified internucleoside linkage selected from among phosphorothioate, phosphorodithioate, phosphoramidate, boranophosphonoate, and amide linkages.

In another embodiment, said two RNA strands are fully complementary to each other.

In another embodiment, said siRNA comprises a 1 to 6 nucleotide overhang on at least one of the 5' end or 3' end. The siRNA can contain at least one strand which is complementary over at least 15 nucleotides to the mRNA or pre-mRNA of VEGFR-1, VEGFR-2, VEGFR3, Tie2, bFGFR, IL8RA, IL8RB, Fas, or IGF2R. The siRNA can contain at least one strand which comprises a sequence selected from SEQ ID NO 1 - 900. The siRNA can be chosen from the group consisting of SEQ ID NO 901-930. The siRNA can have a stability in a standard gastric acid assay that is greater than an unmodified siRNA with the same nucleotide sequence. The siRNA can have a stability in a standard gastric acid assay that is greater than or equal to 50% after 30 minutes exposure. The siRNA can have a stability in a standard serum assay greater than unmodified siRNA. The siRNA can have a stability in a standard serum assay that is greater than or equal to 50% after 30 minutes exposure. The siRNA can have a stability in a standard intestinal lavage assay that is greater than unmodified siRNA. The siRNA can have an enhanced oral bioavailability compared to an unmodified siRNA of the same nucleotide sequence.

In one aspect, the invention provides a pharmaceutical composition comprising an siRNA with any one or more of the above properties.

In another aspect, the invention provides an siRNA with any one or more of the above properties for use as a medicament.

In another aspect, the invention provides the use of an siRNA with any one or more of the above properties in the preparation of a medicament for treating an angiogenic disorder.

In another aspect, the invention provides the use of an siRNA with any one or more of the above properties to inhibit an angiogenic process *in vitro.*

### Brief Description of the Drawings:

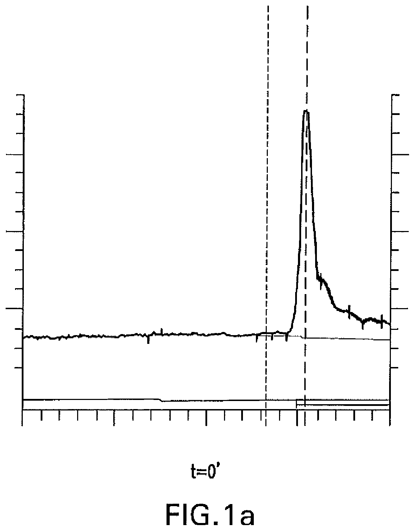

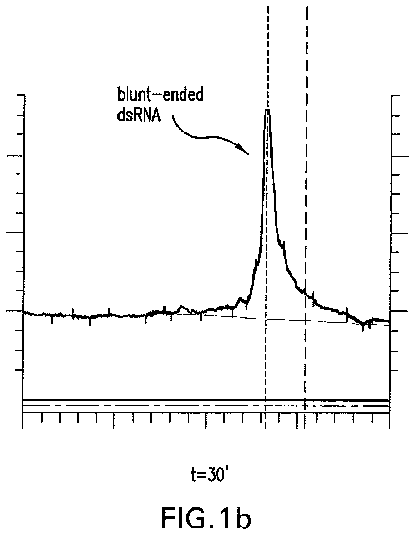

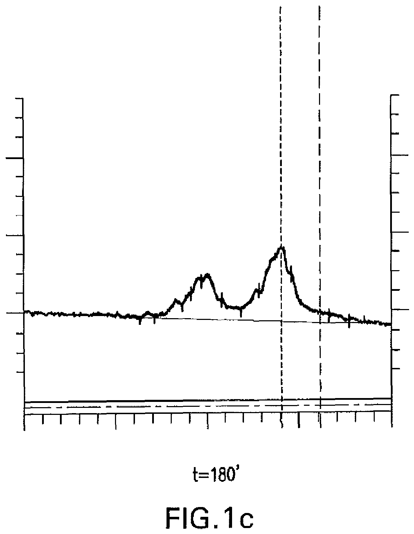

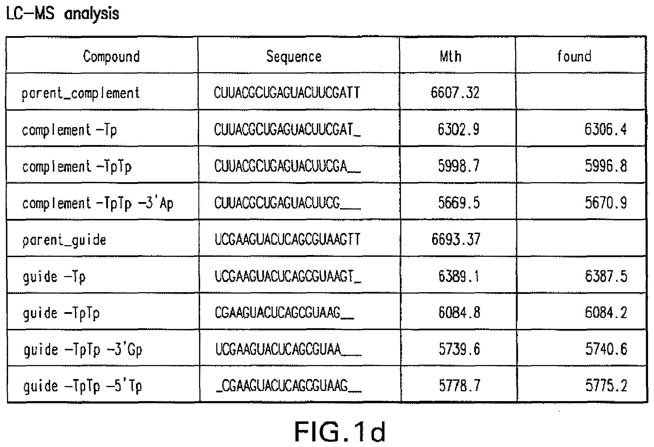

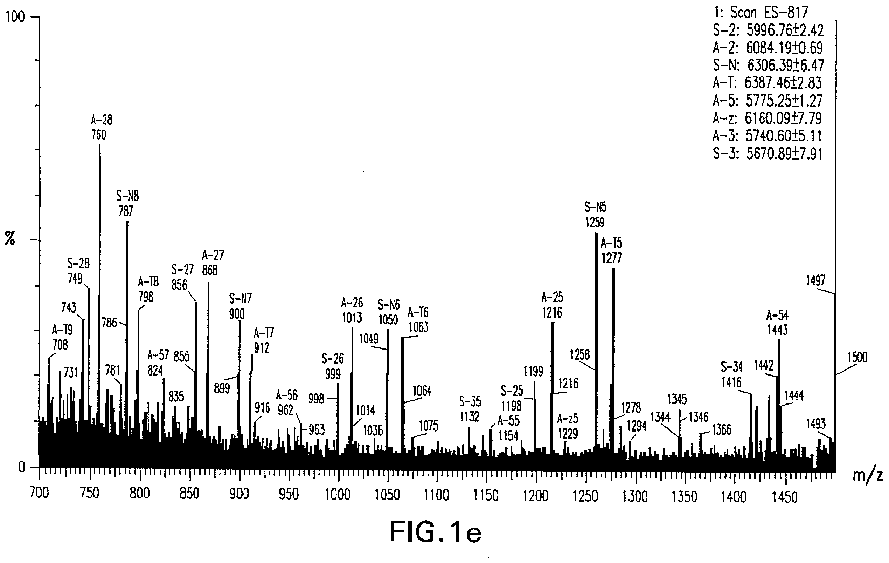

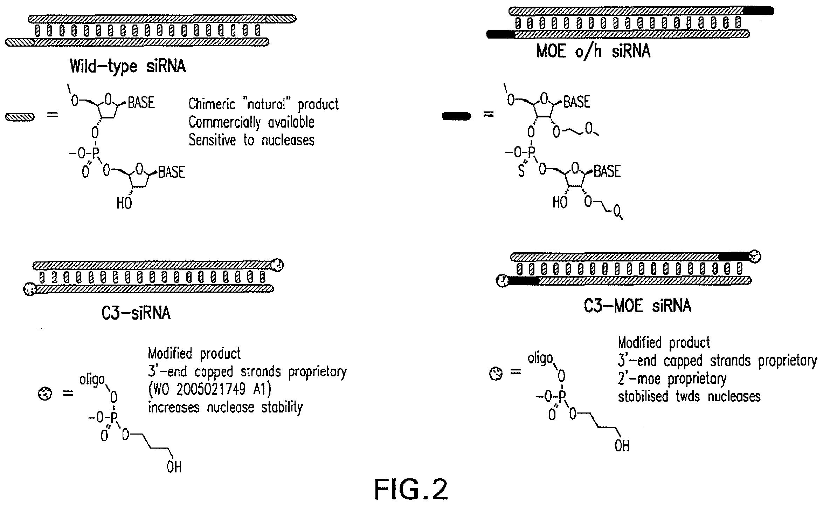

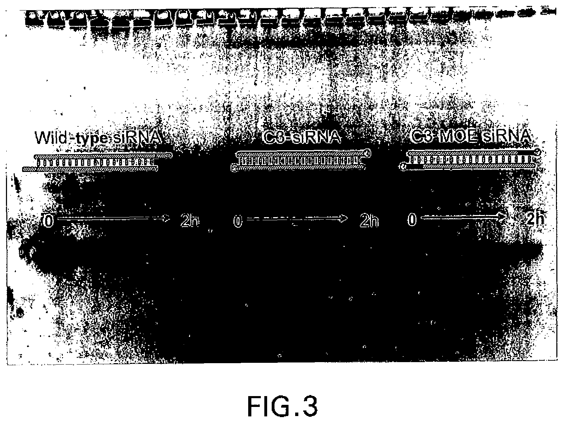

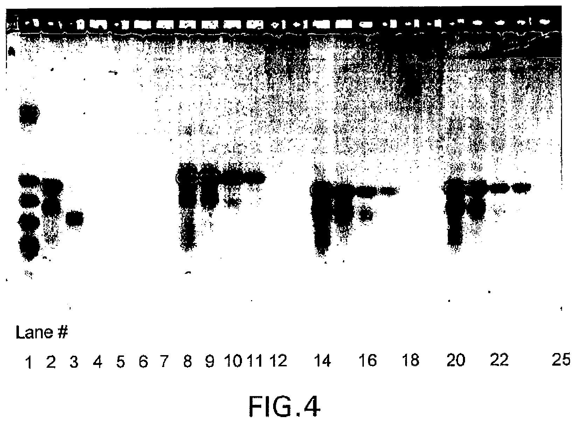

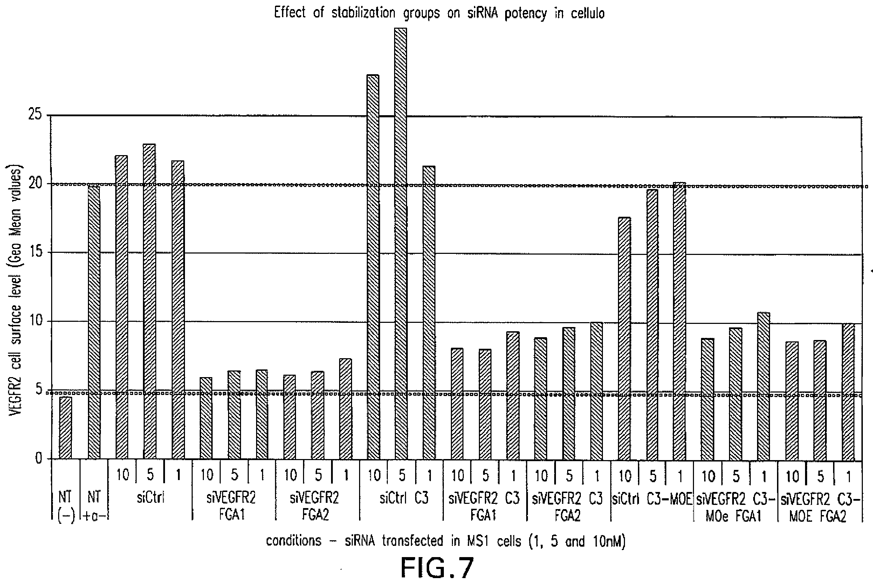

**Figure 1a****,** **1b****,** **1c****,** **1d** **and** **1e****:** Metabolic degradation of unmodified siRNA pG13-siRNA (wild-type siRNA in mouse serum); a-c) Ion Exchange-HPLC analysis of unmodified siRNAs after incubation in mouse serum for 0', 30' and 180'; After 30' of incubation at 37°C, major peak in the Ion Exchange HPLC was isolated and re-injected in LC-MS, d) table of detected molecular weights and their assignments; e) ESI-MS spectrum **Figure 2****:** illustration of four double-stranded RNA formats: wild-type (or unmodified) siRNA. MOE o/h siRNA, C3-siRNA and C3-MOE siRNA. **Figure 3****:** Stability of siRNA in 3 different formats in mouse gastric acid. Samples were incubated at 37°C in mouse gastric acid at a 2 micromolar concentration. Disappearance of parent compound was followed over a 2-6 hours period by quantifying the parent compound band.Lane 1-7: wild-type siRNA in gastric acid at t=0, 5, 10, 15, 30, 60 and 120 min
   Lane 8: ds RNA ladder (30, 21, 19, 16, 13, 10 bp)
   Lane 9-15: C3 siRNA in gastric acid at t=0, 5, 10, 15, 30, 60 and 120 min
   Lane 16: ds RNA ladder (30, 21, 19, 16, 13, 10 bp)
   Lane 17-24: C3-MOE siRNA in gastric acid at t=0, 5, 10, 15, 30, 60 and 120 min
**Figure 4****:** Stability of siRNA in 4 different formats in intestinal lavage. Samples were incubated at 37°C in liver microsomes at a 5 micromolar concentration.
   (From left to right)
   Lane 1: ds RNA ladder (30, 21, 19, 16, 13, 10 bp)
   Lane 2-7: wild-type siRNA in intestinal lavage at t=0, 15, 30, 60, 180 and 360 min
   Lane 8-13: moe o/h siRNA in intestinal lavage at t=0, 15, 30, 60, 180 and 360 min
   Lane 14-19: C3 siRNA in intestinal lavage at t=0, 15, 30, 60, 180 and 360 min
   Lane 20-25: C3-MOE siRNA in intestinal lavage at t=0, 15, 30, 60, 180 and 360 min
**Figure 5****:** Stability of siRNA in 4 different formats in liver microsomes. Samples were incubated at 37°C in intestinal fluid from rat intestinal lavage at a 2 micromolar concentration.
   (From left to right)
   Lane 1: ds
**Figure 6****:** Stability of siRNA in 4 different formats in mouse serum. Samples were incubated at 37°C in mouse serum at a 2 micromolar concentration. Disappearance of parent compound was followed over a 6 hours period by quantifying the parent compound band.
   (From left to right)
   Lane 1: ds RNA ladder (30, 21, 19, 16, 13, 10 bp) RNA ladder (30, 21, 19, 16, 13, 10 bp)
   Lane 2: wild-type siRNA untreated
   Lane 3: moe o/h siRNA untreated
   Lane 4: C3 siRNA untreated
   Lane 5: C3-MOE siRNA untreated
   Lane 6-9: same as 2-5 in liver microsomes t=0
   Lane 10-13: same as 2-5 in liver microsomes t=60'
   Lane 14-17: same as 2-5 in supernatant S12 t=0
   Lane 18-21: same as 2-5 in supernatant S12 t=60'
   Lane 2-7: wild-type siRNA in mouse serum at t=0, 15, 30, 60, 180 and 360 min
   Lane 8-13: moe o/h siRNA in mouse serum at t=0, 15, 30, 60, 180 and 360 min
   Lane 14-19: C3 siRNA in mouse serum at t=0, 15, 30, 60, 180 and 360 min
   Lane 20-25: C3-MOE siRNA mouse serum at t=0, 15, 30, 60, 180 and 360 min
**Figure 7****:** Characterization *in cellulo* of 3 formats of anti-VEGFR2 siRNA (2 independent sequences). Wild-type siRNA, C3-siRNA and C3-MOE siRNA were transfected into MS1 cells at three concentrations (1, 5, 10 nM). Silencing potency was assessed by measuring VEGFR2 cell surface level by FACS.

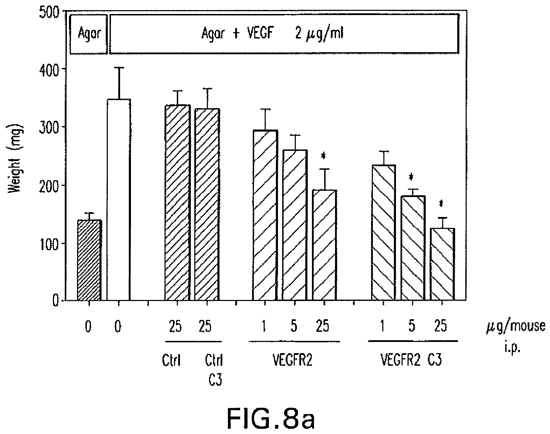

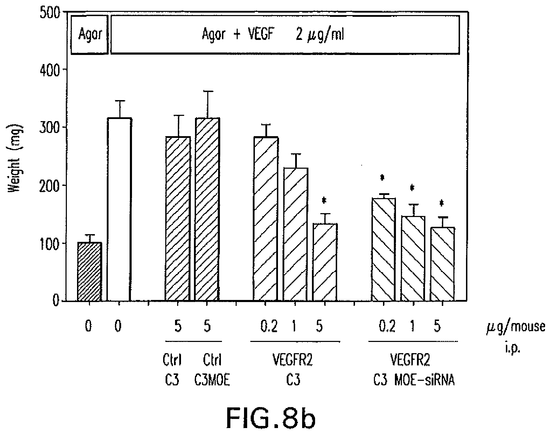

**Figure 8a** **and** **8b****:** *In vivo* testing of wild-type siRNA, C3-siRNA and C3-Moe siRNA in a growth factor induced angiogenesis "Agar Chamber" mouse model. Figure 8a shows the results of controls, unmodified VEGFR2 siRNA and C3 modified VEGFR2 siRNA at 1, 5 and 25 micrograms per mouse per day. Figure 8b shows controls, C3 modified VEGFR2 siRNA and of C3-MOE VEGFR2 siRNA at 0.2, 1 and 5 micrograms per mouse per day. In each case pools of 2 anti-VEGFR2 siRNAs were given daily intraperitoneally for three days.

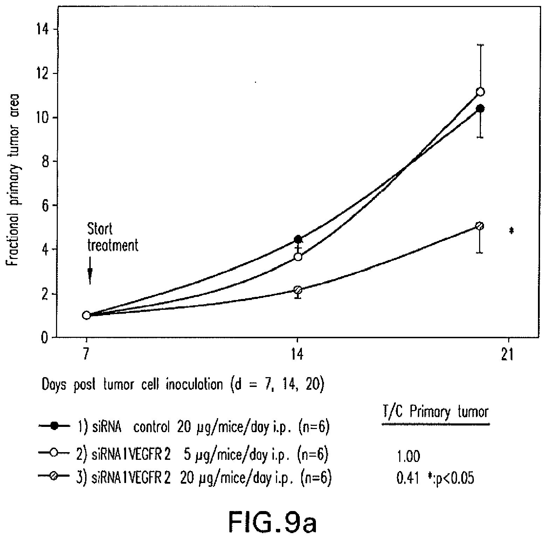

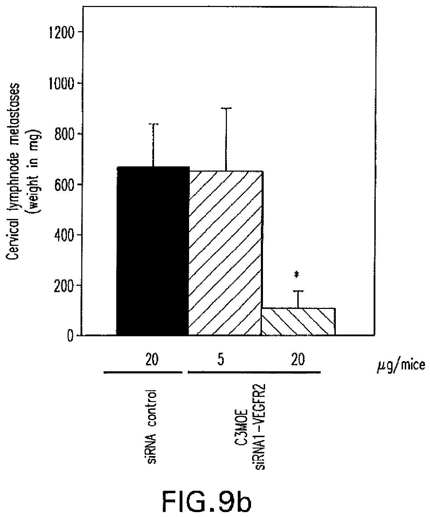

**Figure 9****:** *In vivo* testing of anti-VEGFR2 C3-MOE siRNA given intraperitoneally (i.p.) in a B16 homograft melanoma tumor mouse model at 5 and 20 micrograms per mouse per day. **Figure 9a** shows that i.p. treatment with modified VEGFR2 siRNA significantly reduces tumour development. **Figure 9b** also shows that i.p. injection of VEGFR2 siRNA at 20 ug per mouse results in significant inhibition of tumour growth.

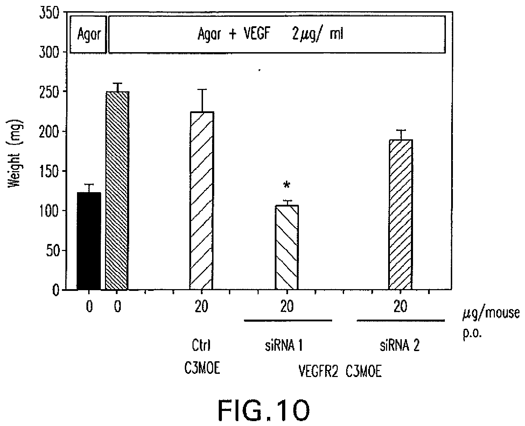

**Figure 10****:** *In vivo* testing of C3-MOE siRNA in a growth factor induced angiogenesis mouse model. anti-VEGFR2 siRNAs were given daily orally for three days at 20 micrograms per mouse per day.

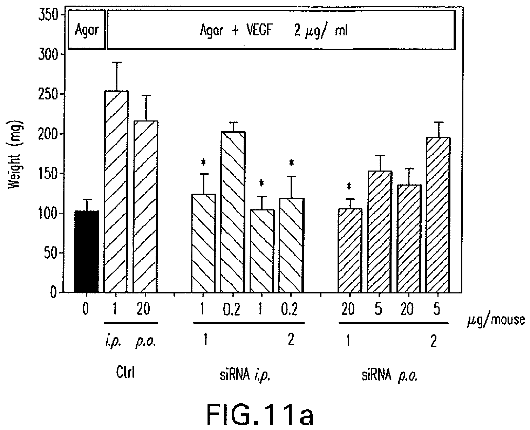

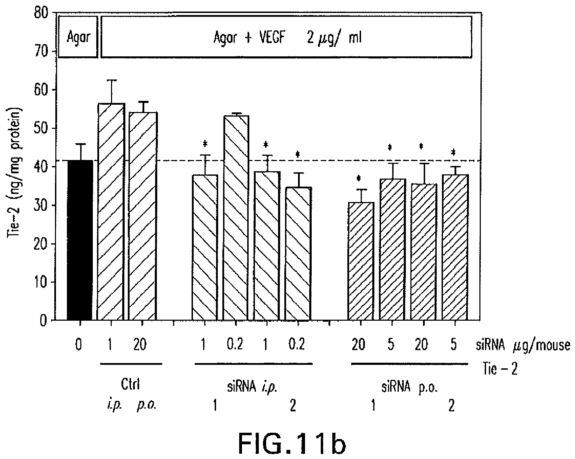

**Figure 11****:** *In vivo* testing of C3-MOE siRNA in a growth factor induced angiogenesis mouse model. anti-Tie2 siRNAs were given daily intraperitoneally (1 and 0.2 micrograms per mouse per day) or orally (20 and 5 micrograms per mouse per day) for three days. Figure 11a: weight of excised tissue; Figure 11b: Tie2 protein knock-down

### Detailed Disclosure of the Invention:

The present disclosure relates to compositions and methods for treating angiogenic disorders in a mammal. Specifically, the invention relates to small-interfering RNA ("siRNA") which may be used to treat angiogenic disorders upon oral administration to a mammal.

Angiogenesis targets in vascular endothelial cells include the following targets/genes: VEGFR-1 (GenBank Accession # AF06365); VEGFR-2 (GenBank Accession # AF063658); VEGFR-3 (GenBank Accession # (NM_002020); Tie2 (TEK) (GenBank Accession # NM_000459); bFGFR (GenBank Accession # M60485); IL8RA (GenBank Accession # L19591); IL8RB (GenBank Accession # L19593); Fas (GenBank Accession # X89101); IGF2R (GenBank Accession # NM_000876).

The siRNA molecules according to the present invention mediate RNA interference ("RNAi"). The term "RNAi" is well known in the art and is commonly understood to mean the inhibition of one or more target genes in a cell by siRNA with a region which is complementary to the target gene. Various assays are known in the art to test siRNA for its ability to mediate RNAi (see for instance Elbashir et al., Methods 26 (2002), 199-213). The effect of the siRNA according to the present invention on gene expression will typically result in expression of the target gene being inhibited by at least 10%, 33%, 50%, 90%, 95% or 99% when compared to a cell not treated with the RNA molecules according to the present invention.

"siRNA" or "small-interfering ribonucleic acid" according to the invention has the meanings known in the art, including the following aspects. The siRNA consists of two strands of ribonucleotides which hybridize along a complementary region under physiological conditions. The strands are separate but they may be joined by a molecular linker in certain embodiments. The individual ribonucleotides may be unmodified naturally occurring ribonucleotides, unmodified naturally occurring deoxyribonucleotides or they may be chemically modified or synthetic as described elsewhere herein.

The siRNA molecules in accordance with the present invention comprise a double-stranded region which is substantially identical to a region of the mRNA of the target gene. A region with 100% identity to the corresponding sequence of the target gene is suitable. This state is referred to as "fully complementary". However, the region may also contain one, two or three mismatches as compared to the corresponding region of the target gene, depending on the length of the region of the mRNA that is targeted, and as such may be not fully complementary. In an embodiment, the RNA molecules of the present invention specifically target one given gene. In order to only target the desired mRNA, the siRNA reagent may have 100% homology to the target mRNA and at least 2 mismatched nucleotides to all other genes present in the cell or organism. Methods to analyze and identify siRNAs with sufficient sequence identity in order to effectively inhibit expression of a specific target sequence are known in the art. Sequence identity may be optimized by sequence comparison and alignment algorithms known in the art (see Gribskov and Devereux, Sequence Analysis Primer, Stockton Press, 1991, and references cited therein) and calculating the percent difference between the nucleotide sequences by, for example, the Smith-Waterman algorithm as implemented in the BESTFIT software program using default parameters (e.g., University of Wisconsin Genetic Computing Group).

Another factor affecting the efficiency of the RNAi reagent is the target region of the target gene. The region of a target gene effective for inhibition by the RNAi reagent may be determined by experimentation. A suitable mRNA target region would be the coding region. Also suitable are untranslated regions, such as the 5'-UTR, the 3'-UTR, and splice junctions. For instance, transfection assays as described in Elbashir S.M. et al, 2001 EMBO J., 20, 6877-6888 may be performed for this purpose. A number of other suitable assays and methods exist in the art which are well known to the skilled person.

The length of the region of the siRNA complementary to the target, in accordance with the present invention, may befrom 10 to 100 nucleotides, 12 to 25 nucleotides, 14 to 22 nucleotides or 15, 16, 17 or 18 nucleotides. Where there are mismatches to the corresponding target region, the length of the complementary region is generally required to be somewhat longer.

Because the siRNA may carry overhanging ends (which may or may not be complementary to the target), or additional nucleotides complementary to itself but not the target gene, the total length of each separate strand of siRNA may be 10 to 100 nucleotides, 15 to 49 nucleotides, 17 to 30 nucleotides or 19 to 25 nucleotides.

The phrase "each strand is 49 nucleotides or less" means the total number of consecutive nucleotides in the strand, including all modified or unmodified nucleotides, but not including any chemical moieties which may be added to the 3' or 5' end of the strand. Short chemical moieties inserted into the strand are not counted, but a chemical linker designed to join two separate strands is not considered to create consecutive nucleotides.

The phrase "a 1 to 6 nucleotide overhang on at least one of the 5' end or 3' end" refers to the architecture of the complementary siRNA that forms from two separate strands under physiological conditions. If the terminal nucleotides are part of the double-stranded region of the siRNA, the siRNA is considered blunt ended. If one or more nucleotides are unpaired on an end, an overhang is created. The overhang length is measured by the number of overhanging nucleotides. The overhanging nucleotides can be either on the 5' end or 3' end of either strand.

The siRNA according to the present invention confer a high *in vivo* stability suitable for oral delivery by including at least one modified nucleotide in at least one of the strands. Thus the siRNA according to the present invention contains at least one modified or non-natural ribonucleotide. A lengthy description of many known chemical modifications are set out in published PCT patent application WO 200370918 and will not be repeated here. Suitable modifications for oral delivery are more specifically set out in the Examples and description herein. Suitable modifications include, but are not limited to modifications to the sugar moiety (i.e. the 2' position of the sugar moiety, such as for instance 2'-O-(2-methoxyethyl) or 2'-MOE) (Martin et al., Helv. Chim. Acta, 1995, 78, 486-504) i.e., an alkoxyalkoxy group) or the base moiety (i.e. a non-natural or modified base which maintains ability to pair with another specific base in an alternate nucleotide chain). Other modifications include so-called 'backbone' modifications including, but not limited to, replacing the phosphoester group (connecting adjacent ribonuclotides with for instance phosphorothioates, chiral phosphorothioates or phosphorodithioates). Finally, end modifications sometimes referred to herein as 3' caps or 5' caps may be of significance. As illustrated in Table 1, caps may consist of simply adding additional nucleotides, such as "T-T" which has been found to confer stability on an siRNA. Caps may consist of more complex chemistries which are known to those skilled in the art.

In an embodiment used in the Examples below, the 3' cap is a chemical moiety conjugated to the 3' end via the 3' carbon and is selected from among compounds of Formula I: 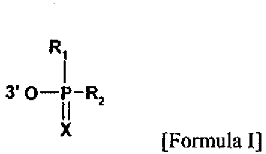 wherein
X is O or S
R₁ and R₂ are independently OH, NH₂, SH, alkyl, aryl, alkyl-aryl, aryl-alkyl, where alkyl, aryl, alkyl-aryl, aryl-alkyl can be substituted by additional heteroatoms and functional groups, preferably a heteroatom selected from the group of N, O, or S or a functional group selected from the group OH, NH₂, SH, carboxylic acid or ester;
or R₁ and R₂ may be of formula Y-Z where Y is O, N, S and Z is H, alkyl, aryl, alkyl-aryl, aryl-alkyl, where alkyl, aryl, alkyl-aryl, aryl-alkyl can be substituted by additional heteroatoms, preferably a heteroatom selected from the group of N, O, or S.

Examples of modifications on the sugar moiety include 2' alkoxyribonucleotide, 2' alkoxyalkoxy ribonucleotide, locked nucleic acid ribonucleotide (LNA), 2'-fluoro ribonucleotide, morpholino nucleotide.

The internucleoside linkage may also be modified. Examples of internucleoside linkage include phosphorothioate, phosphorodithioate, phosphoramidate, and amide linkages.

R₁ may be OH.

R₁ and R₂ together may comprise from 1 to 24 C-atoms, from 1 to 12 C-atoms, from 2 to 10 C-atoms, from 1 to 8 or from 2 to 6 C-atoms. In another embodiment, R₁ and R₂ are independently OH, lower alkyl, lower aryl, lower alkyl-aryl, lower aryl-alkyl, where lower alkyl, lower aryl, lower alkyl-aryl, lower aryl-alkyl can be substituted by additional heteroatoms and functional groups as defined above. In another embodiment, R₁ and R₂ are not both OH.

The term "lower" in connection with organic radicals or compounds means a compound or radical which may be branched or unbranched with up to and including 7 carbon atoms, preferably 1-4 carbon atoms. Lower alkyl represents, for example, methyl, ethyl, n-propyl, isopropyl, n-butyl, sec-butyl, tert-butyl, n-pentyl and branched pentyl, n-hexyl and branched hexyl.

Examples of alkoxys include O-Met, O-Eth, O-prop, O-but, O-pent, O-hex.

Methods for the synthesis of siRNA, including siRNA containing at least one modified or non-natural ribonucleotides are well known and readily available to those of skill in the art For example, a variety of synthetic chemistries are set out in published PCT patent applications WO2005021749 and WO200370918, both incorporated herein by reference. The reaction may be carried out in solution or, preferably, on solid phase or by using polymer supported reagents, followed by combining the synthesized RNA strands under conditions, wherein a siRNA molecule is formed, which is capable of mediating RNAi.

The present disclosure provides an siRNA containing at least one modified nucleotide which is suitable for oral delivery. In functional terms this means siRNA will have suitable pharmacokinetics and biodistribution upon oral administration to achieve delivery to the target tissue of concern. In particular this requires serum stability, lack of immune response, and drug like behaviour.. Many of these features of siRNA can be anticipated based on the standard gastric acid assays and standard serum assays disclosed elsewhere herein.

In another aspect, the present disclosure provides methods for the inhibition of a target gene comprising introducing into a cell and siRNA according to the present invention, which is capable of inhibiting at least one target gene by RNAi. Also, more than one species of siRNA, which are each specific for another target region, may be introduced into a cell at the same time or sequentially.

The present disclosure is not limited to any type of target gene or nucleotide sequence. For example, the target gene can be a cellular gene, an endogenous gene, a pathogen-associated gene, a viral gene or an oncogene. Angiogenic genes are of particular importance to the invention because some of the Examples highlight that the orally delivered siRNA of the invention may accumulate at sites of vasculogenesis, neovascularization or angiogenesis. An updated listing of angiogenic genes at these sites of particular interest for the invention are listed in AngioDB: database of angiogenesis and angiogenesis-related molecules Tae-Kwon Sohn, Eun-Joung Moon1, Seok-Ki Lee1, Hwan-Gue Cho2 and Kyu-Won Kim3, Nucleic Acids Research, 2002, Vol. 30, No. 1 369-371 and online at http://angiodb.snu.ac.kr/. Genes of particular significance have been analyzed in detail and are set out elsewhere herein.

In another aspect, the disclosure also provides a kit comprising reagents for inhibiting expression of a target gene in a cell, wherein said kit comprises dsRNA according to the present invention. The kit comprises at least one of the reagents necessary to carry out the *in vitro* or *in vivo* introduction of the dsRNA according to the present invention to test samples or subjects. In a preferred aspect, such kits also comprise instructions detailing the procedures by which the kit components are to be used.

"Treatment of an angiogenic disorder" as used in this disclosure means use of a modified siRNA of the invention in a pharmaceutical composition for the treatment of diseases involving the physiological and pathological processes of neovascularization, vasculogenesis and/or angiogenesis. As such, these pharmaceutical compositions are useful for treating diseases, conditions and disorders that require inhibition of neovascularization, vasculogenesis or angiogenesis, including but not limited to cancer tumour growth and metastasis, neoplasm, ocular neovascularization (including macular degeneration, diabetic retinopathy, ischemic retinopathy, retinopathy of prematurity, choroidal neovascularization), rheumatoid arthritis, osteoarthritis, chronic asthma, spectic shock, inflammatory diseases, synovitis, bone and cartilage destruction, pannus growth, osteophyte formation, osteomyelitis, psoriasis, obesity, haemangioma, Kaposi's sarcoma, atherosclerosis (including atherosclerotic plaque rupture), endometriosis, warts, excess hair growth, scar keloids, allergic oedema, dysfunctional uterine bleeding, follicular cysts, ovarian hyperstimulation, endometriosis, osteomyelitis, inflammatory and infectious processes (hepatitis, pneumonia, glumerulonephtritis), asthma, nasal polyps, transplantation, liver regeneration, leukomalacia, thyroiditis, thyroid enlargement, lymphoproliferative disorders, haematologic malignancies, vascular malformations, and pre-eclampsia.

As used herein, "treatment" means an action taken to inhibit or reduce a process of a disease, disorder or condition, to inhibit or reduce a symptom of a disease, disorder or condition, or to prophylactically prevent the onset or further development of a disease, disorder or condition. "Treat" is the cognitive verb thereof.

An effective dose of the therapeutic agent of the invention is that dose required to treat a disease state. The effective dose depends on the type of disease, the composition used, the route of administration, the type of mammal being treated, the physical characteristics of the specific mammal under consideration, concurrent medication, and other factors that those skilled in the medical arts will recognize. Generally, an amount between 0.1 mg/kg and 100 mg/kg body weight/day of siRNA is administered dependent upon potency. The nucleic acid molecules of the invention and formulations thereof can be administered orally, topically, parenterally, by inhalation or spray, or rectally in dosage unit formulations containing conventional non-toxic pharmaceutically acceptable carriers, adjuvants and/or vehicles. The term parenteral as used herein includes percutaneous, subcutaneous, intravascular (e.g., intravenous), intramuscular, intraperitoneal, or intrathecal injection, or infusion techniques and the like. In addition, there is provided a pharmaceutical formulation comprising a nucleic acid molecule of the invention and a pharmaceutically acceptable carrier. One or more nucleic acid molecules of the invention can be present in association with one or more non-toxic pharmaceutically acceptable carriers and/or diluents and/or adjuvants, and if desired other active ingredients. The pharmaceutical compositions containing nucleic acid molecules of the invention can be in a form suitable for oral use, for example, as tablets, troches, lozenges, aqueous or oily suspensions, dispersible powders or granules, emulsion, hard or soft capsules, or syrups or elixirs.

Compositions intended for oral use can be prepared according to any method known to the art for the manufacture of pharmaceutical compositions and such compositions can contain one or more such sweetening agents, flavoring agents, coloring agents or preservative agents in order to provide pharmaceutically elegant and palatable preparations. Tablets contain the active ingredient in admixture with non-toxic pharmaceutically acceptable excipients that are suitable for the manufacture of tablets. These excipients can be, for example, inert diluents; such as calcium carbonate, sodium carbonate, lactose, calcium phosphate or sodium phosphate; granulating and disintegrating agents, for example, corn starch, or alginic acid; binding agents, for example starch, gelatin or acacia; and lubricating agents, for example magnesium stearate, stearic acid or talc. The tablets can be uncoated or they can be coated by known techniques. Formulations for oral use can also be presented as hard gelatin capsules wherein the active ingredient is mixed with an inert solid diluent, for example, calcium carbonate, calcium phosphate or kaolin, or as soft gelatin capsules wherein the active ingredient is mixed with water or an oil medium, for example peanut oil, liquid paraffin or olive oil. Aqueous suspensions contain the active materials in a mixture with excipients suitable for the manufacture of aqueous suspensions.

Oral administration of the compositions of the invention include all standard techniques for administering substances directly to the stomach or gut, most importantly by patient controlled swallowing of the dosage form, but also by other mechanical and assisted means of such delivery.

Dosage levels of the order of from about 0.1 mg to about 140 mg per kilogram of body weight per day are useful in the treatment of the above- indicated conditions (about 0.5 mg to about 7 g per subject per day). The amount of active ingredient that can be combined with the carrier materials to produce a single dosage form varies depending upon the host treated and the particular mode of administration. Dosage unit forms generally contain between from about 1 mg to about 500 mg of an active ingredient. It is understood that the specific dose level for any particular subject depends upon a variety of factors including the activity of the specific compound employed, the age, body weight, general health, sex, diet, time of administration, route of administration, and rate of excretion, drug combination and the severity of the particular disease undergoing therapy.

Therapeutic effect of the therapeutic agents of the invention may be enhanced by combination with other agents. Typically such other agents will include agents known for use in treating similar diseases, such as angiogenic disorders. Alternatively, such agents may be used to reduce side-effects or unwanted effects caused by the therapeutic agents of the invention.

The siRNA of the invention also have important research uses. One such study includes research into an angiogenic process *in vitro.* By "angiogenic process *in vitro*" is meant any process for studying angiogenesis or vasculogenesis which does not employ a whole animal. As such, *in vitro* or *ex vivo* methods and assays which study the steps of the angiogenic process using markers or indicators of angiogenesis are included hereby.

### RNA Strand Nucleotide Sequences

The siRNA strand sequences identified in **Table 1** have been identified as suitable siRNA sequences against the following targets: VEGFR-1 (GenBank Accession # AF06365); VEGFR-2 (GenBank Accession # AF063658); VEGFR-3 (GenBank Accession # (NM_002020); Tie2 (TEK) (GenBank Accession # NM_000459); bFGFR (GenBank Accession # M60485); IL8RA (GenBank Accession # L19591); IL8RB (GenBank Accession # L19593); Fas (GenBank Accession # X89101); IGF2R (GenBank Accession # NM_000876).

**Table 1: siRNAs against human VEGFR-1, VEGFR-2, VEGFR-3, Tie2, bFGFR, IL8RA, IL8RB, Fas, IGF2R**

| | **pos** | | **SEQ ID** | | **SEQ ID** |
|---|---|---|---|---|---|
| **Target Name** | | **siRNA guide sequence** | **NO** | **siRNA complement** | **NO** |
| VEGFR-1 | 1731 | UAUAAGAACUUGUUAACUGTG | 1 | CAGUUAACAAGUUCUUAUATT | 451 |
| VEGFR-1 | 1021 | UACGGUUUCAAGCACCUGCTG | 2 | GCAGGUGCUUGAAACCGUATT | 452 |
| VEGFR-1 | 1209 | UUUAUGCUCAGCAAGAUUGTA | 3 | CAAUCUUGCUGAGCAUAAATT | 453 |
| VEGFR-1 | 2904 | UUAUCUUCCUGAAAGCCGGAG | 4 | CCGGCUUUCAGGAAGAUAATT | 454 |
| VEGFR-1 | 1363 | UUGAGGGAUACCAUAUGCGGT | 5 | CGCAUAUGGUAUCCCUCAATT | 455 |
| VEGFR-1 | 1158 | UUGAUAAUUAACGAGUAGCCA | 6 | GCUACUCGUUAAUUAUCAATT | 456 |
| VEGFR-1 | 1091 | UUAACCAUACAACUUCCGGCG | 7 | CCGGAAGUUGUAUGGUUAATT | 457 |
| VEGFR-1 | 471 | UUAGGUGACGUAACCCGGCAG | 8 | GCCGGGUUACGUCACCUAATT | 458 |
| VEGFR-1 | 2751 | UUGCUCUUGAGGUAGUUGGAG | 9 | CCAACUACCUCAAGAGCAATT | 459 |
| VEGFR-1 | 636 | UUUGUCUUAUACAAAUGCCCA | 10 | GGCAUUUGUAUAAGACAAATT | 460 |
| VEGFR-1 | 1254 | UUGACAAUUAGAGUGGCAGTG | 11 | CUGCCACUCUAAUUGUCAATT | 461 |
| VEGFR-1 | 2375 | UUAUAAUUGAUAGGUAGUCAG | 12 | GACUACCUAUCAAUUAUAATT | 462 |
| VEGFR-1 | 3536 | UUGAGUAUGUAAACCCACUAT | 13 | AGUGGGUUUACAUACUCAATT | 463 |
| VEGFR-1 | 2971 | UUCCAUAGUGAUGGGCUCCTT | 14 | GGAGCCCAUCACUAUGGAATT | 464 |
| VEGFR-1 | 1774 | UCUGUUAUUAACUGUCCGCAG | 15 | GCGGACAGUUAAUAACAGATT | 465 |
| VEGFR-1 | 3494 | UUGGGAUGUAGUCUUUACCAT | 16 | GGUAAAGACUACAUCCCAATT | 466 |
| VEGFR-1 | 2269 | UGUUAGAGUGAUCAGCUCCAG | 17 | GGAGCUGAUCACUCUAACATT | 467 |
| VEGFR-1 | 525 | UUUCCAUCAGGGAUCAAAGTG | 18 | CUUUGAUCCCUGAUGGAAATT | 468 |
| VEGFR-1 | 769 | UUGAACUCUCGUGUUCAAGGG | 19 | CUUGAACACGAGAGUUCAATT | 469 |
| VEGFR-1 | 2246 | UAGACUUGUCCGAGGUUCCTT | 20 | GGAACCUCGGACAAGUCUATT | 470 |
| VEGFR-1 | 732 | UUGAGGACAAGAGUAUGGCCT | 21 | GCCAUACUCUUGUCCUCAATT | 471 |
| VEGFR-1 | 3813 | UUACUGGUUACUCUCAAGUCA | 22 | ACUUGAGAGUAACCAGUAATT | 472 |
| VEGFR-1 | 3925 | UUCCAGCUCAGCGUGGUCGTA | 23 | CGACCACGCUGAGCUGGAATT | 473 |
| VEGFR-1 | 1414 | UGCUUCGGAAUGAUUAUGGTT | 24 | CCAUAAUCAUUCCGAAGCATT | 474 |
| VEGFR-1 | 615 | UUGACUGUUGCUUCACAGGTC | 25 | CCUGUGAAGCAACAGUCAATT | 475 |
| VEGFR-1 | 3300 | UCAUCCAUUUGUACUCCUGGG | 26 | CAGGAGUACAAAUGGAUGATT | 476 |
| VEGFR-1 | 2845 | UGGUUUCUUGCCUUGUUCCAG | 27 | GGAACAAGGCAAGAAACCATT | 477 |
| VEGFR-1 | 2802 | UUAGGCUCCAUGUGUAGUGCT | 28 | CACUACACAUGGAGCCUAATT | 478 |
| VEGFR-1 | 1564 | UCUAGAGUCAGCCACAACCAA | 29 | GGUUGUGGCUGACUCUAGATT | 479 |
| VEGFR-1 | 1154 | UAAUUAACGAGUAGCCACGAG | 30 | CGUGGCUACUCGUUAAUUATT | 480 |
| VEGFR-1 | 1090 | UAACCAUACAACUUCCGGCGA | 31 | GCCGGAAGUUGUAUGGUUATT | 481 |
| VEGFR-1 | 1260 | UUCACAUUGACAAUUAGAGTG | 32 | CUCUAAUUGUCAAUGUGAATT | 482 |
| VEGFR-1 | 3530 | AUGUAAACCCACUAUUUCCTG | 33 | GGAAAUAGUGGGUUUACAUTT | 483 |
| VEGFR-1 | 1177 | AUCCUCUUCAGUUACGUCCTT | 34 | GGACGUAACUGAAGAGGAUTT | 484 |
| VEGFR-1 | 1193 | UUGUAUAAUUCCCUGCAUCCT | 35 | GAUGCAGGGAAUUAUACAATT | 485 |
| VEGFR-1 | 1092 | UUUAACCAUACAACUUCCGGC | 36 | CGGAAGUUGUAUGGUUAAATT | 486 |
| VEGFR-1 | 627 | UACAAAUGCCCAUUGACUGTT | 37 | CAGUCAAUGGGCAUUUGUATT | 487 |
| VEGFR-1 | 474 | AUGUUAGGUGACGUAACCCGG | 38 | GGGUUACGUCACCUAACAUTT | 488 |
| VEGFR-1 | 2761 | UAAGUCACGUUUGCUCUUGAG | 39 | CAAGAGCAAACGUGACUUATT | 489 |
| VEGFR-1 | 2752 | UUUGCUCUUGAGGUAGUUGGA | 40 | CAACUACCUCAAGAGCAAATT | 490 |
| VEGFR-1 | 3516 | UUUCCUGUCAGUAUGGCAUTG | 41 | AUGCCAUACUGACAGGAAATT | 491 |
| VEGFR-1 | 1790 | UACUGUAGUGCAUUGUUCUGT | 42 | AGAACAAUGCACUACAGUATT | 492 |
| VEGFR-1 | 1155 | AUAAUUAACGAGUAGCCACGA | 43 | GUGGCUACUCGUUAAUUAUTT | 493 |
| VEGFR-1 | 1370 | UUGUAGGUUGAGGGAUACCAT | 44 | GGUAUCCCUCAACCUACAATT | 494 |
| VEGFR-1 | 2227 | UUGAACAGUGAGGUAUGCUGA | 45 | AGCAUACCUCACUGUUCAATT | 495 |
| VEGFR-1 | 3481 | UUUACCAUCCUGUUGUACATT | 46 | UGUACAACAGGAUGGUAAATT | 496 |
| VEGFR-1 | 1261 | UUUCACAUUGACAAUUAGAGT | 47 | UCUAAUUGUCAAUGUGAAATT | 497 - |
| VEGFR-1 | 1791 | AUACUGUAGUGCAUUGUUCTG | 48 | GAACAAUGCACUACAGUAUTT | 498 |
| VEGFR-1 | 3805 | UACUCUCAAGUCAAUCUUGAG | 49 | CAAGAUUGACUUGAGAGUATT | 499 |
| VEGFR-1 | 2764 | AAAUAAGUCACGUUUGCUCTT | 50 | GAGCAAACGUGACUUAUUUTT | 500 |
| VEGFR-2 | 617 | UAAUAGACUGGUAACUUUCAT | 51 | GAAAGUUACCAGUCUAUUATT | 501 |
| VEGFR-2 | 2686 | UAGAAGGUUGACCACAUUGAG | 52 | CAAUGUGGUCAACCUUCUATT | 502 |
| VEGFR-2 | 561 | UAGCUGAUCAUGUAGCUGGGA | 53 | CCAGCUACAUGAUCAGCUATT | 503 |
| VEGFR-2 | 525 | UUGCUGUCCCAGGAAAUUCTG | 54 | GAAUUUCCUGGGACAGCAATT | 504 |
| VEGFR-2 | 2277 | AUGAUUUCCAAGUUCGUCUTT | 55 | AGACGAACUUGGAAAUCAUTT | 505 |
| VEGFR-2 | 395 | UAAUGUACACGACUCCAUGTT | 56 | CAUGGAGUCGUGUACAUUATT | 506 |
| VEGFR-2 | 2410 | UUCAUCUGGAUCCAUGACGAT | 57 | CGUCAUGGAUCCAGAUGAATT | 507 |
| VEGFR-2 | 2007 | UGAUUCUCCAGGUUUCCUGTG | 58 | CAGGAAACCUGGAGAAUCATT | 508 |
| VEGFR-2 | 1323 | UAGACCGUACAUGUCAGCGTT | 59 | CGCUGACAUGUACGGUCUATT | 509 |
| VEGFR-2 | 3382 | UUCUGGUGUAGUAUAAUCAGG | 60 | UGAUUAUACUACACCAGAATT | 510 |
| VEGFR-2 | 3078 | UUUCGUGCCGCCAGGUCCCTG | 61 | GGGACCUGGCGGCACGAAATT | 511 |
| VEGFR-2 | 1432 | UUCUUCACAAGGGUAUGGGTT | 62 | CCCAUACCCUUGUGAAGAATT | 512 |
| VEGFR-2 | 1817 | UCAAUUUCCAAAGAGUAUCCA | 63 | GAUACUCUUUGGAAAUUGATT | 513 |
| VEGFR-2 | 688 | UAGUUCAAUUCCAUGAGACGG | 64 | GUCUCAUGGAAUUGAACUATT | 514 |
| VEGFR-2 | 2310 | AACAUGGCAAUCACCGCCGTG | 65 | CGGCGGUGAUUGCCAUGUUTT | 515 |
| VEGFR-2 | 2130 | UCCUUCAAUACAAUGCCUGAG | 66 | CAGGCAUUGUAUUGAAGGATT | 516 |
| VEGFR-2 | 799 | UACAAGUUUCUUAUGCUGATG | 67 | UCAGCAUAAGAAACUUGUATT | 517 |
| VEGFR-2 | 3523 | UGAUAUCGGAAGAACAAUGTA | 68 | CAUUGUUCUUCCGAUAUCATT | 518 |
| VEGFR-2 | 1843 | UGUGCUAUUAGAGAACAUGGT | 69 | CAUGUUCUCUAAUAGCACATT | 519 |
| VEGFR-2 | 2941 | UUCUACAUCACUGAGGGACTT | 70 | GUCCCUCAGUGAUGUAGAATT | 520 |
| VEGFR-2 | 2088 | UCUUUAAACCACAUGAUCUGT | 71 | AGAUCAUGUGGUUUAAAGATT | 521 |
| VEGFR-2 | 472 | UCUUGCACAAAGUGACACGTT | 72 | CGUGUCACUUUGUGCAAGATT | 522 |
| VEGFR-2 | 180 | UGAUUAUUGGGCCAAAGCCAG | 73 | GGCUUUGGCCCAAUAAUCATT | 523 |
| VEGFR-2 | 1568 | AUUUGUACAAAGCUGACACAT | 74 | GUGUCAGCUUUGUACAAAUTT | 524 |
| VEGFR-2 | 3141 | UAAAUAUCCCGGGCCAAGCCA | 75 | GCUUGGCCCGGGAUAUUUATT | 525 |
| VEGFR-2 | 3769 | AACCAUACCACUGUCCGUCTG | 76 | GACGGACAGUGGUAUGGUUTT | 526 |
| VEGFR2 | 3920 | UGUCAUCGGAGUGAUAUCCGG | 77 | GGAUAUCACUCCGAUGACATT | 527 |
| VEGFR-2 | 1718 | UCUCAAACGUAGAUCUGUCTG | 78 | GACAGAUCUACGUUUGAGATT | 528 |
| VEGFR-2 | 2919 | UCCUCCACAAAUCCAGAGCTG | 79 | GCUCUGGAUUUGUGGAGGATT | 529 |
| VEGFR-2 | 324 | UAAAUGACCGAGGCCAAGUCA | 80 | ACUUGGCCUCGGUCAUUUATT | 530 |
| VEGFR-2 | 1050 | UAACCAAGGUACUUCGCAGGG | 81 | CUGCGAAGUACCUUGGUUATT | 531 |
| VEGFR-2 | 56 | UAGGCAAACCCACAGAGGCGG | 82 | GCCUCUGUGGGUUUGCCUATT | 532 |
| VEGFR-2 | 2453 | UGGCAUCAUAAGGCAGUCGTT | 83 | CGACUGCCUUAUGAUGCCATT | 533 |
| VEGFR-2 | 1303 | UUGAGUGGUGCCGUACUGGTA | 84 | CCAGUACGGCACCACUCAATT | 534 |
| VEGFR-2 | 1813 | UUUCCAAAGAGUAUCCAAGTT | 85 | CUUGGAUACUCUUUGGAAATT | 535 |
| VEGFR-2 | 2015 | UUGUCGUCUGAUUCUCCAGGT | 86 | CUGGAGAAUCAGACGACAATT | 536 |
| VEGFR-2 | 3088 | UAAGAGGAUAUUUCGUGCCGC | 87 | GGCACGAAAUAUCCUCUUATT | 537 |
| VEGFR-2 | 625 | UAUGUACAUAAUAGACUGGTA | 88 | CCAGUCUAUUAUGUACAUATT | 538 |
| VEGFR-2 | 800 | UUACAAGUUUCUUAUGCUGAT | 89 | CAGCAUAAGAAACUUGUAATT | 539 |
| VEGFR-2 | 811 | UAGGUCUCGGUUUACAAGUTT | 90 | ACUUGUAAACCGAGACCUATT | 540 |
| VEGFR-2 | 812 | UUAGGUCUCGGUUUACAAGTT | 91 | CUUGUAAACCGAGACCUAATT | 541 |
| VEGFR-2 | 3093 | UCCGAUAAGAGGAUAUUUCGT | 92 | GAAAUAUCCUCUUAUCGGATT | 542 |
| VEGFR-2 | 801 | UUUACAAGUUUCUUAUGCUGA | 93 | AGCAUAAGAAACUUGUAAATT | 543 |
| VEGFR-2 | 2009 | UCUGAUUCUCCAGGUUUCCTG | 94 | GGAAACCUGGAGAAUCAGATT | 544 |
| VEGFR-2 | 2127 | UUCAAUACAAUGCCUGAGUCT | 95 | ACUCAGGCAUUGUAUUGAATT | 545 |
| VEGFR-2 | 1585 | UUUGUUGACCGCUUCACAUTT | 96 | AUGUGAAGCGGUCAACAAATT | 546 |
| VEGFR-2 | 562 | AUAGCUGAUCAUGUAGCUGGG | 97 | CAGCUACAUGAUCAGCUAUTT | 547 |
| VEGFR-2 | 3906 | UAUCCGGACUGGUAGCCGCTT | 98 | GCGGCUACCAGUCCGGAUATT | 548 |
| VEGFR-2 | 1316 | UACAUGUCAGCGUUUGAGUGG | 99 | ACUCAAACGCUGACAUGUATT | 549 |
| VEGFR-2 | 3520 | UAUCGGAAGAACAAUGUAGTC | 1 00 | CUACAUUGUUCUUCCGAUATT | 550 |
| VEGFR-3 | 453 | UUCCUGUUGACCAAGAGCGTG | 101 | CGCUCUUGGUCAACAGGAATT | 551 |
| VEGFR-3 | 2694 | UUGAGCUCCGACAUCAGCGCG | 102 | CGCUGAUGUCGGAGCUCAATT | 552 |
| VEGFR-3 | 1689 | UUGGAUUCGAUGGUGAAGCCG | 103 | GCUUCACCAUCGAAUCCAATT | 553 |
| VEGFR-3 | 988 | UUCAUGCACAAUGACCUCGGT | 104 | CGAGGUCAUUGUGCAUGAATT | 554 |
| VEGFR-3 | 4374 | UUACCAAGGAAUAAUCGGCGG | 105 | GCCGAUUAUUCCUUGGUAATT | 555 |
| VEGFR-3 | 2142 | UCUUUGUACCACACGAUGCTG | 106 | GCAUCGUGUGGUACAAAGATT | 556 |
| VEGFR-3 | 1833 | UUGCAGUCGAGCAGAAGCGGG | 107 | CGCUUCUGCUCGACUGCAATT | 557 |
| VEGFR-3 | 3903 | UUCAGCUACCUGAAGCCGCTT | 108 | GCGGCUUCAGGUAGCUGAATT | 558 |
| VEGFR-3 | 3273 | UACACCUUGUCGAAGAUGCTT | 109 | GCAUCUUCGACAAGGUGUATT | 559 |
| VEGFR-3 | 1107 | UACCACUGGAACUCGGGCGGG | 110 | CGCCCGAGUUCCAGUGGUATT | 560 |
| VEGFR-3 | 336 | UAGCAGACGUAGCUGCCUGTG | 111 | CAGGCAGCUACGUCUGCUATT | 561 |
| VEGFR-3 | 2607 | UUGUGGAUGCCGAAAGCGGAG | 112 | CCGCUUUCGGCAUCCACAATT | 562 |
| VEGFR-3 | 1556 | UCACAGUCUUAUUCUUUCCCT | 113 | GGAAAGAAUAAGACUGUGATT | 563 |
| VEGFR-3 | 108 | UCCGUGAUGUUCAAGGUCGGG | 114 | CGACCUUGAACAUCACGGATT | 564 |
| VEGFR-3 | 1954 | AUAGUGGCCCUCGUGCUCGGG | 115 | CGAGCACGAGGGCCACUAUTT | 565 |
| VEGFR-3 | 2100 | AAGCACUGCAUCUCCAGCGAG | 116 | CGCUGGAGAUGCAGUGCUUTT | 566 |
| VEGFR-3 | 693 | UCAUAGAGCUCGUUGCCUGTG | 117 | CAGGCAACGAGCUCUAUGATT | 567 |
| VEGFR-3 | 2337 | AGGAUCACGAUCUCCAUGCTG | 118 | GCAUGGAGAUCGUGAUCCUTT | 568 |
| VEGFR-3 | 2054 | UCAAGUUCUGCGUGAGCCGAG | 119 | CGGCUCACGCAGAACUUGATT | 569 |
| VEGFR-3 | 860 | UCUGUUGGGAGCGUCGCUCGG | 120 | GAGCGACGCUCCCAACAGATT | 570 |
| VEGFR-3 | 2436 | UAGCCCGUCUUGAUGUCUGCG | 121 | CAGACAUCAAGACGGGCUATT | 571 |
| VEGFR-3 | 3759 | UUCAUCCUGGAGGAACCACGG | 122 | GUGGUUCCUCCAGGAUGAATT | 572 |
| VEGFR-3 | 288 | AACACCUUGCAGUAGGGCCTG | 123 | GGCCCUACUGCAAGGUGUUTT | 573 |
| VEGFR-3 | 1485 | UGCGUGGUCACCGCCCUCCAG | 124 | GGAGGGCGGUGACCACGCATT | 574 |
| VEGFR-3 | 2502 | UCGUAGGACAGGUAUUCGCAT | 125 | GCGAAUACCUGUCCUACGATT | 575 |
| VEGFR-3 | 925 | AUACGAGCCCAGGUCGUGCTG | 126 | GCACGACCUGGGCUCGUAUTT | 576 |
| VEGFR-3 | 426 | UUGUUGAUGAAUGGCUGCUCA | 127 | AGCAGCCAUUCAUCAACAATT | 577 |
| VEGFR-3 | 3189 | UAGAUGUCCCGGGCAAGGCCA | 128 | GCCUUGCCCGGGACAUCUATT | 578 |
| VEGFR-3 | 2274 | UUGACGCAGCCCUUGGGUCTG | 129 | GACCCAAGGGCUGCGUCAATT | 579 |
| VEGFR-3 | 2196 | UUCUGGUUGGAGUCCGCCAAG | 130 | UGGCGGACUCCAACCAGAATT | 580 |
| VEGFR-3 | 2019 | UGCACCGACAGGUACUUCUTG | 131 | AGAAGUACCUGUCGGUGCATT | 581 |
| VEGFR-3 | 360 | AUGCGUGCCUUGAUGUACUTG | 132 | AGUACAUCAAGGCACGCAUTT | 582 |
| VEGFR-3 | 1755 | UACUUGUAGCUGUCGGCUUGG | 133 | AAGCCGACAGCUACAAGUATT | 583 |
| VEGFR-3 | 3037 | UUCCAUGGUCAGCGGGCUCAG | 134 | GAGCCCGCUGACCAUGGAATT | 584 |
| VEGFR-3 | 1018 | UUUGAGCCACUCGACGCUGAT | 135 | CAGCGUCGAGUGGCUCAAATT | 585 |
| VEGFR-3 | 1684 | UUCGAUGGUGAAGCCGUCGGG | 136 | CGACGGCUUCACCAUCGAATT | 586 |
| VEGFR-3 | 4373 | UACCAAGGAAUAAUCGGCGGG | 137 | CGCCGAUUAUUCCUUGGUATT | 587 |
| VEGFR-3 | 987 | UCAUGCACAAUGACCUCGGTG | 138 | CCGAGGUCAUUGUGCAUGATT | 588 |
| VEGFR-3 | 3267 | UUGUCGAAGAUGCUUUCAGGG | 139 | CUGAAAGCAUCUUCGACAATT | 589 |
| VEGFR-3 | 4387 | UGUAUUACUCAUAUUACCAAG | 140 | UGGUAAUAUGAGUAAUACATT | 590 |
| VEGFR-3 | 3883 | UUCUUGUCUAUGCCUGCUCTC | 141 | GAGCAGGCAUAGACAAGAATT | 591 |
| VEGFR-3 | 4376 | UAUUACCAAGGAAUAAUCGGC | 142 | CGAUUAUUCCUUGGUAAUATT | 592 |
| VEGFR-3 | 2140 | UUUGUACCACACGAUGCUGGG | 143 | CAGCAUCGUGUGGUACAAATT | 593 |
| VEGFR-3 | 978 | AUGACCUCGGUGCUCUCCCGA | 144 | GGGAGAGCACCGAGGUCAUTT | 594 |
| VEGFR-3 | 2427 | UUGAUGUCUGCGUGGGCCGGC | 145 | CGGCCCACGCAGACAUCAATT | 595 |
| VEGFR-3 | 1109 | UGUACCACUGGAACUCGGGCG | 146 | CCCGAGUUCCAGUGGUACATT | 596 |
| VEGFR-3 | 319 | UGUGUCGUUGGCAUGUACCTC | 147 | GGUACAUGCCAACGACACATT | 597 |
| VEGFR-3 | 1843 | AUGCACGUUCUUGCAGUCGAG | 148 | CGACUGCAAGAACGUGCAUTT | 598 |
| VEGFR-3 | 317 | UGUCGUUGGCAUGUACCUCGT | 149 | GAGGUACAUGCCAACGACATT | 599 |
| VEGFR-3 | 700 | CUGGAUGUCAUAGAGCUCGTT | 150 | CGAGCUCUAUGACAUCCAGTT | 600 |
| Tie-2 (TEK) | 1223 | UAAGCUUACAAUCUGGCCCGT | 151 | GGGCCAGAUUGUAAGCUUATT | 601 |
| Tie-2 (TEK) | 2350 | UAUCUUCACAUCAACGUGCTG | 152 | GCACGUUGAUGUGAAGAUATT | 602 |
| Tie-2 (TEK) | 706 | UAUGUUCACGUUAUCUCCCTT | 153 | GGGAGAUAACGUGAACAUATT | 603 |
| Tie-2 (TEK) | 3561 | UUUAAGGACACCAAUAUCUGG | 154 | AGAUAUUGGUGUCCUUAAATT | 604 |
| Tie-2 (TEK) | 2763 | UGAAAUUUGAUGUCAUUCCAG | 155 | GGAAUGACAUCAAAUUUCATT | 605 |
| Tie-2 (TEK) | 174 | UUGUUUACAAGUUAGAGGCAA | 156 | GCCUCUAACUUGUAAACAATT | 606 |
| Tie-2 (TEK) | 1183 | UUCAUUGCACUGCAGACCCTT | 157 | GGGUCUGCAGUGCAAUGAATT | 607 |
| Tie-2 (TEK) | 805 | UAGAAUAUCAGGUACUUCATG | 158 | UGAAGUACCUGAUAUUCUATT | 608 |
| Tie-2 (TEK) | 2601 | UUCAAUUGCAAUAUGAUCAGA | 159 | UGAUCAUAUUGCAAUUGAATT | 609 |
| Tie-2 (TEK) | 2277 | UAGCCAUCCAAUAUUGUCCAA | 160 | GGACAAUAUUGGAUGGCUATT | 610 |
| Tie-2 (TEK) | 1366 | UACUUCUAUAUGAUCUGGCAA | 161 | GCCAGAUCAUAUAGAAGUATT | 611 |
| Tie-2 (TEK) | 32 | UUUGGUAUCAGCAGGGCUGGG | 162 | CAGCCCUGCUGAUACCAAATT | 612 |
| Tie-2 (TEK) | 4085 | UGUACUAUCAGGGUCAUUGTT | 163 | CAAUGACCCUGAUAGUACATT | 613 |
| Tie-2 (TEK) | 3881 | UUCUGAUUUCAGCCCAUUCTT | 164 | GAAUGGGCUGAAAUCAGAATT | 614 |
| Tie-2 (TEK) | 646 | UUGUUGACGCAUCUUCAUGGT | 165 | CAUGAAGAUGCGUCAACAATT | 615 |
| Tie-2 (TEK) | 4021 | AUAGCAUUCAACAUAAAGGTA | 166 | CCUUUAUGUUGAAUGCUAUTT | 616 |
| Tie-2 (TEK) | 209 | UUUGUGACUUUCCAUUAGCAT | 167 | GCUAAUGGAAAGUCACAAATT | 617 |
| Tie-2 (TEK) | 4223 | UAAAUGAAACGGGACUGGCTG | 168 | GCCAGUCCCGUUUCAUUUATT | 618 |
| Tie-2 (TEK) | 3961 | UACUAAUUGUACUCACGCCTT | 169 | GGCGUGAGUACAAUUAGUATT | 619 |
| Tie-2 (TEK) | 1771 | UUGAAUAUGUUGCCAAGCCTC | 170 | GGCUUGGCAACAUAUUCAATT | 620 |
| Tie-2 (TEK) | 3909 | UUAUUGCAUAUGAAACCACAA | 171 | GUGGUUUCAUAUGCAAUAATT | 621 |
| Tie-2 (TEK) | 3606 | UAAAGCGUGGUAUUCACGUAG | 172 | ACGUGAAUACCACGCUUUATT | 622 |
| Tie-2 (TEK) | 477 | AUUAAGGCUUCAAAGUCCCTT | 173 | GGGACUUUGAAGCCUUAAUTT | 623 |
| Tie-2 (TEK) | 3421 | UUCUGCACAAGUCAUCCCGCA | 174 | CGGGAUGACUUGUGCAGAATT | 624 |
| Tie-2 (TEK) | 2730 | UAAAUUGUAGGAUCUGGGUTG | 175 | ACCCAGAUCCUACAAUUUATT | 625 |
| Tie-2 (TEK) | 1800 | UAGUUGAGUGUAACAAUCUCA | 176 | AGAUUGUUACACUCAACUATT | 626 |
| Tie-2 (TEK) | 3385 | UAAGCUAACAAUCUCCCAUAG | 177 | AUGGGAGAUUGUUAGCUUATT | 627 |
| Tie-2 (TEK) | 1692 | UAAGGCUCAGAGCUGAUGUTG | 178 | ACAUCAGCUCUGAGCCUUATT | 628 |
| Tie-2 (TEK) | 1657 | AUGUCCAGUGUCAAUCACGTT | 179 | CGUGAUUGACACUGGACAUTT | 629 |
| Tie-2 (TEK) | 3665 | UUCUGUCCUAGGCCGCUUCTT | 180 | GAAGCGGCCUAGGACAGAATT | 630 |
| Tie-2 (TEK) | 2091 | UUAAGUAGCACCGAAGUCAAG | 181 | UGACUUCGGUGCUACUUAATT | 631 |
| Tie-2 (TEK) | 2827 | UAACCCAUCCUUCUUGAUGCG | 182 | CAUCAAGAAGGAUGGGUUATT | 632 |
| Tie-2 (TEK) | 1979 | UUGGUUGCCAGGUCAAAUUTA | 183 | AAUUUGACCUGGCAACCAATT | 633 |
| Tie-2 (TEK) | 67 | UAGAUUAGGAUGGGAAAGGCT | 184 | CCUUUCCCAUCCUAAUCUATT | 634 |
| Tie-2 (TEK) | 3459 | UUCUCCAGUCUGUAGCCCUGG | 185 | AGGGCUACAGACUGGAGAATT | 635 |
| Tie-2 (TEK) | 2764 | UUGAAAUUUGAUGUCAUUCCA | 186 | GAAUGACAUCAAAUUUCAATT | 636 |
| Tie-2 (TEK) | 3560 | UUAAGGACACCAAUAUCUGGG | 187 | CAGAUAUUGGUGUCCUUAATT | 637 |
| Tie-2 (TEK) | 715 | UUUGAAAGAUAUGUUCACGTT | 188 | CGUGAACAUAUCUUUCAAATT | 638 |
| Tie-2 (TEK) | 1368 | UUUACUUCUAUAUGAUCUGGC | 189 | CAGAUCAUAUAGAAGUAAATT | 639 |
| Tie-2 (TEK) | 2351 | UUAUCUUCACAUCAACGUGCT | 190 | CACGUUGAUGUGAAGAUAATT | 640 |
| Tie-2 (TEK) | 205 | UGACUUUCCAUUAGCAUCGTC | 191 | CGAUGCUAAUGGAAAGUCATT | 641 |
| Tie-2 (TEK) | 3957 | AAUUGUACUCACGCCUUCCTA | 192 | GGAAGGCGUGAGUACAAUUTT | 642 |
| Tie-2 (TEK) | 3962 | AUACUAAUUGUACUCACGCCT | 193 | GCGUGAGUACAAUUAGUAUTT | 643 |
| Tie-2 (TEK) | 2352 | UUUAUCUUCACAUCAACGUGC | 194 | ACGUUGAUGUGAAGAUAAATT | 644 |
| Tie-2 (TEK) | 3963 | UAUACUAAUUGUACUCACGCC | 195 | CGUGAGUACAAUUAGUAUATT | 645 |
| Tie-2 (TEK) | 1777 | UGUCACUUGAAUAUGUUGCCA | 196 | GCAACAUAUUCAAGUGACATT | 646 |
| Tie-2 (TEK) | 3388 | UCCUAAGCUAACAAUCUCCCA | 197 | GGAGAUUGUUAGCUUAGGATT | 647 |
| Tie-2 (TEK) | 636 | AUCUUCAUGGUUCGUAUCCTG | 198 | GGAUACGAACCAUGAAGAUTT | 648 |
| Tie-2 (TEK) | 74 | UCCUUUGUAGAUUAGGAUGGG | 199 | CAUCCUAAUCUACAAAGGATT | 649 |
| Tie-2 (TEK) | 707 | AUAUGUUCACGUUAUCUCCCT | 200 | GGAGAUAACGUGAACAUAUTT | 650 |
| bFGFR | 3814 | UAAAUCUCUGGUAACGACCCT | 201 | GGUCGUUACCAGAGAUUUATT | 651 |
| bFGFR | 1478 | UUACACAUGAACUCCACGUTG | 202 | ACGUGGAGUUCAUGUGUAATT | 652 |
| bFGFR | 3773 | UAUACUCAGAUUUAUCAACTT | 203 | GUUGAUAAAUCUGAGUAUATT | 653 |
| bFGFR | 715 | UAGCGGUGCAGAGUGUGGCTG | 204 | GCCACACUCUGCACCGCUATT | 654 |
| bFGFR | 575 | UUCAAACUGACCCUCGCUCGG | 205 | GAGCGAGGGUCAGUUUGAATT | 655 |
| bFGFR | 646 | UUCUGCAGUUAGAGGUUGGTG | 206 | CCAACCUCUAACUGCAGAATT | 656 |
| bFGFR | 3625 | AUCGGAAUUAAUAAGCCACTG | 207 | GUGGCUUAUUAAUUCCGAUTT | 657 |
| bFGFR | 2318 | UACAAGGGACCAUCCUGCGTG | 208 | CGCAGGAUGGUCCCUUGUATT | 658 |
| bFGFR | 1439 | UUGUUGGCGGGCAACCCUGCT | 209 | CAGGGUUGCCCGCCAACAATT | 659 |
| bFGFR | 3860 | AUAGCAACUGAUGCCUCCCAG | 210 | GGGAGGCAUCAGUUGCUAUTT | 660 |
| bFGFR | 3163 | UGAGGGUUACAGCUGACGGTG | 211 | CCGUCAGCUGUAACCCUCATT | 661 |
| bFGFR | 2600 | UCGAUGUGGUGAAUGUCCCGT | 212 | GGGACAUUCACCACAUCGATT | 662 |
| bFGFR | 2513 | UCUCGGUGUAUGCACUUCUTG | 213 | AGAAGUGCAUACACCGAGATT | 663 |
| bFGFR | 2214 | UUUCUCUGUUGCGUCCGACTT | 214 | GUCGGACGCAACAGAGAAATT | 664 |
| bFGFR | 1346 | UUCUCCACAAUGCAGGUGUAG | 215 | ACACCUGCAUUGUGGAGAATT | 665 |
| bFGFR | 1556 | UUGUCUGGGCCAAUCUUGCTC | 216 | GCAAGAUUGGCCCAGACAATT | 666 |
| bFGFR | 2671 | UCCGGUCAAAUAAUGCCUCGG | 217 | GAGGCAUUAUUUGACCGGATT | 667 |
| bFGFR | 3105 | UUUGAGUCCGCCAUUGGCAAG | 218 | UGCCAAUGGCGGACUCAAATT | 668 |
| bFGFR | 2091 | UUUGCCUAAGACCAGUCUGTC | 219 | CAGACUGGUCUUAGGCAAATT | 669 |
| bFGFR | 1590 | UCCAGCAGUCUUCAAGAUCTG | 220 | GAUCUUGAAGACUGCUGGATT | 670 |
| bFGFR | 1689 | UCCGAUAGAGUUACCCGCCAA | 221 | GGCGGGUAACUCUAUCGGATT | 671 |
| bFGFR | 1319 | UUGUCAGAGGGCACCACAGAG | 222 | CUGUGGUGCCCUCUGACAATT | 672 |
| bFGFR | 2342 | UUGGAGGCAUACUCCACGATG | 223 | UCGUGGAGUAUGCCUCCAATT | 673 |
| bFGFR | 107 | UCUCGGUCCCGACCGGACGTG | 224 | CGUCCGGUCGGGACCGAGATT | 674 |
| bFGFR | 3662 | UCUGGUACCAGGCAUUUGGTC | 225 | CCAAAUGCCUGGUACCAGATT | 675 |
| bFGFR | 2150 | UUGUCCAGCCCGAUAGCCUCT | 226 | AGGCUAUCGGGCUGGACAATT | 676 |
| bFGFR | 1517 | UUUAGCCACUGGAUGUGCGGC | 227 | CGCACAUCCAGUGGCUAAATT | 677 |
| bFGFR | 1264 | UGUAGCCUCCAAUUCUGUGGT | 228 | CACAGAAUUGGAGGCUACATT | 678 |
| bFGFR | 3576 | UUCAAUCGUGGCUCGAAGCAC | 229 | GCUUCGAGCCACGAUUGAATT | 679 |
| bFGFR | 613 | AUCUCCAUGGAUACUCCACAG | 230 | GUGGAGUAUCCAUGGAGAUTT | 680 |
| bFGFR | 1221 | UUUCAACCAGCGCAGUGUGGG | 231 | CACACUGCGCUGGUUGAAATT | 681 |
| bFGFR | 3004 | UAGAGCUCCGGGUGUCGGGAA | 232 | CCCGACACCCGGAGCUCUATT | 682 |
| bFGFR | 3825 | UUACCGAUGGGUAAAUCUCTG | 233 | GAGAUUUACCCAUCGGUAATT | 683 |
| bFGFR | 3813 | AAAUCUCUGGUAACGACCCTT | 234 | GGGUCGUUACCAGAGAUUUTT | 684 |
| bFGFR | 3861 | UAUAGCAACUGAUGCCUCCCA | 235 | GGAGGCAUCAGUUGCUAUATT | 685 |
| bFGFR | 576 | UUUCAAACUGACCCUCGCUCG | 236 | AGCGAGGGUCAGUUUGAAATT | 686 |
| bFGFR | 3772 | AUACUCAGAUUUAUCAACUTT | 237 | AGUUGAUAAAUCUGAGUAUTT | 687 |
| bFGFR | 3824 | UACCGAUGGGUAAAUCUCUGG | 238 | AGAGAUUUACCCAUCGGUATT | 688 |
| bFGFR | 2319 | AUACAAGGGACCAUCCUGCGT | 239 | GCAGGAUGGUCCCUUGUAUTT | 689 |
| bFGFR | 3771 | UACUCAGAUUUAUCAACUUTG | 240 | AAGUUGAUAAAUCUGAGUATT | 690 |
| bFGFR | 2511 | UCGGUGUAUGCACUUCUUGGA | 241 | CAAGAAGUGCAUACACCGATT | 691 |
| bFGFR | 2333 | UACUCCACGAUGACAUACAAG | 242 | UGUAUGUCAUCGUGGAGUATT | 692 |
| bFGFR | 3624 | UCGGAAUUAAUAAGCCACUGG | 243 | AGUGGCUUAUUAAUUCCGATT | 693 |
| bFGFR | 1304 | ACAGAGUCCAUUAUGAUGCTC | 244 | GCAUCAUAAUGGACUCUGUTT | 694 |
| bFGFR | 1608 | UUUGUCGGUGGUAUUAACUCC | 245 | AGUUAAUACCACCGACAAATT | 695 |
| bFGFR | 1301 | GAGUCCAUUAUGAUGCUCCAG | 246 | GGAGCAUCAUAAUGGACUCTT | 696 |
| bFGFR | 3626 | UAUCGGAAUUAAUAAGCCACT | 247 | UGGCUUAUUAAUUCCGAUATT | 697 |
| bFGFR | 2672 | AUCCGGUCAAAUAAUGCCUCG | 248 | AGGCAUUAUUUGACCGGAUTT | 698 |
| bFGFR | 2213 | UUCUCUGUUGCGUCCGACUTC | 249 | AGUCGGACGCAACAGAGAATT | 699 |
| bFGFR | 2597 | AUGUGGUGAAUGUCCCGUGCG | 250 | CACGGGACAUUCACCACAUTT | 700 |
| IL8RA | 1971 | UUUAUUAGGAACAUCUGCCTG | 251 | GGCAGAUGUUCCUAAUAAATT | 701 |
| IL8RA | 75 | UUGAUCUAACUGAAGCACCGG | 252 | GGUGCUUCAGUUAGAUCAATT | 702 |
| IL8RA | 645 | AUUGUUUGGAUGGUAAGCCTG | 253 | GGCUUACCAUCCAAACAAUTT | 703 |
| IL8RA | 1431 | UAAUUAGCCAGUUAGUGGGTT | 254 | CCCACUAACUGGCUAAUUATT | 704 |
| IL8RA | 1378 | UUCGUUUCCAUGGAGGUGCAA | 255 | GCACCUCCAUGGAAACGAATT | 705 |
| IL8RA | 1470 | UCAUCUAAUGUCAGAUUCGGG | 256 | CGAAUCUGACAUUAGAUGATT | 706 |
| IL8RA | 218 | UACUUGUUGAGUGUCUCAGTT | 257 | CUGAGACACUCAACAAGUATT | 707 |
| IL8RA | 1101 | AUGACGUGCCAAGAACUCCTT | 258 | GGAGUUCUUGGCACGUCAUTT | 708 |
| IL8RA | 677 | UUUCCCAGGACCUCAUAGCAA | 259 | GCUAUGAGGUCCUGGGAAATT | 709 |
| IL8RA | 1178 | AAGAGAUAUUCCUUCAUCGAT | 260 | CGAUGAAGGAAUAUCUCUUTT | 710 |
| IL8RA | 1543 | UUGAGGAGAUGCUCCUGUGAG | 261 | CACAGGAGCAUCUCCUCAATT | 711 |
| IL8RA | 1783 | UCUUGUGGCAUAGAUCUGGCT | 262 | CCAGAUCUAUGCCACAAGATT | 712 |
| IL8RA | 1249 | AUAGUGCCUGUCCAGAGCCAG | 263 | GGCUCUGGACAGGCACUAUTT | 713 |
| IL8RA | 1520 | UCAACGAGAGCAUCCAGCCCT | 264 | GGCUGGAUGCUCUCGUUGATT | 714 |
| IL8RA | 1068 | AUGCAUAGCCAGGAUCUUGAG | 265 | CAAGAUCCUGGCUAUGCAUTT | 715 |
| IL8RA | 1347 | UUGGAGGUACCUCAACAGCTC | 266 | GCUGUUGAGGUACCUCCAATT | 716 |
| IL8RA | 1208 | UCAGGGUGUUGGUUAUUCUTT | 267 | AGAAUAACCAACACCCUGATT | 717 |
| IL8RA | 117 | AUCUGUAAUAUUUGACAUGTC | 268 | CAUGUCAAAUAUUACAGAUTT | 718 |
| IL8RA | 1862 | UGCUUGUCUCGUUCCACUUGG | 269 | AAGUGGAACGAGACAAGCATT | 719 |
| IL8RA | 1153 | UUCAGAGGUUGGAAGAGACAT | 270 | GUCUCUUCCAACCUCUGAATT | 720 |
| IL8RA | 640 | UUGGAUGGUAAGCCUGGCGGA | 271 | CGCCAGGCUUACCAUCCAATT | 721 |
| IL8RA | 1411 | UAAAGAUGUGACGUUCAACGG | 272 | GUUGAACGUCACAUCUUUATT | 722 |
| ILBRA | 71 | UCUAACUGAAGCACCGGCCAG | 273 | GGCCGGUGCUUCAGUUAGATT | 723 |
| IL8RA | 1397 | UCAACGGGAAUGAUGGUGCTT | 274 | GCACCAUCAUUCCCGUUGATT | 724 |
| IL8RA | 644 | UUGUUUGGAUGGUAAGCCUGG | 275 | AGGCUUACCAUCCAAACAATT | 725 |
| ILBRA | 641 | UUUGGAUGGUAAGCCUGGCGG | 276 | GCCAGGCUUACCAUCCAAATT | 726 |
| IL8RA | 76 | UUUGAUCUAACUGAAGCACCG | 277 | GUGCUUCAGUUAGAUCAAATT | 727 |
| IL8RA | 1398 | UUCAACGGGAAUGAUGGUGCT | 278 | CACCAUCAUUCCCGUUGAATT | 728 |
| IL8RA | 1381 | UGCUUCGUUUCCAUGGAGGTG | 279 | CCUCCAUGGAAACGAAGCATT | 729 |
| IL8RA | 1769 | UCUGGCUUCCAAACCCUCUTT | 280 | AGAGGGUUUGGAAGCCAGATT | 730 |
| IL8RA | 1435 | AUGCUAAUUAGCCAGUUAGTG | 281 | CUAACUGGCUAAUUAGCAUTT | 731 |
| IL8RA | 1175 | AGAUAUUCCUUCAUCGAUGGT | 282 | CAUCGAUGAAGGAAUAUCUTT | 732 |
| IL8RA | 1970 | UUAUUAGGAACAUCUGCCUGC | 283 | AGGCAGAUGUUCCUAAUAATT | 733 |
| IL8RA | 1432 | CUAAUUAGCCAGUUAGUGGGT | 284 | CCACUAACUGGCUAAUUAGTT | 734 |
| IL8RA | 74 | UGAUCUAACUGAAGCACCGGC | 285 | CGGUGCUUCAGUUAGAUCATT | 735 |
| IL8RA | 646 | AAUUGUUUGGAUGGUAAGCCT | 286 | GCUUACCAUCCAAACAAUUTT | 736 |
| IL8RA | 639 | UGGAUGGUAAGCCUGGCGGAA | 287 | CCGCCAGGCUUACCAUCCATT | 737 |
| IL8RA | 1082 | UUGCUGACCAGGCCAUGCATA | 288 | UGCAUGGCCUGGUCAGCAATT | 738 |
| IL8RA | 1770 | AUCUGGCUUCCAAACCCUCTT | 289 | GAGGGUUUGGAAGCCAGAUTT | 739 |
| IL8RA | 81 | AAUGGUUUGAUCUAACUGAAG | 290 | UCAGUUAGAUCAAACCAUUTT | 740 |
| IL8RA | 1372 | UCCAUGGAGGUGCAAAGGCCG | 291 | GCCUUUGCACCUCCAUGGATT | 741 |
| IL8RA | 1388 | AUGAUGGUGCUUCGUUUCCAT | 292 | GGAAACGAAGCACCAUCAUTT | 742 |
| IL8RA | 643 | UGUUUGGAUGGUAAGCCUGGC | 293 | CAGGCUUACCAUCCAAACATT | 743 |
| IL8RA | 1784 | UUCUUGUGGCAUAGAUCUGGC | 294 | CAGAUCUAUGCCACAAGAATT | 744 |
| IL8RA | 1524 | AGGGUCAACGAGAGCAUCCAG | 295 | GGAUGCUCUCGUUGACCCUTT | 745 |
| IL8RA | 237 | AUAGGCGAUGAUCACAACATA | 296 | UGUUGUGAUCAUCGCCUAUTT | 746 |
| IL8RA | 219 | AUACUUGUUGAGUGUCUCAGT | 297 | UGAGACACUCAACAAGUAUTT | 747 |
| IL8RA | 1389 | AAUGAUGGUGCUUCGUUUCCA | 298 | GAAACGAAGCACCAUCAUUTT | 748 |
| IL8RA | 1972 | CUUUAUUAGGAACAUCUGCCT | 299 | GCAGAUGUUCCUAAUAAAGTT | 749 |
| IL8RA | 1115 | UAGGAGGUAACACGAUGACGT | 300 | GUCAUCGUGUUACCUCCUATT | 750 |
| IL8RB | 2648 | UUAAGUGUCAAUUUAGUGGCA | 301 | CCACUAAAUUGACACUUAATT | 751 |
| IL8RB | 2184 | UUUCUUGUGGGUCAAUUCCTA | 302 | GGAAUUGACCCACAAGAAATT | 752 |
| IL8RB | 2250 | UUGGGUCUUGUGAAUAAGGTG | 303 | GCUUAUUCACAAGACCCAATT | 753 |
| IL8RB | 1746 | UUCACUUCUUAGAACAUAGAG | 304 | CUAUGUUCUAAGAAGUGAATT | 754 |
| IL8RB | 960 | UUGGAUGAGUAGACGGUCCTT | 305 | GGACCGUCUACUCAUCCAATT | 755 |
| IL8RB | 454 | AUUACUAAGAUCUUCACCUTT | 306 | AGGUGAAGAUCUUAGUAAUTT | 756 |
| IL8RB | 2750 | UUGGUUUAAUCAGCCUUGGTG | 307 | CCAAGGCUGAUUAAACCAATT | 757 |
| IL8RB | 2604 | AUCACUACUGUUUAUCUGCAG | 308 | GCAGAUAAACAGUAGUGAUTT | 758 |
| IL8RB | 1026 | AUCCGUAACAGCAUCCGCCAG | 309 | GGCGGAUGCUGUUACGGAUTT | 759 |
| IL8RB | 1384 | AUGUAUAGCUAGAAUCUUGAG | 310 | CAAGAUUCUAGCUAUACAUTT | 760 |
| IL8RB | 1149 | AAGAUGACCCGCAUGGCCCGG | 311 | GGGCCAUGCGGGUCAUCUUTT | 761 |
| IL8RB | 2464 | UCUCAGUACCUCAUGUAGGTG | 312 | CCUACAUGAGGUACUGAGATT | 762 |
| IL8RB | 877 | UUUGACCAAGUAGCGCUUCTG | 313 | GAAGCGCUACUUGGUCAAATT | 763 |
| IL8RB | 2324 | UUCGUUAGGUACAUAUCACAT | 314 | GUGAUAUGUACCUAACGAATT | 764 |
| IL8RB | 2360 | AUGAGUACUUCAUUCCUCUTT | 315 | AGAGGAAUGAAGUACUCAUTT | 765 |
| IL8RB | 265 | UUGGGUGGUAGUCAGAGCUGT | 316 | AGCUCUGACUACCACCCAATT | 766 |
| IL8RB | 1642 | UUUCUAAACCAUGCAAGGGAA | 317 | CCCUUGCAUGGUUUAGAAATT | 767 |
| IL8RB | 2146 | UCAUGUGUUAAUUCUAUGUCT | 318 | ACAUAGAAUUAACACAUGATT | 768 |
| IL8RB | 2627 | UUAAGUCACAUUGCGGUACAA | 319 | GUACCGCAAUGUGACUUAATT | 769 |
| IL8RB | 1000 | UGUAUUGUUGCCCAUGUCCTC | 320 | GGACAUGGGCAACAAUACATT | 770 |
| IL8RB | 315 | UGACCUGCUGUUAUUGGAGTG | 321 | CUCCAAUAACAGCAGGUCATT | 771 |
| IL8RB | 2774 | AAAUAUAGGCAGGUGGUUCTA | 322 | GAACCACCUGCCUAUAUUUTT | 772 |
| IL8RB | 219 | ACCUUGACGAUGAAACUUCTG | 323 | GAAGUUUCAUCGUCAAGGUTT | 773 |
| IL8RB | 2389 | UUUCAAGGUUCGUCCGUGUTG | 324 | ACACGGACGAACCUUGAAATT | 774 |
| IL8RB | 385 | UGAGGUAAACUUAAAUCCUGA | 325 | AGGAUUUAAGUUUACCUCATT | 775 |
| IL8RB | 1347 | UUCUGGCCAAUGAAGGCGUAG | 326 | ACGCCUUCAUUGGCCAGAATT | 776 |
| IL8RB | 2649 | UUUAAGUGUCAAUUUAGUGGC | 327 | CACUAAAUUGACACUUAAATT | 777 |
| IL8RB | 1737 | UAGAACAUAGAGUGCCAUGGG | 328 | CAUGGCACUCUAUGUUCUATT | 778 |
| IL8RB | 455 | AAUUACUAAGAUCUUCACCTT | 329 | GGUGAAGAUCUUAGUAAUUTT | 779 |
| IL8RB | 965 | UAACAUUGGAUGAGUAGACGG | 330 | GUCUACUCAUCCAAUGUUATT | 780 |
| IL8RB | 1740 | UCUUAGAACAUAGAGUGCCAT | 331 | GGCACUCUAUGUUCUAAGATT | 781 |
| IL8RB | 2632 | UGGCAUUAAGUCACAUUGCGG | 332 | GCAAUGUGACUUAAUGCCATT | 782 |
| IL8RB | 2755 | UAGCCUUGGUUUAAUCAGCCT | 333 | GCUGAUUAAACCAAGGCUATT | 783 |
| IL8RB | 2183 | UUCUUGUGGGUCAAUUCCUAT | 334 | AGGAAUUGACCCACAAGAATT | 784 |
| IL8RB | 2605 | UAUCACUACUGUUUAUCUGCA | 335 | CAGAUAAACAGUAGUGAUATT | 785 |
| IL8RB | 2340 | UCAGGCUGAAGGAUACUUCGT | 336 | GAAGUAUCCUUCAGCCUGATT | 786 |
| IL8RB | 2143 | UGUGUUAAUUCUAUGUCUGAA | 337 | CAGACAUAGAAUUAACACATT | 787 |
| IL8RB | 998 | UAUUGUUGCCCAUGUCCUCAT | 338 | GAGGACAUGGGCAACAAUATT | 788 |
| IL8RB | 2180 | UUGUGGGUCAAUUCCUAUAAG | 339 | UAUAGGAAUUGACCCACAATT | 789 |
| IL8RB | 2185 | AUUUCUUGUGGGUCAAUUCCT | 340 | GAAUUGACCCACAAGAAAUTT | 790 |
| IL8RB | 307 | UGUUAUUGGAGUGGCCACCGA | 341 | GGUGGCCACUCCAAUAACATT | 791 |
| IL8RB | 2481 | UCUGUAAAUUUGUUCACUCTC | 342 | GAGUGAACAAAUU UACAGATT | 792 |
| IL8RB | 2617 | UUGCGGUACAACUAUCACUAC | 343 | AGUGAUAGUUGUACCGCAATT | 793 |
| IL8RB | 956 | AUGAGUAGACGGUCCUUCGGA | 344 | CGAAGGACCGUCUACUCAUTT | 794 |
| IL8RB | 456 | UAAUUACUAAGAUCUUCACCT | 345 | GUGAAGAUCUUAGUAAUUATT | 795 |
| IL8RB | 226 | UGAAACAACCUUGACGAUGAA | 346 | CAUCGUCAAGGUUGUUUCATT | 796 |
| IL8RB | 1394 | UGAUCAAGCCAUGUAUAGCTA | 347 | GCUAUACAUGGCUUGAUCATT | 797 |
| IL8RB | 458 | UGUAAUUACUAAGAUCUUCAC | 348 | GAAGAUCUUAGUAAUUACATT | 798 |
| IL8RB | 881 | UGAAUUUGACCAAGUAGCGCT | 349 | CGCUACUUGGUCAAAUUCATT | 799 |
| IL8RB | 2327 | UACUUCGUUAGGUACAUAUCA | 350 | AUAUGUACCUAACGAAGUATT | 800 |
| Fas | 109 | UGUAGUAACAGUCUUCCUCAA | 351 | GAGGAAGACUGUUACUACATT | 801 |
| Fas | 41 | UGGACGAUAAUCUAGCAACAG | 352 | GUUGCUAGAUUAUCGUCCATT | 802 |
| Fas | 161 | UAUGGCAGAAUUGGCCAUCAT | 353 | GAUGGCCAAUUCUGCCAUATT | 803 |
| Fas | 182 | UUUCACCUGGAGGACAGGGCT | 354 | CCCUGUCCUCCAGGUGAAATT | 804 |
| Fas | 62 | UCACUUGGGCAUUAACACUTT | 355 | AGUGUUAAUGCCCAAGUGATT | 805 |
| Fas | 377 | ACUUCCUCUUUGCACUUGGTG | 356 | CCAAGUGCAAAGAGGAAGUTT | 806 |
| Fas | 349 | UGAGUGUGCAUUCCUUGAUGA | 357 | AUCAAGGAAUGCACACUCATT | 807 |
| Fas | 245 | UCCCUUCUUGGCAGGGCACGC | 358 | GUGCCCUGCCAAGAAGGGATT | 808 |
| Fas | 205 | GACUGUGCAGUCCCUAGCUTT | 359 | AGCUAGGGACUGCACAGUCTT | 809 |
| Fas | 145 | AUCAUGAUGCAGGCCUUCCAA | 360 | GGAAGGCCUGCAUCAUGAUTT | 810 |
| Fas | 123 | UUCUGAGUCUCAACUGUAGTA | 361 | CUACAGUUGAGACUCAGAATT | 811 |
| Fas | 34 | UAAUCUAGCAACAGACGUAAG | 362 | UACGUCUGUUGCUAGAUUATT | 812 |
| Fas | 114 | UCAACUGUAGUAACAGUCUTC | 363 | AGACUGUUACUACAGUUGATT | 813 |
| Fas | 115 | CUCAACUGUAGUAACAGUCTT | 364 | GACUGUUACUACAGUUGAGTT | 814 |
| Fas | 28 | AGCAACAGACGUAAGAACCAG | 365 | GGUUCUUACGUCUGUUGCUTT | 815 |
| Fas | 122 | UCUGAGUCUCAACUGUAGUAA | 366 | ACUACAGUUGAGACUCAGATT | 816 |
| Fas | 186 | UUCCUUUCACCUGGAGGACAG | 367 | GUCCUCCAGGUGAAAGGAATT | 817 |
| Fas | 42 | UUGGACGAUAAUCUAGCAACA | 368 | UUGCUAGAUUAUCGUCCAATT | 818 |
| Fas | 111 | ACUGUAGUAACAGUCUUCCTC | 369 | GGAAGACUGUUACUACAGUTT | 819 |
| Fas | 144 | UCAUGAUGCAGGCCUUCCAAG | 370 | UGGAAGGCCUGCAUCAUGATT | 820 |
| Fas | 92 | UCAAUUCCAAUCCCUUGGAGT | 371 | UCCAAGGGAUUGGAAUUGATT | 821 |
| Fas | 201 | GUGCAGUCCCUAGCUUUCCTT | 372 | GGAAAGCUAGGGACUGCACTT | 822 |
| Fas | 128 | CCAAGUUCUGAGUCUCAACTG | 373 | GUUGAGACUCAGAACUUGGTT | 823 |
| Fas | 36 | GAUAAUCUAGCAACAGACGTA | 374 | CGUCUGUUGCUAGAUUAUCTT | 824 |
| Fas | 162 | UUAUGGCAGAAUUGGCCAUCA | 375 | AUGGCCAAUUCUGCCAUAATT | 825 |
| Fas | 127 | CAAGUUCUGAGUCUCAACUGT | 376 | AGUUGAGACUCAGAACUUGTT | 826 |
| Fas | 202 | UGUGCAGUCCCUAGCUUUCCT | 377 | GAAAGCUAGGGACUGCACATT | 827 |
| Fas | 82 | UCCCUUGGAGUUGAUGUCAGT | 378 | UGACAUCAACUCCAAGGGATT | 828 |
| Fas | 160 | AUGGCAGAAUUGGCCAUCATG | 379 | UGAUGGCCAAUUCUGCCAUTT | 829 |
| Fas | 150 | UGGCCAUCAUGAUGCAGGCCT | 380 | GCCUGCAUCAUGAUGGCCATT | 830 |
| Fas | 63 | GUCACUUGGGCAUUAACACTT | 381 | GUGUUAAUGCCCAAGUGACTT | 831 |
| Fas | 164 | GCUUAUGGCAGAAUUGGCCAT | 382 | GGCCAAUUCUGCCAUAAGCTT | 832 |
| Fas | 37 | CGAUAAUCUAGCAACAGACGT | 383 | GUCUGUUGCUAGAUUAUCGTT | 833 |
| Fas | 116 | UCUCAACUGUAGUAACAGUCT | 384 | ACUGUUACUACAGUUGAGATT | 834 |
| Fas | 32 | AUCUAGCAACAGACGUAAGAA | 385 | CUUACGUCUGUUGCUAGAUTT | 835 |
| Fas | 64 | AGUCACUUGGGCAUUAACACT | 386 | UGUUAAUGCCCAAGUGACUTT | 836 |
| Fas | 167 | AGGGCUUAUGGCAGAAUUGGC | 387 | CAAUUCUGCCAUAAGCCCUTT | 837 |
| Fas | 120 | UGAGUCUCAACUGUAGUAACA | 388 | UUACUACAGUUGAGACUCATT | 836 |
| Fas | 125 | AGUUCUGAGUCUCAACUGUAG | 389 | ACAGUUGAGACUCAGAACUTT | 839 |
| Fas | 43 | UUUGGACGAUAAUCUAGCAAC | 390 | UGCUAGAUUAUCGUCCAAATT | 840 |
| Fas | 94 | CCUCAAUUCCAAUCCCUUGGA | 391 | CAAGGGAUUGGAAUUGAGGTT | 841 |
| Fas | 159 | UGGCAGAAUUGGCCAUCAUGA | 392 | AUGAUGGCCAAUUCUGCCATT | 842 |
| Fas | 110 | CUGUAGUAACAGUCUUCCUCA | 393 | AGGAAGACUGUUACUACAGTT | 843 |
| Fas | 31 | UCUAGCAACAGACGUAAGAAC | 394 | UCUUACGUCUGUUGCUAGATT | 844 |
| Fas | 38 | ACGAUAAUCUAGCAACAGACG | 395 | UCUGUUGCUAGAUUAUCGUTT | 845 |
| Fas | 118 | AGUCUCAACUGUAGUAACAGT | 396 | UGUUACUACAGUUGAGACUTT | 846 |
| Fas | 169 | ACAGGGCUUAUGGCAGAAUTG | 397 | AUUCUGCCAUAAGCCCUGUTT | 847 |
| Fas | 33 | AAUCUAGCAACAGACGUAAGA | 398 | UUACGUCUGUUGCUAGAUUTT | 848 |
| Fas | 163 | CUUAUGGCAGAAUUGGCCATC | 399 | UGGCCAAUUCUGCCAUAAGTT | 849 |
| Fas | 233 | AGGGCACGCAGUCUGGUUCAT | 400 | GAACCAGACUGCGUGCCCUTT | 850 |
| IGF2R | 6340 | UUUGUCACCUAUGACACCCAG | 401 | GGGUGUCAUAGGUGACAAATT | 851 |
| IGF2R | 2936 | UUAUAGAGCAAGCCUGGUCTG | 402 | GACCAGGCUUGCUCUAUAATT | 852 |
| IGF2R | 1331 | UCUGAUUGUGGUAUCUUCCTG | 403 | GGAAGAUACCACAAUCAGATT | 853 |
| IGF2R | 4491 | UAUUUCAGGACAAUUAUGCCA | 404 | GCAUAAUUGUCCUGAAAUATT | 854 |
| IGF2R | 2562 | UUAAUGUAGUAUUUCCUCCAC | 405 | GGAGGAAAUACUACAUUAATT | 855 |
| IGF2R | 1456 | UUUCCCAUCGUUACCUGCGGT | 406 | CGCAGGUAACGAUGGGAAATT | 856 |
| IGF2R | 2253 | UAGUUCAGUUGGAUCAUCCCA | 407 | GGAUGAUCCAACUGAACUATT | 857 |
| IGF2R | 3570 | UUGCCUUCUGACACUAAGCAA | 408 | GCUUAGUGUCAGAAGGCAATT | 858 |
| IGF2R | 2274 | UUAUAGGGUGUGCCGCCUCTG | 409 | GAGGCGGCACACCCUAUAATT | 859 |
| IGF2R | 1197 | UUUCCAUCUGAAAUAUAGGAT | 410 | CCUAUAUUUCAGAUGGAAATT | 860 |
| IGF2R | 897 | UUGCGCACCAGCUUCAGUCCG | 411 | GACUGAAGCUGGUGCGCAATT | 861 |
| IGF2R | 5205 | UUGAUGUAGAAAUCAGGGUTG | 412 | ACCCUGAUUUCUACAUCAATT | 862 |
| IGF2R | 8904 | UUCUCAGCAAUAGAACACCAG | 413 | GGUGUUCUAUUGCUGAGAATT | 863 |
| IGF2R | 8604 | UAAGGCUUCUUAUAGGUCGAA | 414 | CGACCUAUAAGAAGCCUUATT | 864 |
| IGF2R | 3629 | UCAAAGAUCCAUUCGCCGCGG | 415 | GCGGCGAAUGGAUCUUUGATT | 865 |
| IGF2R | 4344 | UUGAUGAGGUAGUGCUCCGGG | 416 | CGGAGCACUACCUCAUCAATT | 866 |
| IGF2R | 1419 | UUUAUGACGCUCAUCCGCUGA | 417 | AGCGGAUGAGCGUCAUAAATT | 867 |
| IGF2R | 7185 | UAUUUGUAGGACACGUUGGAA | 418 | CCAACGUGUCCUACAAAUATT | 868 |
| IGF2R | 4447 | UACCCUGCCGAGGUUCACGGG | 419 | CGUGAACCUCGGCAGGGUATT | 869 |
| IGF2R | 3706 | UAUCUGAGCACACUCAAACGT | 420 | GUUUGAGUGUGCUCAGAUATT | 870 |
| IGF2R | 6422 | UCUUUGUACAGGUCAAUUCTA | 421 | GAAUUGACCUGUACAAAGATT | 871 |
| IGF2R | 1306 | UUUGACUUGAGAGGUAUCGCT | 422 | CGAUACCUCUCAAGUCAAATT | 872 |
| IGF2R | 6129 | UUGUGUUUCUGGACGAAUUTG | 423 | AAUUCGUCCAGAAACACAATT | 873 |
| IGF2R | 5105 | UAGAGCUUCCAUUCCUCACGG | 424 | GUGAGGAAUGGAAGCUCUATT | 874 |
| IGF2R | 4572 | UUCACUUGGCUCUCGCUGCAG | 425 | GCAGCGAGAGCCAAGUGAATT | 875 |
| IGF2R | 5308 | UACCCGGCCGAUAUCUAUGGG | 426 | CAUAGAUAUCGGCCGGGUATT | 876 |
| IGF2R | 3153 | UUCUCAAUUCCGACUGGCCTT | 427 | GGCCAGUCGGAAUUGAGAATT | 877 |
| IGF2R | 9029 | UAUUACAGUAAAGUUGAUUGA | 428 | AAUCAACUUUACUGUAAUATT | 878 |
| IGF2R | 1530 | UUAACACAGGCGUAUUCCGTG | 429 | CGGAAUACGCCUGUGUUAATT | 879 |
| IGF2R | 8364 | AAAUGUGCUCUGUACGCCCAG | 430 | GGGCGUACAGAGCACAUUUTT | 880 |
| IGF2R | 5400 | UAGUUGAAAUGCUUGUCCGCT | 431 | CGGACAAGCAUUUCAACUATT | 881 |
| IGF2R | 6702 | UUGGCUCCAGAGCACGCCGGG | 432 | CGGCGUGCUCUGGAGCCAATT | 882 |
| IGF2R | 8479 | UUCUCUGACACCUCAACUCCA | 433 | GAGUUGAGGUGUCAGAGAATT | 883 |
| IGF2R | 4723 | UAAGGAGCUCAGAUCAAACAG | 434 | GUUUGAUCUGAGCUCCUUATT | 884 |
| IGF2R | 4237 | UGAACAUUCAGUCAGAUCGAA | 435 | CGAUCUGACUGAAUGUUCATT | 885 |
| IGF2R | 6203 | UAUAGUACGAGACUCCGUUGT | 436 | AACGGAGUCUCGUACUAUATT | 886 |
| IGF2R | 753 | AUGAAUAGAGAAGUGUCCGGA | 437 | CGGACACUUCUCUAUUCAUTT | 887 |
| IGF2R | 8554 | AUAAGCACAGUAAAGGUGGTA | 438 | CCACCUUUACUGUGCUUAUTT | 888 |
| IGF2R | 5462 | UUAACAGCUUAGGCGUUCCCA | 439 | GGAACGCCUAAGCUGUUAATT | 889 |
| IGF2R | 1460 | UUCCUUUCCCAUCGUUACCTG | 440 | GGUAACGAUGGGAAAGGAATT | 890 |
| IGF2R | 5206 | AUUGAUGUAGAAAUCAGGGTT | 441 | CCCUGAUUUCUACAUCAAUTT | 891 |
| IGF2R | 2559 | AUGUAGUAUUUCCUCCACGTG | 442 | CGUGGAGGAAAUACUACAUTT | 892 |
| IGF2R | 8605 | UUAAGGCUUCUUAUAGGUCGA | 443 | GACCUAUAAGAAGCCUUAATT | 893 |
| IGF2R | 4345 | AUUGAUGAGGUAGUGCUCCGG | 444 | GGAGCACUACCUCAUCAAUTT | 894 |
| IGF2R | 1187 | AAAUAUAGGAUGAACCUCCGC | 445 | GGAGGUUCAUCCUAUAUUUTT | 895 |
| IGF2R | 1184 | UAUAGGAUGAACCUCCGCUCT | 446 | AGCGGAGGUUCAUCCUAUATT | 896 |
| IGF2R | 7190 | UUGAGUAUUUGUAGGACACGT | 447 | GUGUCCUACAAAUACUCAATT | 897 |
| IGF2R | 7182 | UUGUAGGACACGUUGGAACTT | 448 | GUUCCAACGUGUCCUACAATT | 898 |
| IGF2R | 2941 | AUCCCUUAUAGAGCAAGCCTG | 449 | GGCUUGCUCUAUAAGGGAUTT | 899 |
| IGF2R | 3693 | UCAAACGUGAUCCUGGUGGAG | 450 | CCACCAGGAUCACGUUUGATT | 900 |

| | | | | | |
|---|---|---|---|---|---|
| Chemical Modification of RNA Strand Nucleotides | | | | | |

The siRNA according to the invention may comprise at least one modified nucleotide in at least one of the RNA strands. A range of potential modified nucleotides are disclosed elsewhere herein. Useful modifications and combinations of modifications for use according to the invention are shown in **Table 2:**

**Table 2: Chemical Modifications and Sequence Architecture**

| # | Modification Name | Format |
|---|---|---|
| 1 | PS DNA o/h | NNNNNNNNNNNNNNNNNNNsnsn |
| | | nsnsNNNNNNNNNNNNNNNNNNN |
| 2 | Full PS | NsNsNsNsNsNsNsNsNsNsNsNsNsNsNsNsNsNsNsNsN |
| | | NsNsNsNsNsNsNsNsNsNsNsNsNsNsNsNsNsNsNsNsN |
| 3 | RNA o/h | NNNNNNNNNNNNNNNNNNNNN |
| | | NNNNNNNNNNNNNNNNNNNNN |
| 4 | Blunt-ended | NNNNNNNNNNNNNNNNNNN |
| | | NNNNNNNNNNNNNNNNNNN |
| 5 | 2'-OMe o/h | NNNNNNNNNNNNNNNNNNNNN^{p}N^{p} |
| | | N^{p}N^{p}NNNNNNNNNNNNNN |
| 6 | 2'-OMe/2'F | NNNNNNNNNNNNNNNNNNNN^{p}N^{p} |
| | | N^{p}N^{p}NNNNNNNNNNNNNNNNNNN |
| 7 | LNA (3-7 incorporations in ds region) | NNNNNNNNNNNNNNNNNNNsnsn |
| | | nsnsNNNNNNNNNNNNNNNNNNN |

| | | |
|---|---|---|
| N = any unmodified RNA nucleotide n = unmodified DNA nucleotide N^{p} = modified RNA nucleotide s = identifies phosphorthioate internucleoside linkage o/h = overhang | | |

The following modifications added to the 3' position of the 3'-terminus of the siRNA strands , sometimes referred to as a '3' end cap' are also recognized as useful embodiments of the invention and may be used with any of the siRNA according to the invention: 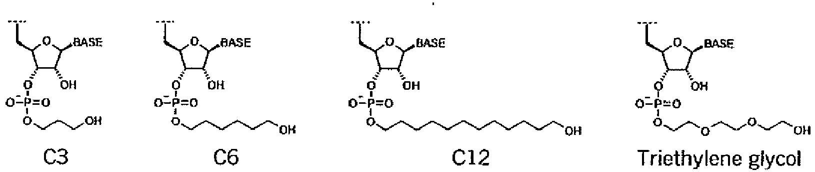 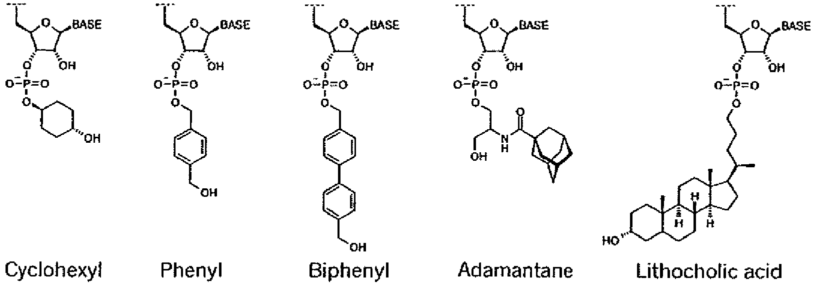 Specific compounds with activity according to the invention include the following, shown in Table 3:

**Table 3: Sequences and Chemistries of siRNA used in Examples**

| Name | strand | Sequence (N: RNA; dN; DNA; n: 2'-moe RNA; s: phosphorothioate) | SEQ ID NO |
|---|---|---|---|
| pGl3-siRNA | guide strand | UCG AAG UAC UCA GCG UAA GdTdT | 901 |
| | complement strand | CUU ACG CUG AGU ACU UCG AdTdT | 902 |
| | | | |
| pGL3 MOE o/h siRNA | guide strand | CUU ACG CUG AGU ACU UCG Atst | 903 |
| | complement strand | UCG AAG UAC UCA GCG UAA Gtst | 904 |
| | | | |
| pGl3-C3-siRNA | guide strand | UCG AAG UAC UCA GCG UAA G-C3 | 905 |
| | complement strand | CUU ACG CUG AGU ACU UCG A-C3 | 906 |
| | | | |
| pGl3-C3-MOE-siRNA | guide strand | UCG AAG UAC UCA GCG UAa g-C3 | 907 |
| | complement strand | CUU ACG CUG AGU ACU UCg a-C3 | 908 |
| VEGFR2-siRNA1 | guide strand | UUG AGG UUU GAA AUC GAC CdCdT | 909 |
| | complement strand | GGU CGA UUU CAA ACC UCA AdTdT | 910 |
| | | | |
| VEGFR2-siRNA2 | guide strand | UAA UUU GUU CCU GUC UUC CdAdG | 911 |
| | complement strand | GGA AGA CAG GAA CAA AUU AdTdT | 912 |
| | | | |
| siRNA control | guide strand | ACG UGA CAC GUU CGG AGA AdTdT | 913 |
| | complement strand | UUC UCC GAA CGU GUC ACG UdTdT | 914 |
| | | | |
| VEGFR2-C3-siRNA1 | guide strand | UUG AGG UUU GAA AUC GAC C-C3 | 915 |
| | complement strand | GGU CGA UUU CAA ACC UCA A-C3 | 916 |
| | | | |
| VEGFR2-C3-siRNA2 | guide strand | UAA UUU GUU CCU GUC UUC C-C3 | 917 |
| | complement strand | GGA AGA CAG GAA CAA AUU A-C3 | 918 |
| C3-siRNA control | guide strand | ACG UGA CAC GUU CGG AGA A-C3 | 919 |
| | complement strand | UUC UCC GAA CGU GUC ACG U-C3 | 920 |
| | | | |
| VEGFR2-C3-MOE-siRNA1 | guide strand | UUG AGG UUU GAA AUC GAc c-C3 | 921 |
| | complement strand | GGU CGA UUU CAA ACC UCa a-C3 | 922 |
| | | | |
| VEGFR2-C3-MOE-siRNA2 | guide strand | UAA UUU GUU CCU GUC UUc c-C3 | 923 |
| | complement strand | GGA AGA CAG GAA CAA AUu a-C3 | 924 |
| | | | |
| Tie2-C3-MOE-siRNA1 | guide strand | UUC UUC UUU AAU UAA CAc c-C3 | 925 |
| | complement strand | GGU GUU AAU UAA AGA AGa a-C3 | 926 |
| | | | |
| Tie2-C3-MOE-siRNA2 | guide strand | UCU GAG UUU GUA AAU AUc g-C3 | 927 |
| | complement strand | CGA UAU UUA CAA ACU CAg a-C3 | 928 |
| | | | |
| C3-MOE-siRNA control | guide strand | ACG UGA CAC GUU CGG AGa a-C3 | 929 |
| | complement strand | UUC UCC GAA CGU GUC ACg t-C3 | 930 |

### Examples:

The following Examples illustrate aspects of the application, and are not intended to limit the embodiments included in the claims recited below. The results and discussion section further below refers to experiments conducted according to the following protocols and employing the following materials. Materials and protocols that are not specifically described are considered to be routinely available to those skilled in the art.

### Example 1

### Preparation of siRNAs.

Single strand siRNA derivatives were synthesized by standard 2'-O-TOM phosphoamidite technology and purified by Oasis® HLB Extraction Plates (Waters). Sense- and antisense stranded siRNA were mixed in hybridization buffer (100 mM potassium acetate, 2 mM magnesium acetate, 30 mM Hepes, pH 7.6) heat-denatured at 90° C for 3 min and annealed at 37° C for 60 min. 100 µM stock solutions of siRNA duplexes were stored at -20° C.

### Example 2

### Incubation in Serum and analysis by IE-HPLC (LC-MS).

In a standard serum assay, 6 µL 20 µM of each siRNA were mixed with 54 µL serum or CSF and heated at 37° C in an incubator. 50 µL of the cooled mixture was loaded on an analytical DNA-pac PA-100 Column (Dionex) and analyzed with a NaCl gradient (0 - 0.6 M in 30 min) in a 1:10 Acetonitrile:Buffer (20 mM sodium acetate, 1 mM magnesium acetate, pH 6.5) solution.

For LC-MS analysis 100 µL (20 µM or 50 µM) each siRNA was mixed with 900 µL sterile fetal bovine serum (GIBCO) incubated at 37° C and separated by HPLC as indicated previously (except of the NaCl gradient: 0M - 0.36M in 9'/0.36M - 0.6M in 12'). Degradation products were desalted on NAP columns and analyzed by LC-ESI⁻-MS.

### Example 3

### Incubation in gastric acid

To prepare a standard gastric acid assay, FVB and C57BL6 mice, weighing 18 to 20 g (6 to 8 weeks old), were obtained from Charles River Laboratories (Les Oncins, France). Animals were sacrificed using CO₂, and then stomachs were quickly recovered. Gastric fluid as well as stomach contents were collected and pooled, then loaded on centrifugal filter devices (Ultrafree MC, Millipores). Filter units were spun for 10 minutes according to manufacturer's recommendations. The filtrate, corresponding to mouse gastric fluid, was recovered, aliquoted and frozen prior further experiments.
For each assay, 20 µM of siRNA solutions were diluted in 9x volume of gastric acid as above described and incubated at 37°C for 0, 5, 10, 15, 30, 60 and 120 min.

### Example 4

### Incubation in intestinal lavage

To prepare a standard intestinal lavage assay, Male Wistar rat were fasted, anesthetized with isoflurane. Intestinal lavage was obtained by in situ perfusion of the small intestine (duodenum, jejunum, ileum) with 10 mL saline (0.5 mL/min) followed by 20 mL water (1 mL/min). Outlet collected was centrifuged (3000 x g, 15 min, 22°C), and supernatant passed through a 1.2-µm filter and stored at -20°C.
For each assay, 20 µM siRNA solutions were diluted in 9x volume of intestinal lavage and incubated at 37°C for 0, 15, 30, 60, 180 and 360 min.

### Example 5

### Incubation in mouse liver microsomes

In a standard liver microsome assay, to 10 µl of a 250 µM solution of siRNA were added 25 µl of mouse liver microsomes (GEntest 452701 Charge 11) at 20 mg protein /ml, 365 µl of 100 mM phosphate buffer (pH 7.4), 50 µl of UDPGA cofactor (24 mM in water), 50 µl of NADPH. Incubation was quenched by freezing at t=0 min and t= 60 min.

### Example 6

### Incubation in rat S12 supernatant

For a standard rat S12 supernatant assay, 10 µl of a 250 µM solution of siRNA were added to 17 µl of rat liver S12 at 29.9 mg protein /ml, 373 µl of 100 mM phosphate buffer (pH 7.4), 50 µl of UDPGA cofactor (24 mM in water), 50 µl of NADPH. Incubation was quenched by freezing at t=0 min and t= 60 min.

### Example 7

### Incubation in mouse serum

For a standard incubation in mouse serum, 20 µM siRNA solutions were diluted in 9x volume of murine serum (Harlan nude mouse) and incubated at 37°C for 0, 15, 30, 60, 180 and 360 min.

### Example 8

### Gel electrophoresis stability assay.

A 10 µL aliquot of incubation solution was taken immediately after shaking and shock-frozen on dry ice, the mixtures were incubated at 37° C and aliquots were shock frozen at various time points. Aliquots were thawed in 30 µL (15 µL respectively) Loading Buffer (Elchrom Sc., Cham, Switzerland) and separated on a SF50 gels (Elchrom Sc., Cham, Switzerland) at 120 V, 8° C for 240 min. Bands were stained with SYBR Gold (Molecular Probes) and picture were taken with a BIORAD ChemiDoc™ XRS system.

### Example 9

### Cell culture

The mouse immortalized endothelial cell line MS1 (ATCC CRL-2279) was grown in DMEM high glucose (4.5g/l) supplemented with L-Glutamine and 10% heat-inactivated FCS (AMIMED, Switzerland) on 1.5% Gelatine-coated culture dishes. MS1 cells were transfected in 24 well-format with siRNA using HiPerfect (QIAGEN) according to manufacturer procedure (tetraplicate, final siRNA concentration was 10nM or as indicated).

### Example 10

### FACS analysis

Non-transfected and siRNA transfected MS1 cells were analyzed by FACS for VEGFR2 levels. Briefly, cells were trypsinized from duplicate or triplicate wells, pooled for each conditions, then washed twice with PBS+10% FCS and incubated 10 minutes on ice prior addition of RPE-conjugated anti-VEGFR2 Ab (1µg/10⁶ cells; Avas 12α1, BD Pharmingen). RPE-labeled isotype IgG2α were used as FACS control (BD Pharmingen). FACS acquisition and analysis were performed on a FACScalibur using Cell Quest Software (Becton-Dickinson).

### Example 11

### Animal studies

Female FVB mice (6 to 8 weeks old), were obtained from Charles River Laboratories (Les Oncins, France). Mice were identified via ear markings and kept in groups (6 animals per cage) under normal conditions and observed daily. Six mice were used per treatment group and all animal experiments were performed in strict adherence to the Swiss law for animal protection.

The reference chamber model has been described in publications (e.g. Wood J, Bold G, Buchdunger E, et al. PTK787/ZK 222584, a novel and potent inhibitor of vascular endothelial growth factor receptor tyrosine kinases, impairs vascular endothelial growth factor-induced responses and tumor growth after oral administration. Cancer Res 2000;60:2178-89) In brief, porous tissue chambers made of perfluoro-alkoxy-Teflon (Teflon®-PFA, 21 mm x 8 mm diameter, 550 µl volume) were filled with 0.8% agar (BBL® Nr. 11849, Becton Dickinson, Meylan, France) and 20U/ml heparin, (Novo Nordisk A/S, Bagsvaerd, Denmark) supplemented with or without 3 µg/ml recombinant human VEGF and siRNAs as indicated. Solutions were maintained at 42 °C prior the filling procedure. Mice were anesthetized using 3% Isoflurane (Forene®, Abbott AG, Cham, Switzerland) inhalation. For subcutaneous implantation, a small skin incision was made at the base of the tail to allow the insertion of an implant trocar. The chamber was implanted under aseptic conditions through the small incision onto the back of the animal. The skin incision was closed by wound clips (Autoclip 9 mm Clay Adams). Depending on the required dose, siRNAs were diluted in "injectable quality grade" 0.9% saline solution then delivered to animals either i.p. (200µL /dose) or p.o. by gavage (100µL /dose). The mice were receiving the first dose 2 to 4 hours before implanting chambers; then treated daily for 2 days. If not otherwise indicated, mice were sacrificed three days after implantation, chambers excised and the vascularized fibrous tissue formed around each implant carefully removed. Body weight was used to monitor the general condition of the mice. Statistical analysis was done using one-way ANOVA followed by Dunnett test.

### Example 12

### B16 melanoma xenograft model

The syngeneic B16/BL6 murine melanoma model, previously identified to be responsive to antiangiogenic therapy (e.g. LaMontagne K, Littlewood-Evans A, Schnell C, O'Reilly T, Wyder L, Sanchez T, Probst B, Butler J, Wood A, Liau G, Billy E, Theuer A, Hla T, Wood J. Antagonism of sphingosine-1-phosphate receptors by FTY720 inhibits angiogenesis and tumor vascularization. Cancer Res. 2006 Jan 1;66(1):221-31), was used to evaluate the antitumor activity of standard or modified siRNAs. Tumor cells (1 µL, 5 x 10⁴/µL) were injected intradermally into the dorsal pinna of both ears of syngeneic female C57BL/6 mice. Measurements of primary tumor area (mm²) were carried out on days 7, 14, and 21 after tumor cell inoculation using computer-assisted image analysis software (KS-400 3.0 imaging system, Zeiss) and a specifically designed macro. From days 7 to 21, mice were receiving siRNAs diluted in" injectable quality grade" 0.9% saline solution either i.p. (200µL /dose) or p.o. by gavage (100µL /dose) once a day. Mice were sacrificed on day 21, and cranial lymph node metastases were weighed and then frozen.

In these results, actual siRNA sequences and chemistries employed may be determined by reference to **Table 3.**

### Wild-type siRNAs are degraded in mouse serum from both 3'-ends

Oligonucleotide degradation by nucleases is predominantly 3'-exonucleolytic. Modification of antisense oligonucleotides at their termini by the introduction of aromatic or lipophilic residues delays their nucleolytic degradation¹⁷. To verify whether this metabolic pathway would also be dominant for siRNA, we incubated at 37°C a unmodified siRNA (wild-type siRNA) in mouse serum for up to 3 hours.

The unmodified siRNA sequence employed was pGl3-siRNA (see **Table 3**)

The mixtures were analyzed with Strong Anion Exchange HPLC at t=0 min., t=30 min, t= 180 min.

As shown in **Figure la, 1b and 1c,** at t= 30 min, a well defined peak corresponding to blunt ended siRNA was observed. By t=3h substantial degradation is observed. **Figure 1d** **and** **1e** illustrate the metabolites identified by HPLC-ESI-MS analysis. This analysis revealed the presence of several metabolites corresponding to the loss of the 3' overhangs and of the 3'- terminal first base pairing ribonucleotide on both strands. Digestion of the 5'-terminal ribonucleotide of the guide strand was also observed.

**Figure 1** suggests the degradation pathway of unmodified siRNAs in serum. DNA overhangs are first digested, possibly by 3'-exonucleases. In the LC-MS, additional metabolites were also detected which correspond to the loss of the first base-pairing 3'-ribonucleotide of both strands and also the first 5'-base-pairing ribonucleotide of the guide strand.

### 3'-modified siRNAs are stable through the GI tract

siRNAs with 2'-methoxyethyl ribonucleotides overhangs (MOE o/h siRNA), blunt-ended siRNAs 3'-capped with a hydroxypropoxy phosphodiester moiety (C3-siRNA), and hydroxypropoxy phosphodiester 3'-capped siRNAs where the two first base paring nucleotide at 3'-end of each strand were modified by 2'-methoxyethyl ribonucleotides residues (C3-MOE siRNA) were synthesized. These compounds are illustrated schematically in **Figure 2****.**

First siRNAs were incubated in mouse gastric acid for 2h (**Figure 3**). No degradation was observed in the cases of C3 siRNA and C3-MOE sRNA, while degradation of wild-type siRNA was observed after 30 minutes.

Stability in intestinal fluid obtained from intestinal lavage of rats revealed almost complete degradation of wild-type siRNA after 15 minutes whereas parent compound in the MOE o/h siRNA, C3-siRNA and C3-Moe siRNA were observed for 60 minutes. (**Figure 4**)

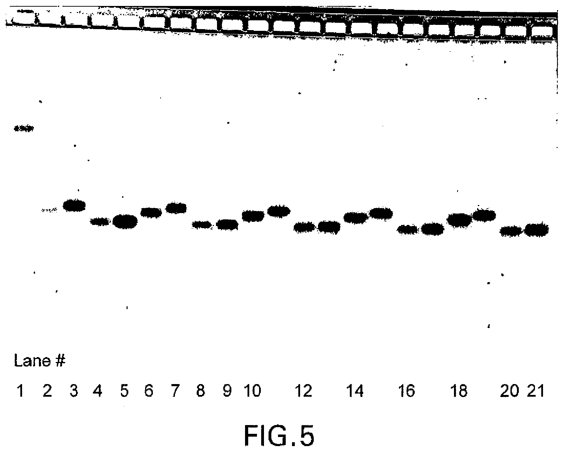

Stability in liver was evaluated using a liver microsome assay and a S12 assay (representative of liver cytosolic enzymatic activity). Results are shown in **Figure 5****.** In both cases, no degradation was observed after 60 minutes of incubation.

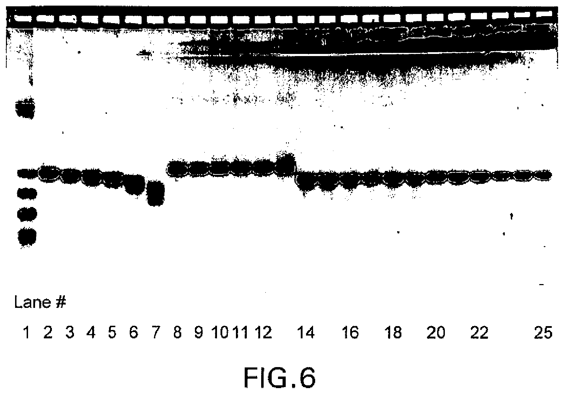

Finally, siRNAs were tested in mouse serum by incubation at 2 micromolar for up to 6 hours at 37°C (results in **Figure 6**). Parent compound stability was followed by gel electrophoresis. In the cases of modified siRNAs (C3 siRNA, C3-MOE siRNA of MOE o/h siRNA), no significant degradation was observed while the wild-type siRNA

This study indicate that wild type (unmodified) siRNAs are metabolized in mouse gastric acid and in mouse serum. In case of 3'-ends modified siRNAs, no degradation was observed in the GI tract. Therefore it is likely that 3'-modified siRNAs will have a higher oral bioavailability than wild-type siRNAs

### Systemically delivered 3'-modified siRNAs are more active in an in vivo growth factor induced angiogenesis model¹⁸.

Firstly, the ability of modified siRNAs (C3-siRNA and CE-MOE siRNA) to down regulate a target gene was checked in cellulo by measuring VEGFR2 surface level of MS1 cells transfected with anti-VEGFR2 siRNAs.

Pools of 2 anti VEGFR2 siRNAs as wild-type siRNAs, C3-siRNAs and C3-MOE siRNAs were administered intraperitoneally. Results are shown in **Figure 7****.** Pooled Wild type siRNAs reduced significantly the VEGF induced vascularization at the higher dose of 25 micrograms per mice per day. The same level of inhibition was observed at a 5-fold lower dose with C3-siRNA. In the case of the C3-MOE siRNAs pool, significant reduction of vascularized tissue weight was observed at all tested doses including the lowest 0.2 microgram per mouse per day.

**Figure 8a** **and** **8b** show that, when given intraperitoneally, both VEGFR2 - C3 and C3-MOE siRNAs were active at below 1 microgram per mouse per day dose.

*In vivo* testing of anti-VEGFR2 C3-MOE siRNA given intraperitoneally (i.p.) in a B16 homograft melanoma tumor mouse model. **Figure 9a** shows that i.p. treatment with modified VEGFR2-C3-MOE-siRNA significantly reduces tumour development. **Figure 9b** also shows that i.p. injection of VEGFR2-C3-MOE-siRNA at 20 ug per mouse results in significant inhibition of tumour growth.

### Oral Delivery of siRNA for Treatment of Angiogenic Disorders

**Figure 10** shows that given orally, at a dose of 20 micrograms per mouse per day, the VEGFR2-C3-MOE-siRNA 1 reduced vascularization weight down to basal level (e.g. weight without growth factor induction). Actual siRNA sequences used are referred to in **Table 3.**

Anti Tie2 C3-MOE siRNAs were also tested in the growth factor induced angiogenesis model under both intraperitoneal and oral deliveries. **Figure 11a** **and** **11b** show that given orally, both C3-MOE siRNAs directed at Tie2 were active at 20 microgram per mouse per day. Actual siRNA sequences used may be determined by reference to **Table 3.**

The data shows that 3'-end modified siRNAs with or without additional internal modifications are able to demonstrate therapeutic effect at reasonable doses upon oral administration.

### REFERENCES

1. a) Y. Tomari et al. Genes and Development 19 (2005), 517; b) P. Shankar et al. JAMA 11 (2005), 1367; c)Y. Dorsett et al. Nature Reviews 3 (2004), 318
2. a) P.D. Zamore et al. Cell 101, (2000), 25; b) S.M. Hammond et al. Nature 404 (2000), 293
3. a) G. Meister et al. Molecular Cell 15 (2004), 185.
4. S.M. Elbashir et al. Genes Dev. 15 (2001), 188.
5. S.J. Reich et al. Molecular Vision 9 (2003), 210.
6. a) Dom et al. Nucleic Acids Research 32 (2004), e49; b) D. R.Thakker et al. PNAS 101 (2004), 17270; c) D.R. Thakker et al. Molecular Psychiatry 10 (2005), 714
7. V. Bitko et al. Nature Medicine 11 (2005), 50.
8. E. Song et al. Nature Medicine 9 (2003), 347.
9. D.A.Braasch et al. Biochemistry 42 (2003), 7967.
10. Harborth, Antisense Nucleic Acid Drug Devt, 2003
11. A.H.S. Hall et al. Nucleic Acids Research 32 (2004), 5991.
12. M. Amarzguioui et al. Nucleic Acids Research 31 (2003), 589.
13. F. Czaudema et al. Nucleic Acids Research 31 (2003), 2705.
14. T. Prakash et al. Journal of Medicinal Chemistry 48 (2005), 4247.
15. J. Elmen et al. Nucleic Acids Research 33 (2005), 439.
16. A.S. Boutorin, L.V. Guskova, E.M. Ivanova, N.D. Kobetz, V.F. Zafytova, A.S. Ryte, L.V. Yurchenko and V.V. Vlassov FEBS Lett. 254 (1989), p. 129
17. J. Wood et al. Cancer Research 60 (2000), 2178.
18. K. LaMontagne et al. Cancer Res. 66 (2006), 221.

### SEQUENCE LISTING

<110> Novartis AG
<120> Short interfering ribonucleic acid (siRNA) for oral administration
<130> P108459EPAA
<140> PCT/EP2007/003867
   <141> 2007-05-02
<150> GB0608838.9
   <151> 2006-05-04
<160> 930
<170> PatentIn version 3.3
<210> 1
   <211> 21
   <212> DNA
   <213> homo sapiens
<400> 1
   uauaagaacu uguuaacugt g 21
<210> 2
   <211> 21
   <212> DNA
   <213> homo sapiens
<400> 2
   uacgguuuca agcaccugct g 21
<210> 3
   <211> 21
   <212> DNA
   <213> homo sapiens
<400> 3
   uuuaugcuca gcaagauugt a 21
<210> 4
   <211> 21
   <212> DNA
   <213> homo sapiens
<400> 4
   uuaucuuccu gaaagccgga g 21
<210> 5
   <211> 21
   <212> DNA
   <213> homo sapiens
<400> 5
   uugagggaua ccauaugcgg t 21
<210> 6
   <211> 21
   <212> DNA
   <213> homo sapiens
<400> 6
   uugauaauua acgaguagcc a 21
<210> 7
   <211> 21
   <212> DNA
   <213> homo sapiens
<400> 7
   uuaaccauac aacuuccggc g 21
<210> 8
   <211> 21
   <212> DNA
   <213> homo sapiens
<400> 8
   uuaggugacg uaacccggca g 21
<210> 9
   <211> 21
   <212> DNA
   <213> homo sapiens
<400> 9
   uugcucuuga gguaguugga g 21
<210> 10
   <211> 21
   <212> DNA
   <213> homo sapiens
<400> 10
   uuugucuuau acaaaugccc a 21
<210> 11
   <211> 21
   <212> DNA
   <213> homo sapiens
<400> 11
   uugacaauua gaguggcagt g 21
<210> 12
   <211> 21
   <212> DNA
   <213> homo sapiens
<400> 12
   uuauaauuga uagguaguca g 21
<210> 13
   <211> 21
   <212> DNA
   <213> homo sapiens
<400> 13
   uugaguaugu aaacccacua t 21
<210> 14
   <211> 21
   <212> DNA
   <213> homo sapiens
<400> 14
   uuccauagug augggcucct t 21
<210> 15
   <211> 21
   <212> DNA
   <213> homo sapiens
<400> 15
   ucuguuauua acuguccgca g 21
<210> 16
   <211> 21
   <212> DNA
   <213> homo sapiens
<400> 16
   uugggaugua gucuuuacca t 21
<210> 17
   <211> 21
   <212> DNA
   <213> homo sapiens
<400> 17
   uguuagagug aucagcucca g 21
<210> 18
   <211> 21
   <212> DNA
   <213> homo sapiens
<400> 18
   uuuccaucag ggaucaaagt g 21
<210> 19
   <211> 21
   <212> DNA
   <213> homo sapiens
<400> 19
   uugaacucuc guguucaagg g 21
<210> 20
   <211> 21
   <212> DNA
   <213> homo sapiens
<400> 20
   uagacuuguc cgagguucct t 21
<210> 21
   <211> 21
   <212> DNA
   <213> homo sapiens
<400> 21
   uugaggacaa gaguauggcc t 21
<210> 22
   <211> 21
   <212> DNA
   <213> homo sapiens
<400> 22
   uuacugguua cucucaaguc a 21
<210> 23
   <211> 21
   <212> DNA
   <213> homo sapiens
<400> 23
   uuccagcuca gcguggucgt a 21
<210> 24
   <211> 21
   <212> DNA
   <213> homo sapiens
<400> 24
   ugcuucggaa ugauuauggt t 21
<210> 25
   <211> 21
   <212> DNA
   <213> homo sapiens
<400> 25
   uugacuguug cuucacaggt c 21
<210> 26
   <211> 21
   <212> DNA
   <213> homo sapiens
<400> 26
   ucauccauuu guacuccugg g 21
<210> 27
   <211> 21
   <212> DNA
   <213> homo sapiens
<400> 27
   ugguuucuug ccuuguucca g 21
<210> 28
   <211> 21
   <212> DNA
   <213> homo sapiens
<400> 28
   uuaggcucca uguguagugc t 21
<210> 29
   <211> 21
   <212> DNA
   <213> homo sapiens
<400> 29
   ucuagaguca gccacaacca a 21
<210> 30
   <211> 21
   <212> DNA
   <213> homo sapiens
<400> 30
   uaauuaacga guagccacga g 21
<210> 31
   <211> 21
   <212> DNA
   <213> homo sapiens
<400> 31
   uaaccauaca acuuccggcg a 21
<210> 32
   <211> 21
   <212> DNA
   <213> homo sapiens
<400> 32
   uucacauuga caauuagagt g 21
<210> 33
   <211> 21
   <212> DNA
   <213> homo sapiens
<400> 33
   auguaaaccc acuauuucct g 21
<210> 34
   <211> 21
   <212> DNA
   <213> homo sapiens
<400> 34
   auccucuuca guuacgucct t 21
<210> 35
   <211> 21
   <212> DNA
   <213> homo sapiens
<400> 35
   uuguauaauu cccugcaucc t 21
<210> 36
   <211> 21
   <212> DNA
   <213> homo sapiens
<400> 36
   uuuaaccaua caacuuccgg c 21
<210> 37
   <211> 21
   <212> DNA
   <213> homo sapiens
<400> 37
   uacaaaugcc cauugacugt t 21
<210> 38
   <211> 21
   <212> DNA
   <213> homo sapiens
<400> 38
   auguuaggug acguaacccg g 21
<210> 39
   <211> 21
   <212> DNA
   <213> homo sapiens
<400> 39
   uaagucacgu uugcucuuga g 21
<210> 40
   <211> 21
   <212> DNA
   <213> homo sapiens
<400> 40
   uuugcucuug agguaguugg a 21
<210> 41
   <211> 21
   <212> DNA
   <213> homo sapiens
<400> 41
   uuuccuguca guauggcaut g 21
<210> 42
   <211> 21
   <212> DNA
   <213> homo sapiens
<400> 42
   uacuguagug cauuguucug t 21
<210> 43
   <211> 21
   <212> DNA
   <213> homo sapiens
<400> 43
   auaauuaacg aguagccacg a 21
<210> 44
   <211> 21
   <212> DNA
   <213> homo sapiens
<400> 44
   uuguagguug agggauacca t 21
<210> 45
   <211> 21
   <212> DNA
   <213> homo sapiens
<400> 45
   uugaacagug agguaugcug a 21
<210> 46
   <211> 21
   <212> DNA
   <213> homo sapiens
<400> 46
   uuuaccaucc uguuguacat t 21
<210> 47
   <211> 21
   <212> DNA
   <213> homo sapiens
<400> 47
   uuucacauug acaauuagag t 21
<210> 48
   <211> 21
   <212> DNA
   <213> homo sapiens
<400> 48
   auacuguagu gcauuguuct g 21
<210> 49
   <211> 21
   <212> DNA
   <213> homo sapiens
<400> 49
   uacucucaag ucaaucuuga g 21
<210> 50
   <211> 21
   <212> DNA
   <213> homo sapiens
<400> 50
   aaauaaguca cguuugcuct t 21
<210> 51
   <211> 21
   <212> DNA
   <213> homo sapiens
<400> 51
   uaauagacug guaacuuuca t 21
<210> 52
   <211> 21
   <212> DNA
   <213> homo sapiens
<400> 52
   uagaagguug accacauuga g 21
<210> 53
   <211> 21
   <212> DNA
   <213> homo sapiens
<400> 53
   uagcugauca uguagcuggg a 21
<210> 54
   <211> 21
   <212> DNA
   <213> homo sapiens
<400> 54
   uugcuguccc aggaaauuct g 21
<210> 55
   <211> 21
   <212> DNA
   <213> homo sapiens
<400> 55
   augauuucca aguucgucut t 21
<210> 56
   <211> 21
   <212> DNA
   <213> homo sapiens
<400> 56
   uaauguacac gacuccaugt t 21
<210> 57
   <211> 21
   <212> DNA
   <213> homo sapiens
<400> 57
   uucaucugga uccaugacga t 21
<210> 58
   <211> 21
   <212> DNA
   <213> homo sapiens
<400> 58
   ugauucucca gguuuccugt g 21
<210> 59
   <211> 21
   <212> DNA
   <213> homo sapiens
<400> 59
   uagaccguac augucagcgt t 21
<210> 60
   <211> 21
   <212> DNA
   <213> homo sapiens
<400> 60
   uucuggugua guauaaucag g 21
<210> 61
   <211> 21
   <212> DNA
   <213> homo sapiens
<400> 61
   uuucgugccg ccagguccct g 21
<210> 62
   <211> 21
   <212> DNA
   <213> homo sapiens
<400> 62
   uucuucacaa ggguaugggt t 21
<210> 63
   <211> 21
   <212> DNA
   <213> homo sapiens
<400> 63
   ucaauuucca aagaguaucc a 21
<210> 64
   <211> 21
   <212> DNA
   <213> homo sapiens
<400> 64
   uaguucaauu ccaugagacg g 21
<210> 65
   <211> 21
   <212> DNA
   <213> homo sapiens
<400> 65
   aacauggcaa ucaccgccgt g 21
<210> 66
   <211> 21
   <212> DNA
   <213> homo sapiens
<400> 66
   uccuucaaua caaugccuga g 21
<210> 67
   <211> 21
   <212> DNA
   <213> homo sapiens
<400> 67
   uacaaguuuc uuaugcugat g 21
<210> 68
   <211> 21
   <212> DNA
   <213> homo sapiens
<400> 68
   ugauaucgga agaacaaugt a 21
<210> 69
   <211> 21
   <212> DNA
   <213> homo sapiens
<400> 69
   ugugcuauua gagaacaugg t 21
<210> 70
   <211> 21
   <212> DNA
   <213> homo sapiens
<400> 70
   uucuacauca cugagggact t 21
<210> 71
   <211> 21
   <212> DNA
   <213> homo sapiens
<400> 71
   ucuuuaaacc acaugaucug t 21
<210> 72
   <211> 21
   <212> DNA
   <213> homo sapiens
<400> 72
   ucuugcacaa agugacacgt t 21
<210> 73
   <211> 21
   <212> DNA
   <213> homo sapiens
<400> 73
   ugauuauugg gccaaagcca g 21
<210> 74
   <211> 21
   <212> DNA
   <213> homo sapiens
<400> 74
   auuuguacaa agcugacaca t 21
<210> 75
   <211> 21
   <212> DNA
   <213> homo sapiens
<400> 75
   uaaauauccc gggccaagcc a 21
<210> 76
   <211> 21
   <212> DNA
   <213> homo sapiens
<400> 76
   aaccauacca cuguccguct g 21
<210> 77
   <211> 21
   <212> DNA
   <213> homo sapiens
<400> 77
   ugucaucgga gugauauccg g 21
<210> 78
   <211> 21
   <212> DNA
   <213> homo sapiens
<400> 78
   ucucaaacgu agaucuguct g 21
<210> 79
   <211> 21
   <212> DNA
   <213> homo sapiens
<400> 79
   uccuccacaa auccagagct g 21
<210> 80
   <211> 21
   <212> DNA
   <213> homo sapiens
<400> 80
   uaaaugaccg aggccaaguc a 21
<210> 81
   <211> 21
   <212> DNA
   <213> homo sapiens
<400> 81
   uaaccaaggu acuucgcagg g 21
<210> 82
   <211> 21
   <212> DNA
   <213> homo sapiens
<400> 82
   uaggcaaacc cacagaggcg g 21
<210> 83
   <211> 21
   <212> DNA
   <213> homo sapiens
<400> 83
   uggcaucaua aggcagucgt t 21
<210> 84
   <211> 21
   <212> DNA
   <213> homo sapiens
<400> 84
   uugaguggug ccguacuggt a 21
<210> 85
   <211> 21
   <212> DNA
   <213> homo sapiens
<400> 85
   uuuccaaaga guauccaagt t 21
<210> 86
   <211> 21
   <212> DNA
   <213> homo sapiens
<400> 86
   uugucgucug auucuccagg t 21
<210> 87
   <211> 21
   <212> DNA
   <213> homo sapiens
<400> 87
   uaagaggaua uuucgugccg c 21
<210> 88
   <211> 21
   <212> DNA
   <213> homo sapiens
<400> 88
   uauguacaua auagacuggt a 21
<210> 89
   <211> 21
   <212> DNA
   <213> homo sapiens
<400> 89
   uuacaaguuu cuuaugcuga t 21
<210> 90
   <211> 21
   <212> DNA
   <213> homo sapiens
<400> 90
   uaggucucgg uuuacaagut t 21
<210> 91
   <211> 21
   <212> DNA
   <213> homo sapiens
<400> 91
   uuaggucucg guuuacaagt t 21
<210> 92
   <211> 21
   <212> DNA
   <213> homo sapiens
<400> 92
   uccgauaaga ggauauuucg t 21
<210> 93
   <211> 21
   <212> DNA
   <213> homo sapiens
<400> 93
   uuuacaaguu ucuuaugcug a 21
<210> 94
   <211> 21
   <212> DNA
   <213> homo sapiens
<400> 94
   ucugauucuc cagguuucct g 21
<210> 95
   <211> 21
   <212> DNA
   <213> homo sapiens
<400> 95
   uucaauacaa ugccugaguc t 21
<210> 96
   <211> 21
   <212> DNA
   <213> homo sapiens
<400> 96
   uuuguugacc gcuucacaut t 21
<210> 97
   <211> 21
   <212> DNA
   <213> homo sapiens
<400> 97
   auagcugauc auguagcugg g 21
<210> 98
   <211> 21
   <212> DNA
   <213> homo sapiens
<400> 98
   uauccggacu gguagccgct t 21
<210> 99
   <211> 21
   <212> DNA
   <213> homo sapiens
<400> 99
   uacaugucag cguuugagug g 21
<210> 100
   <211> 21
   <212> DNA
   <213> homo sapiens
<400> 100
   uaucggaaga acaauguagt c 21
<210> 101
   <211> 21
   <212> DNA
   <213> homo sapiens
<400> 101
   uuccuguuga ccaagagcgt g 21
<210> 102
   <211> 21
   <212> DNA
   <213> homo sapiens
<400> 102
   uugagcuccg acaucagcgc g 21
<210> 103
   <211> 21
   <212> DNA
   <213> homo sapiens
<400> 103
   uuggauucga uggugaagcc g 21
<210> 104
   <211> 21
   <212> DNA
   <213> homo sapiens
<400> 104
   uucaugcaca augaccucgg t 21
<210> 105
   <211> 21
   <212> DNA
   <213> homo sapiens
<400> 105
   uuaccaagga auaaucggcg g 21
<210> 106
   <211> 21
   <212> DNA
   <213> homo sapiens
<400> 106
   ucuuuguacc acacgaugct g 21
<210> 107
   <211> 21
   <212> DNA
   <213> homo sapiens
<400> 107
   uugcagucga gcagaagcgg g 21
<210> 108
   <211> 21
   <212> DNA
   <213> homo sapiens
<400> 108
   uucagcuacc ugaagccgct t 21
<210> 109
   <211> 21
   <212> DNA
   <213> homo sapiens
<400> 109
   uacaccuugu cgaagaugct t 21
<210> 110
   <211> 21
   <212> DNA
   <213> homo sapiens
<400> 110
   uaccacugga acucgggcgg g 21
<210> 111
   <211> 21
   <212> DNA
   <213> homo sapiens
<400> 111
   uagcagacgu agcugccugt g 21
<210> 112
   <211> 21
   <212> DNA
   <213> homo sapiens
<400> 112
   uuguggaugc cgaaagcgga g 21
<210> 113
   <211> 21
   <212> DNA
   <213> homo sapiens
<400> 113
   ucacagucuu auucuuuccc t 21
<210> 114
   <211> 21
   <212> DNA
   <213> homo sapiens
<400> 114
   uccgugaugu ucaaggucgg g 21
<210> 115
   <211> 21
   <212> DNA
   <213> homo sapiens
<400> 115
   auaguggccc ucgugcucgg g 21
<210> 116
   <211> 21
   <212> DNA
   <213> homo sapiens
<400> 116
   aagcacugca ucuccagcga g 21
<210> 117
   <211> 21
   <212> DNA
   <213> homo sapiens
<400> 117
   ucauagagcu cguugccugt g 21
<210> 118
   <211> 21
   <212> DNA
   <213> homo sapiens
<400> 118
   aggaucacga ucuccaugct g 21
<210> 119
   <211> 21
   <212> DNA
   <213> homo sapiens
<400> 119
   ucaaguucug cgugagccga g 21
<210> 120
   <211> 21
   <212> DNA
   <213> homo sapiens
<400> 120
   ucuguuggga gcgucgcucg g 21
<210> 121
   <211> 21
   <212> DNA
   <213> homo sapiens
<400> 121
   uagcccgucu ugaugucugc g 21
<210> 122
   <211> 21
   <212> DNA
   <213> homo sapiens
<400> 122
   uucauccugg aggaaccacg g 21
<210> 123
   <211> 21
   <212> DNA
   <213> homo sapiens
<400> 123
   aacaccuugc aguagggcct g 21
<210> 124
   <211> 21
   <212> DNA
   <213> homo sapiens
<400> 124
   ugcgugguca ccgcccucca g 21
<210> 125
   <211> 21
   <212> DNA
   <213> homo sapiens
<400> 125
   ucguaggaca gguauucgca t 21
<210> 126
   <211> 21
   <212> DNA
   <213> homo sapiens
<400> 126
   auacgagccc aggucgugct g 21
<210> 127
   <211> 21
   <212> DNA
   <213> homo sapiens
<400> 127
   uuguugauga auggcugcuc a 21
<210> 128
   <211> 21
   <212> DNA
   <213> homo sapiens
<400> 128
   uagauguccc gggcaaggcc a 21
<210> 129
   <211> 21
   <212> DNA
   <213> homo sapiens
<400> 129
   uugacgcagc ccuuggguct g 21
<210> 130
   <211> 21
   <212> DNA
   <213> homo sapiens
<400> 130
   uucugguugg aguccgccaa g 21
<210> 131
   <211> 21
   <212> DNA
   <213> homo sapiens
<400> 131
   ugcaccgaca gguacuucut g 21
<210> 132
   <211> 21
   <212> DNA
   <213> homo sapiens
<400> 132
   augcgugccu ugauguacut g 21
<210> 133
   <211> 21
   <212> DNA
   <213> homo sapiens
<400> 133
   uacuuguagc ugucggcuug g 21
<210> 134
   <211> 21
   <212> DNA
   <213> homo sapiens
<400> 134
   uuccaugguc agcgggcuca g 21
<210> 135
   <211> 21
   <212> DNA
   <213> homo sapiens
<400> 135
   uuugagccac ucgacgcuga t 21
<210> 136
   <211> 21
   <212> DNA
   <213> homo sapiens
<400> 136
   uucgauggug aagccgucgg g 21
<210> 137
   <211> 21
   <212> DNA
   <213> homo sapiens
<400> 137
   uaccaaggaa uaaucggcgg g 21
<210> 138
   <211> 21
   <212> DNA
   <213> homo sapiens
<400> 138
   ucaugcacaa ugaccucggt g 21
<210> 139
   <211> 21
   <212> DNA
   <213> homo sapiens
<400> 139
   uugucgaaga ugcuuucagg g 21
<210> 140
   <211> 21
   <212> DNA
   <213> homo sapiens
<400> 140
   uguauuacuc auauuaccaa g 21
<210> 141
   <211> 21
   <212> DNA
   <213> homo sapiens
<400> 141
   uucuugucua ugccugcuct c 21
<210> 142
   <211> 21
   <212> DNA
   <213> homo sapiens
<400> 142
   uauuaccaag gaauaaucgg c 21
<210> 143
   <211> 21
   <212> DNA
   <213> homo sapiens
<400> 143
   uuuguaccac acgaugcugg g 21
<210> 144
   <211> 21
   <212> DNA
   <213> homo sapiens
<400> 144
   augaccucgg ugcucucccg a 21
<210> 145
   <211> 21
   <212> DNA
   <213> homo sapiens
<400> 145
   uugaugucug cgugggccgg c 21
<210> 146
   <211> 21
   <212> DNA
   <213> homo sapiens
<400> 146
   uguaccacug gaacucgggc g 21
<210> 147
   <211> 21
   <212> DNA
   <213> homo sapiens
<400> 147
   ugugucguug gcauguacct c 21
<210> 148
   <211> 21
   <212> DNA
   <213> homo sapiens
<400> 148
   augcacguuc uugcagucga g 21
<210> 149
   <211> 21
   <212> DNA
   <213> homo sapiens
<400> 149
   ugucguuggc auguaccucg t 21
<210> 150
   <211> 21
   <212> DNA
   <213> homo sapiens
<400> 150
   cuggauguca uagagcucgt t 21
<210> 151
   <211> 21
   <212> DNA
   <213> homo sapiens
<400> 151
   uaagcuuaca aucuggcccg t 21
<210> 152
   <211> 21
   <212> DNA
   <213> homo sapiens
<400> 152
   uaucuucaca ucaacgugct g 21
<210> 153
   <211> 21
   <212> DNA
   <213> homo sapiens
<400> 153
   uauguucacg uuaucuccct t 21
<210> 154
   <211> 21
   <212> DNA
   <213> homo sapiens
<400> 154
   uuuaaggaca ccaauaucug g 21
<210> 155
   <211> 21
   <212> DNA
   <213> homo sapiens
<400> 155
   ugaaauuuga ugucauucca g 21
<210> 156
   <211> 21
   <212> DNA
   <213> homo sapiens
<400> 156
   uuguuuacaa guuagaggca a 21
<210> 157
   <211> 21
   <212> DNA
   <213> homo sapiens
<400> 157
   uucauugcac ugcagaccct t 21
<210> 158
   <211> 21
   <212> DNA
   <213> homo sapiens
<400> 158
   uagaauauca gguacuucat g 21
<210> 159
   <211> 21
   <212> DNA
   <213> homo sapiens
<400> 159
   uucaauugca auaugaucag a 21
<210> 160
   <211> 21
   <212> DNA
   <213> homo sapiens
<400> 160
   uagccaucca auauugucca a 21
<210> 161
   <211> 21
   <212> DNA
   <213> homo sapiens
<400> 161
   uacuucuaua ugaucuggca a 21
<210> 162
   <211> 21
   <212> DNA
   <213> homo sapiens
<400> 162
   uuugguauca gcagggcugg g 21
<210> 163
   <211> 21
   <212> DNA
   <213> homo sapiens
<400> 163
   uguacuauca gggucauugt t 21
<210> 164
   <211> 21
   <212> DNA
   <213> homo sapiens
<400> 164
   uucugauuuc agcccauuct t 21
<210> 165
   <211> 21
   <212> DNA
   <213> homo sapiens
<400> 165
   uuguugacgc aucuucaugg t 21
<210> 166
   <211> 21
   <212> DNA
   <213> homo sapiens
<400> 166
   auagcauuca acauaaaggt a 21
<210> 167
   <211> 21
   <212> DNA
   <213> homo sapiens
<400> 167
   uuugugacuu uccauuagca t 21
<210> 168
   <211> 21
   <212> DNA
   <213> homo sapiens
<400> 168
   uaaaugaaac gggacuggct g 21
<210> 169
   <211> 21
   <212> DNA
   <213> homo sapiens
<400> 169
   uacuaauugu acucacgcct t 21
<210> 170
   <211> 21
   <212> DNA
   <213> homo sapiens
<400> 170
   uugaauaugu ugccaagcct c 21
<210> 171
   <211> 21
   <212> DNA
   <213> homo sapiens
<400> 171
   uuauugcaua ugaaaccaca a 21
<210> 172
   <211> 21
   <212> DNA
   <213> homo sapiens
<400> 172
   uaaagcgugg uauucacgua g 21
<210> 173
   <211> 21
   <212> DNA
   <213> homo sapiens
<400> 173
   auuaaggcuu caaaguccct t 21
<210> 174
   <211> 21
   <212> DNA
   <213> homo sapiens
<400> 174
   uucugcacaa gucaucccgc a 21
<210> 175
   <211> 21
   <212> DNA
   <213> homo sapiens
<400> 175
   uaaauuguag gaucugggut g 21
<210> 176
   <211> 21
   <212> DNA
   <213> homo sapiens
<400> 176
   uaguugagug uaacaaucuc a 21
<210> 177
   <211> 21
   <212> DNA
   <213> homo sapiens
<400> 177
   uaagcuaaca aucucccaua g 21
<210> 178
   <211> 21
   <212> DNA
   <213> homo sapiens
<400> 178
   uaaggcucag agcugaugut g 21
<210> 179
   <211> 21
   <212> DNA
   <213> homo sapiens
<400> 179
   auguccagug ucaaucacgt t 21
<210> 180
   <211> 21
   <212> DNA
   <213> homo sapiens
<400> 180
   uucuguccua ggccgcuuct t 21
<210> 181
   <211> 21
   <212> DNA
   <213> homo sapiens
<400> 181
   uuaaguagca ccgaagucaa g 21
<210> 182
   <211> 21
   <212> DNA
   <213> homo sapiens
<400> 182
   uaacccaucc uucuugaugc g 21
<210> 183
   <211> 21
   <212> DNA
   <213> homo sapiens
<400> 183
   uugguugcca ggucaaauut a 21
<210> 184
   <211> 21
   <212> DNA
   <213> homo sapiens
<400> 184
   uagauuagga ugggaaaggc t 21
<210> 185
   <211> 21
   <212> DNA
   <213> homo sapiens
<400> 185
   uucuccaguc uguagcccug g 21
<210> 186
   <211> 21
   <212> DNA
   <213> homo sapiens
<400> 186
   uugaaauuug augucauucc a 21
<210> 187
   <211> 21
   <212> DNA
   <213> homo sapiens
<400> 187
   uuaaggacac caauaucugg g 21
<210> 188
   <211> 21
   <212> DNA
   <213> homo sapiens
<400> 188
   uuugaaagau auguucacgt t 21
<210> 189
   <211> 21
   <212> DNA
   <213> homo sapiens
<400> 189
   uuuacuucua uaugaucugg c 21
<210> 190
   <211> 21
   <212> DNA
   <213> homo sapiens
<400> 190
   uuaucuucac aucaacgugc t 21
<210> 191
   <211> 21
   <212> DNA
   <213> homo sapiens
<400> 191
   ugacuuucca uuagcaucgt c 21
<210> 192
   <211> 21
   <212> DNA
   <213> homo sapiens
<400> 192
   aauuguacuc acgccuucct a 21
<210> 193
   <211> 21
   <212> DNA
   <213> homo sapiens
<400> 193
   auacuaauug uacucacgcc t 21
<210> 194
   <211> 21
   <212> DNA
   <213> homo sapiens
<400> 194
   uuuaucuuca caucaacgug c 21
<210> 195
   <211> 21
   <212> DNA
   <213> homo sapiens
<400> 195
   uauacuaauu guacucacgc c 21
<210> 196
   <211> 21
   <212> DNA
   <213> homo sapiens
<400> 196
   ugucacuuga auauguugcc a 21
<210> 197
   <211> 21
   <212> DNA
   <213> homo sapiens
<400> 197
   uccuaagcua acaaucuccc a 21
<210> 198
   <211> 21
   <212> DNA
   <213> homo sapiens
<400> 198
   aucuucaugg uucguaucct g 21
<210> 199
   <211> 21
   <212> DNA
   <213> homo sapiens
<400> 199
   uccuuuguag auuaggaugg g 21
<210> 200
   <211> 21
   <212> DNA
   <213> homo sapiens
<400> 200
   auauguucac guuaucuccc t 21
<210> 201
   <211> 21
   <212> DNA
   <213> homo sapiens
<400> 201
   uaaaucucug guaacgaccc t 21
<210> 202
   <211> 21
   <212> DNA
   <213> homo sapiens
<400> 202
   uuacacauga acuccacgut g 21
<210> 203
   <211> 21
   <212> DNA
   <213> homo sapiens
<400> 203
   uauacucaga uuuaucaact t 21
<210> 204
   <211> 21
   <212> DNA
   <213> homo sapiens
<400> 204
   uagcggugca gaguguggct g 21
<210> 205
   <211> 21
   <212> DNA
   <213> homo sapiens
<400> 205
   uucaaacuga cccucgcucg g 21
<210> 206
   <211> 21
   <212> DNA
   <213> homo sapiens
<400> 206
   uucugcaguu agagguuggt g 21
<210> 207
   <211> 21
   <212> DNA
   <213> homo sapiens
<400> 207
   aucggaauua auaagccact g 21
<210> 208
   <211> 21
   <212> DNA
   <213> homo sapiens
<400> 208
   uacaagggac cauccugcgt g 21
<210> 209
   <211> 21
   <212> DNA
   <213> homo sapiens
<400> 209
   uuguuggcgg gcaacccugc t 21
<210> 210
   <211> 21
   <212> DNA
   <213> homo sapiens
<400> 210
   auagcaacug augccuccca g 21
<210> 211
   <211> 21
   <212> DNA
   <213> homo sapiens
<400> 211
   ugaggguuac agcugacggt g 21
<210> 212
   <211> 21
   <212> DNA
   <213> homo sapiens
<400> 212
   ucgauguggu gaaugucccg t 21
<210> 213
   <211> 21
   <212> DNA
   <213> homo sapiens
<400> 213
   ucucggugua ugcacuucut g 21
<210> 214
   <211> 21
   <212> DNA
   <213> homo sapiens
<400> 214
   uuucucuguu gcguccgact t 21
<210> 215
   <211> 21
   <212> DNA
   <213> homo sapiens
<400> 215
   uucuccacaa ugcaggugua g 21
<210> 216
   <211> 21
   <212> DNA
   <213> homo sapiens
<400> 216
   uugucugggc caaucuugct c 21
<210> 217
   <211> 21
   <212> DNA
   <213> homo sapiens
<400> 217
   uccggucaaa uaaugccucg g 21
<210> 218
   <211> 21
   <212> DNA
   <213> homo sapiens
<400> 218
   uuugaguccg ccauuggcaa g 21
<210> 219
   <211> 21
   <212> DNA
   <213> homo sapiens
<400> 219
   uuugccuaag accagucugt c 21
<210> 220
   <211> 21
   <212> DNA
   <213> homo sapiens
<400> 220
   uccagcaguc uucaagauct g 21
<210> 221
   <211> 21
   <212> DNA
   <213> homo sapiens
<400> 221
   uccgauagag uuacccgcca a 21
<210> 222
   <211> 21
   <212> DNA
   <213> homo sapiens
<400> 222
   uugucagagg gcaccacaga g 21
<210> 223
   <211> 21
   <212> DNA
   <213> homo sapiens
<400> 223
   uuggaggcau acuccacgat g 21
<210> 224
   <211> 21
   <212> DNA
   <213> homo sapiens
<400> 224
   ucucgguccc gaccggacgt g 21
<210> 225
   <211> 21
   <212> DNA
   <213> homo sapiens
<400> 225
   ucugguacca ggcauuuggt c 21
<210> 226
   <211> 21
   <212> DNA
   <213> homo sapiens
<400> 226
   uuguccagcc cgauagccuc t 21
<210> 227
   <211> 21
   <212> DNA
   <213> homo sapiens
<400> 227
   uuuagccacu ggaugugcgg c 21
<210> 228
   <211> 21
   <212> DNA
   <213> homo sapiens
<400> 228
   uguagccucc aauucugugg t 21
<210> 229
   <211> 21
   <212> DNA
   <213> homo sapiens
<400> 229
   uucaaucgug gcucgaagca c 21
<210> 230
   <211> 21
   <212> DNA
   <213> homo sapiens
<400> 230
   aucuccaugg auacuccaca g 21
<210> 231
   <211> 21
   <212> DNA
   <213> homo sapiens
<400> 231
   uuucaaccag cgcagugugg g 21
<210> 232
   <211> 21
   <212> DNA
   <213> homo sapiens
<400> 232
   uagagcuccg ggugucggga a 21
<210> 233
   <211> 21
   <212> DNA
   <213> homo sapiens
<400> 233
   uuaccgaugg guaaaucuct g 21
<210> 234
   <211> 21
   <212> DNA
   <213> homo sapiens
<400> 234
   aaaucucugg uaacgaccct t 21
<210> 235
   <211> 21
   <212> DNA
   <213> homo sapiens
<400> 235
   uauagcaacu gaugccuccc a 21
<210> 236
   <211> 21
   <212> DNA
   <213> homo sapiens
<400> 236
   uuucaaacug acccucgcuc g 21
<210> 237
   <211> 21
   <212> DNA
   <213> homo sapiens
<400> 237
   auacucagau uuaucaacut t 21
<210> 238
   <211> 21
   <212> DNA
   <213> homo sapiens
<400> 238
   uaccgauggg uaaaucucug g 21
<210> 239
   <211> 21
   <212> DNA
   <213> homo sapiens
<400> 239
   auacaaggga ccauccugcg t 21
<210> 240
   <211> 21
   <212> DNA
   <213> homo sapiens
<400> 240
   uacucagauu uaucaacuut g 21
<210> 241
   <211> 21
   <212> DNA
   <213> homo sapiens
<400> 241
   ucgguguaug cacuucuugg a 21
<210> 242
   <211> 21
   <212> DNA
   <213> homo sapiens
<400> 242
   uacuccacga ugacauacaa g 21
<210> 243
   <211> 21
   <212> DNA
   <213> homo sapiens
<400> 243
   ucggaauuaa uaagccacug g 21
<210> 244
   <211> 21
   <212> DNA
   <213> homo sapiens
<400> 244
   acagagucca uuaugaugct c 21
<210> 245
   <211> 21
   <212> DNA
   <213> homo sapiens
<400> 245
   uuugucggug guauuaacuc c 21
<210> 246
   <211> 21
   <212> DNA
   <213> homo sapiens
<400> 246
   gaguccauua ugaugcucca g 21
<210> 247
   <211> 21
   <212> DNA
   <213> homo sapiens
<400> 247
   uaucggaauu aauaagccac t 21
<210> 248
   <211> 21
   <212> DNA
   <213> homo sapiens
<400> 248
   auccggucaa auaaugccuc g 21
<210> 249
   <211> 21
   <212> DNA
   <213> homo sapiens
<400> 249
   uucucuguug cguccgacut c 21
<210> 250
   <211> 21
   <212> DNA
   <213> homo sapiens
<400> 250
   auguggugaa ugucccgugc g 21
<210> 251
   <211> 21
   <212> DNA
   <213> homo sapiens
<400> 251
   uuuauuagga acaucugcct g 21
<210> 252
   <211> 21
   <212> DNA
   <213> homo sapiens
<400> 252
   uugaucuaac ugaagcaccg g 21
<210> 253
   <211> 21
   <212> DNA
   <213> homo sapiens
<400> 253
   auuguuugga ugguaagcct g 21
<210> 254
   <211> 21
   <212> DNA
   <213> homo sapiens
<400> 254
   uaauuagcca guuagugggt t 21
<210> 255
   <211> 21
   <212> DNA
   <213> homo sapiens
<400> 255
   uucguuucca uggaggugca a 21
<210> 256
   <211> 21
   <212> DNA
   <213> homo sapiens
<400> 256
   ucaucuaaug ucagauucgg g 21
<210> 257
   <211> 21
   <212> DNA
   <213> homo sapiens
<400> 257
   uacuuguuga gugucucagt t 21
<210> 258
   <211> 21
   <212> DNA
   <213> homo sapiens
<400> 258
   augacgugcc aagaacucct t 21
<210> 259
   <211> 21
   <212> DNA
   <213> homo sapiens
<400> 259
   uuucccagga ccucauagca a 21
<210> 260
   <211> 21
   <212> DNA
   <213> homo sapiens
<400> 260
   aagagauauu ccuucaucga t 21
<210> 261
   <211> 21
   <212> DNA
   <213> homo sapiens
<400> 261
   uugaggagau gcuccuguga g 21
<210> 262
   <211> 21
   <212> DNA
   <213> homo sapiens
<400> 262
   ucuuguggca uagaucuggc t 21
<210> 263
   <211> 21
   <212> DNA
   <213> homo sapiens
<400> 263
   auagugccug uccagagcca g 21
<210> 264
   <211> 21
   <212> DNA
   <213> homo sapiens
<400> 264
   ucaacgagag cauccagccc t 21
<210> 265
   <211> 21
   <212> DNA
   <213> homo sapiens
<400> 265
   augcauagcc aggaucuuga g 21
<210> 266
   <211> 21
   <212> DNA
   <213> homo sapiens
<400> 266
   uuggagguac cucaacagct c 21
<210> 267
   <211> 21
   <212> DNA
   <213> homo sapiens
<400> 267
   ucaggguguu gguuauucut t 21
<210> 268
   <211> 21
   <212> DNA
   <213> homo sapiens
<400> 268
   aucuguaaua uuugacaugt c 21
<210> 269
   <211> 21
   <212> DNA
   <213> homo sapiens
<400> 269
   ugcuugucuc guuccacuug g 21
<210> 270
   <211> 21
   <212> DNA
   <213> homo sapiens
<400> 270
   uucagagguu ggaagagaca t 21
<210> 271
   <211> 21
   <212> DNA
   <213> homo sapiens
<400> 271
   uuggauggua agccuggcgg a 21
<210> 272
   <211> 21
   <212> DNA
   <213> homo sapiens
<400> 272
   uaaagaugug acguucaacg g 21
<210> 273
   <211> 21
   <212> DNA
   <213> homo sapiens
<400> 273
   ucuaacugaa gcaccggcca g 21
<210> 274
   <211> 21
   <212> DNA
   <213> homo sapiens
<400> 274
   ucaacgggaa ugauggugct t 21
<210> 275
   <211> 21
   <212> DNA
   <213> homo sapiens
<400> 275
   uuguuuggau gguaagccug g 21
<210> 276
   <211> 21
   <212> DNA
   <213> homo sapiens
<400> 276
   uuuggauggu aagccuggcg g 21
<210> 277
   <211> 21
   <212> DNA
   <213> homo sapiens
<400> 277
   uuugaucuaa cugaagcacc g 21
<210> 278
   <211> 21
   <212> DNA
   <213> homo sapiens
<400> 278
   uucaacggga augauggugc t 21
<210> 279
   <211> 21
   <212> DNA
   <213> homo sapiens
<400> 279
   ugcuucguuu ccauggaggt g 21
<210> 280
   <211> 21
   <212> DNA
   <213> homo sapiens
<400> 280
   ucuggcuucc aaacccucut t 21
<210> 281
   <211> 21
   <212> DNA
   <213> homo sapiens
<400> 281
   augcuaauua gccaguuagt g 21
<210> 282
   <211> 21
   <212> DNA
   <213> homo sapiens
<400> 282
   agauauuccu ucaucgaugg t 21
<210> 283
   <211> 21
   <212> DNA
   <213> homo sapiens
<400> 283
   uuauuaggaa caucugccug c 21
<210> 284
   <211> 21
   <212> DNA
   <213> homo sapiens
<400> 284
   cuaauuagcc aguuaguggg t 21
<210> 285
   <211> 21
   <212> DNA
   <213> homo sapiens
<400> 285
   ugaucuaacu gaagcaccgg c 21
<210> 286
   <211> 21
   <212> DNA
   <213> homo sapiens
<400> 286
   aauuguuugg augguaagcc t 21
<210> 287
   <211> 21
   <212> DNA
   <213> homo sapiens
<400> 287
   uggaugguaa gccuggcgga a 21
<210> 288
   <211> 21
   <212> DNA
   <213> homo sapiens
<400> 288
   uugcugacca ggccaugcat a 21
<210> 289
   <211> 21
   <212> DNA
   <213> homo sapiens
<400> 289
   aucuggcuuc caaacccuct t 21
<210> 290
   <211> 21
   <212> DNA
   <213> homo sapiens
<400> 290
   aaugguuuga ucuaacugaa g 21
<210> 291
   <211> 21
   <212> DNA
   <213> homo sapiens
<400> 291
   uccauggagg ugcaaaggcc g 21
<210> 292
   <211> 21
   <212> DNA
   <213> homo sapiens
<400> 292
   augauggugc uucguuucca t 21
<210> 293
   <211> 21
   <212> DNA
   <213> homo sapiens
<400> 293
   uguuuggaug guaagccugg c 21
<210> 294
   <211> 21
   <212> DNA
   <213> homo sapiens
<400> 294
   uucuuguggc auagaucugg c 21
<210> 295
   <211> 21
   <212> DNA
   <213> homo sapiens
<400> 295
   agggucaacg agagcaucca g 21
<210> 296
   <211> 21
   <212> DNA
   <213> homo sapiens
<400> 296
   auaggcgaug aucacaacat a 21
<210> 297
   <211> 21
   <212> DNA
   <213> homo sapiens
<400> 297
   auacuuguug agugucucag t 21
<210> 298
   <211> 21
   <212> DNA
   <213> homo sapiens
<400> 298
   aaugauggug cuucguuucc a 21
<210> 299
   <211> 21
   <212> DNA
   <213> homo sapiens
<400> 299
   cuuuauuagg aacaucugcc t 21
<210> 300
   <211> 21
   <212> DNA
   <213> homo sapiens
<400> 300
   uaggagguaa cacgaugacg t 21
<210> 301
   <211> 21
   <212> DNA
   <213> homo sapiens
<400> 301
   uuaaguguca auuuaguggc a 21
<210> 302
   <211> 21
   <212> DNA
   <213> homo sapiens
<400> 302
   uuucuugugg gucaauucct a 21
<210> 303
   <211> 21
   <212> DNA
   <213> homo sapiens
<400> 303
   uugggucuug ugaauaagct g 21
<210> 304
   <211> 21
   <212> DNA
   <213> homo sapiens
<400> 304
   uucacuucuu agaacauaga g 21
<210> 305
   <211> 21
   <212> DNA
   <213> homo sapiens
<400> 305
   uuggaugagu agacggucct t 21
<210> 306
   <211> 21
   <212> DNA
   <213> homo sapiens
<400> 306
   auuacuaaga ucuucaccut t 21
<210> 307
   <211> 21
   <212> DNA
   <213> homo sapiens
<400> 307
   uugguuuaau cagccuuggt g 21
<210> 308
   <211> 21
   <212> DNA
   <213> homo sapiens
<400> 308
   aucacuacug uuuaucugca g 21
<210> 309
   <211> 21
   <212> DNA
   <213> homo sapiens
<400> 309
   auccguaaca gcauccgcca g 21
<210> 310
   <211> 21
   <212> DNA
   <213> homo sapiens
<400> 310
   auguauagcu agaaucuuga g 21
<210> 311
   <211> 21
   <212> DNA
   <213> homo sapiens
<400> 311
   aagaugaccc gcauggcccg g 21
<210> 312
   <211> 21
   <212> DNA
   <213> homo sapiens
<400> 312
   ucucaguacc ucauguaggt g 21
<210> 313
   <211> 21
   <212> DNA
   <213> homo sapiens
<400> 313
   uuugaccaag uagcgcuuct g 21
<210> 314
   <211> 21
   <212> DNA
   <213> homo sapiens
<400> 314
   uucguuaggu acauaucaca t 21
<210> 315
   <211> 21
   <212> DNA
   <213> homo sapiens
<400> 315
   augaguacuu cauuccucut t 21
<210> 316
   <211> 21
   <212> DNA
   <213> homo sapiens
<400> 316
   uuggguggua gucagagcug t 21
<210> 317
   <211> 21
   <212> DNA
   <213> homo sapiens
<400> 317
   uuucuaaacc augcaaggga a 21
<210> 318
   <211> 21
   <212> DNA
   <213> homo sapiens
<400> 318
   ucauguguua auucuauguc t 21
<210> 319
   <211> 21
   <212> DNA
   <213> homo sapiens
<400> 319
   uuaagucaca uugcgguaca a 21
<210> 320
   <211> 21
   <212> DNA
   <213> homo sapiens
<400> 320
   uguauuguug cccaugucct c 21
<210> 321
   <211> 21
   <212> DNA
   <213> homo sapiens
<400> 321
   ugaccugcug uuauuggagt g 21
<210> 322
   <211> 21
   <212> DNA
   <213> homo sapiens
<400> 322
   aaauauaggc aggugguuct a 21
<210> 323
   <211> 21
   <212> DNA
   <213> homo sapiens
<400> 323
   accuugacga ugaaacuuct g 21
<210> 324
   <211> 21
   <212> DNA
   <213> homo sapiens
<400> 324
   uuucaagguu cguccgugut g 21
<210> 325
   <211> 21
   <212> DNA
   <213> homo sapiens
<400> 325
   ugagguaaac uuaaauccug a 21
<210> 326
   <211> 21
   <212> DNA
   <213> homo sapiens
<400> 326
   uucuggccaa ugaaggcgua g 21
<210> 327
   <211> 21
   <212> DNA
   <213> homo sapiens
<400> 327
   uuuaaguguc aauuuagugg c 21
<210> 328
   <211> 21
   <212> DNA
   <213> homo sapiens
<400> 328
   uagaacauag agugccaugg g 21
<210> 329
   <211> 21
   <212> DNA
   <213> homo sapiens
<400> 329
   aauuacuaag aucuucacct t 21
<210> 330
   <211> 21
   <212> DNA
   <213> homo sapiens
<400> 330
   uaacauugga ugaguagacg g 21
<210> 331
   <211> 21
   <212> DNA
   <213> homo sapiens
<400> 331
   ucuuagaaca uagagugcca t 21
<210> 332
   <211> 21
   <212> DNA
   <213> homo sapiens
<400> 332
   uggcauuaag ucacauugcg g 21
<210> 333
   <211> 21
   <212> DNA
   <213> homo sapiens
<400> 333
   uagccuuggu uuaaucagcc t 21
<210> 334
   <211> 21
   <212> DNA
   <213> homo sapiens
<400> 334
   uucuuguggg ucaauuccua t 21
<210> 335
   <211> 21
   <212> **DNA**
   <213> homo sapiens
<400> 335
   uaucacuacu guuuaucugc a 21
<210> 336
   <211> 21
   <212> **DNA**
   <213> homo sapiens
<400> 336
   ucaggcugaa ggauacuucg t 21
<210> 337
   <211> 21
   <212> DNA
   <213> homo sapiens
<400> 337
   uguguuaauu cuaugucuga a 21
<210> 338
   <211> 21
   <212> DNA
   <213> homo sapiens
<400> 338
   uauuguugcc cauguccuca t 21
<210> 339
   <211> 21
   <212> DNA
   <213> homo sapiens
<400> 339
   uuguggguca auuccuauaa g 21
<210> 340
   <211> 21
   <212> DNA
   <213> homo sapiens
<400> 340
   auuucuugug ggucaauucc t 21
<210> 341
   <211> 21
   <212> DNA
   <213> homo sapiens
<400> 341
   uguuauugga guggccaccg a 21
<210> 342
   <211> 21
   <212> DNA
   <213> homo sapiens
<400> 342
   ucuguaaauu uguucacuct c 21
<210> 343
   <211> 21
   <212> DNA
   <213> homo sapiens
<400> 343
   uugcgguaca acuaucacua c 21
<210> 344
   <211> 21
   <212> DNA
   <213> homo sapiens
<400> 344
   augaguagac gguccuucgg a 21
<210> 345
   <211> 21
   <212> DNA
   <213> homo sapiens
<400> 345
   uaauuacuaa gaucuucacc t 21
<210> 346
   <211> 21
   <212> DNA
   <213> homo sapiens
<400> 346
   ugaaacaacc uugacgauga a 21
<210> 347
   <211> 21
   <212> DNA
   <213> homo sapiens
<400> 347
   ugaucaagcc auguauagct a 21
<210> 348
   <211> 21
   <212> DNA
   <213> homo sapiens
<400> 348
   uguaauuacu aagaucuuca c 21
<210> 349
   <211> 21
   <212> DNA
   <213> homo sapiens
<400> 349
   ugaauuugac caaguagcgc t 21
<210> 350
   <211> 21
   <212> DNA
   <213> homo sapiens
<400> 350
   uacuucguua gguacauauc a 21
<210> 351
   <211> 21
   <212> DNA
   <213> homo sapiens
<400> 351
   uguaguaaca gucuuccuca a 21
<210> 352
   <211> 21
   <212> DNA
   <213> homo sapiens
<400> 352
   uggacgauaa ucuagcaaca g 21
<210> 353
   <211> 21
   <212> DNA
   <213> homo sapiens
<400> 353
   uauggcagaa uuggccauca t 21
<210> 354
   <211> 21
   <212> DNA
   <213> homo sapiens
<400> 354
   uuucaccugg aggacagggc t 21
<210> 355
   <211> 21
   <212> DNA
   <213> homo sapiens
<400> 355
   ucacuugggc auuaacacut t 21
<210> 356
   <211> 21
   <212> DNA
   <213> homo sapiens
<400> 356
   acuuccucuu ugcacuuggt g 21
<210> 357
   <211> 21
   <212> DNA
   <213> homo sapiens
<400> 357
   ugagugugca uuccuugaug a 21
<210> 358
   <211> 21
   <212> DNA
   <213> homo sapiens
<400> 358
   ucccuucuug gcagggcacg c 21
<210> 359
   <211> 21
   <212> DNA
   <213> homo sapiens
<400> 359
   gacugugcag ucccuagcut t 21
<210> 360
   <211> 21
   <212> DNA
   <213> homo sapiens
<400> 360
   aucaugaugc aggccuucca a 21
<210> 361
   <211> 21
   <212> DNA
   <213> homo sapiens
<400> 361
   uucugagucu caacuguagt a 21
<210> 362
   <211> 21
   <212> DNA
   <213> homo sapiens
<400> 362
   uaaucuagca acagacguaa g 21
<210> 363
   <211> 21
   <212> DNA
   <213> homo sapiens
<400> 363
   ucaacuguag uaacagucut c 21
<210> 364
   <211> 21
   <212> DNA
   <213> homo sapiens
<400> 364
   cucaacugua guaacaguct t 21
<210> 365
   <211> 21
   <212> DNA
   <213> homo sapiens
<400> 365
   agcaacagac guaagaacca g 21
<210> 366
   <211> 21
   <212> DNA
   <213> homo sapiens
<400> 366
   ucugagucuc aacuguagua a 21
<210> 367
   <211> 21
   <212> DNA
   <213> homo sapiens
<400> 367
   uuccuuucac cuggaggaca g 21
<210> 368
   <211> 21
   <212> DNA
   <213> homo sapiens
<400> 368
   uuggacgaua aucuagcaac a 21
<210> 369
   <211> 21
   <212> DNA
   <213> homo sapiens
<400> 369
   acuguaguaa cagucuucct c 21
<210> 370
   <211> 21
   <212> DNA
   <213> homo sapiens
<400> 370
   ucaugaugca ggccuuccaa g 21
<210> 371
   <211> 21
   <212> DNA
   <213> homo sapiens
<400> 371
   ucaauuccaa ucccuuggag t 21
<210> 372
   <211> 21
   <212> DNA
   <213> homo sapiens
<400> 372
   gugcaguccc uagcuuucct t 21
<210> 373
   <211> 21
   <212> DNA
   <213> homo sapiens
<400> 373
   ccaaguucug agucucaact g 21
<210> 374
   <211> 21
   <212> DNA
   <213> homo sapiens
<400> 374
   gauaaucuag caacagacgt a 21
<210> 375
   <211> 21
   <212> DNA
   <213> homo sapiens
<400> 375
   uuauggcaga auuggccauc a 21
<210> 376
   <211> 21
   <212> DNA
   <213> homo sapiens
<400> 376
   caaguucuga gucucaacug t 21
<210> 377
   <211> 21
   <212> DNA
   <213> homo sapiens
<400> 377
   ugugcagucc cuagcuuucc t 21
<210> 378
   <211> 21
   <212> DNA
   <213> homo sapiens
<400> 378
   ucccuuggag uugaugucag t 21
<210> 379
   <211> 21
   <212> DNA
   <213> homo sapiens
<400> 379
   auggcagaau uggccaucat g 21
<210> 380
   <211> 21
   <212> DNA
   <213> homo sapiens
<400> 380
   uggccaucau gaugcaggcc t 21
<210> 381
   <211> 21
   <212> DNA
   <213> homo sapiens
<400> 381
   gucacuuggg cauuaacact t 21
<210> 382
   <211> 21
   <212> DNA
   <213> homo sapiens
<400> 382
   gcuuauggca gaauuggcca t 21
<210> 383
   <211> 21
   <212> DNA
   <213> homo sapiens
<400> 383
   cgauaaucua gcaacagacg t 21
<210> 384
   <211> 21
   <212> DNA
   <213> homo sapiens
<400> 384
   ucucaacugu aguaacaguc t 21
<210> 385
   <211> 21
   <212> DNA
   <213> homo sapiens
<400> 385
   aucuagcaac agacguaaga a 21
<210> 386
   <211> 21
   <212> DNA
   <213> homo sapiens
<400> 386
   agucacuugg gcauuaacac t 21
<210> 387
   <211> 21
   <212> DNA
   <213> homo sapiens
<400> 387
   agggcuuaug gcagaauugg c 21
<210> 388
   <211> 21
   <212> DNA
   <213> homo sapiens
<400> 388
   ugagucucaa cuguaguaac a 21
<210> 389
   <211> 21
   <212> DNA
   <213> homo sapiens
<400> 389
   aguucugagu cucaacugua g 21
<210> 390
   <211> 21
   <212> DNA
   <213> homo sapiens
<400> 390
   uuuggacgau aaucuagcaa c 21
<210> 391
   <211> 21
   <212> DNA
   <213> homo sapiens
<400> 391
   ccucaauucc aaucccuugg a 21
<210> 392
   <211> 21
   <212> DNA
   <213> homo sapiens
<400> 392
   uggcagaauu ggccaucaug a 21
<210> 393
   <211> 21
   <212> DNA
   <213> homo sapiens
<400> 393
   cuguaguaac agucuuccuc a 21
<210> 394
   <211> 21
   <212> DNA
   <213> homo sapiens
<400> 394
   ucuagcaaca gacguaagaa c 21
<210> 395
   <211> 21
   <212> DNA
   <213> homo sapiens
<400> 395
   acgauaaucu agcaacagac g 21
<210> 396
   <211> 21
   <212> DNA
   <213> homo sapiens
<400> 396
   agucucaacu guaguaacag t 21
<210> 397
   <211> 21
   <212> DNA
   <213> homo sapiens
<400> 397
   acagggcuua uggcagaaut g 21
<210> 398
   <211> 21
   <212> DNA
   <213> homo sapiens
<400> 398
   aaucuagcaa cagacguaag a 21
<210> 399
   <211> 21
   <212> DNA
   <213> homo sapiens
<400> 399
   cuuauggcag aauuggccat c 21
<210> 400
   <211> 21
   <212> DNA
   <213> homo sapiens
<400> 400
   agggcacgca gucugguuca t 21
<210> 401
   <211> 21
   <212> DNA
   <213> homo sapiens
<400> 401
   uuugucaccu augacaccca g 21
<210> 402
   <211> 21
   <212> DNA
   <213> homo sapiens
<400> 402
   uuauagagca agccugguct g 21
<210> 403
   <211> 21
   <212> DNA
   <213> homo sapiens
<400> 403
   ucugauugug guaucuucct g 21
<210> 404
   <211> 21
   <212> DNA
   <213> homo sapiens
<400> 404
   uauuucagga caauuaugcc a 21
<210> 405
   <211> 21
   <212> DNA
   <213> homo sapiens
<400> 405
   uuaauguagu auuuccucca c 21
<210> 406
   <211> 21
   <212> DNA
   <213> homo sapiens
<400> 406
   uuucccaucg uuaccugcgg t 21
<210> 407
   <211> 21
   <212> DNA
   <213> homo sapiens
<400> 407
   uaguucaguu ggaucauccc a 21
<210> 408
   <211> 21
   <212> DNA
   <213> homo sapiens
<400> 408
   uugccuucug acacuaagca a 21
<210> 409
   <211> 21
   <212> DNA
   <213> homo sapiens
<400> 409
   uuauagggug ugccgccuct g 21
<210> 410
   <211> 21
   <212> DNA
   <213> homo sapiens
<400> 410
   uuuccaucug aaauauagga t 21
<210> 411
   <211> 21
   <212> DNA
   <213> homo sapiens
<400> 411
   uugcgcacca gcuucagucc g 21
<210> 412
   <211> 21
   <212> DNA
   <213> homo sapiens
<400> 412
   uugauguaga aaucagggut g 21
<210> 413
   <211> 21
   <212> DNA
   <213> homo sapiens
<400> 413
   uucucagcaa uagaacacca g 21
<210> 414
   <211> 21
   <212> DNA
   <213> homo sapiens
<400> 414
   uaaggcuucu uauaggucga a 21
<210> 415
   <211> 21
   <212> DNA
   <213> homo sapiens
<400> 415
   ucaaagaucc auucgccgcg g 21
<210> 416
   <211> 21
   <212> DNA
   <213> homo sapiens
<400> 416
   uugaugaggu agugcuccgg g 21
<210> 417
   <211> 21
   <212> DNA
   <213> homo sapiens
<400> 417
   uuuaugacgc ucauccgcug a 21
<210> 418
   <211> 21
   <212> DNA
   <213> homo sapiens
<400> 418
   uauuuguagg acacguugga a 21
<210> 419
   <211> 21
   <212> DNA
   <213> homo sapiens
<400> 419
   uacccugccg agguucacgg g 21
<210> 420
   <211> 21
   <212> DNA
   <213> homo sapiens
<400> 420
   uaucugagca cacucaaacg t 21
<210> 421
   <211> 21
   <212> DNA
   <213> homo sapiens
<400> 421
   ucuuuguaca ggucaauuct a 21
<210> 422
   <211> 21
   <212> DNA
   <213> homo sapiens
<400> 422
   uuugacuuga gagguaucgc t 21
<210> 423
   <211> 21
   <212> DNA
   <213> homo sapiens
<400> 423
   uuguguuucu ggacgaauut g 21
<210> 424
   <211> 21
   <212> DNA
   <213> homo sapiens
<400> 424
   uagagcuucc auuccucacg g 21
<210> 425
   <211> 21
   <212> DNA
   <213> homo sapiens
<400> 425
   uucacuuggc ucucgcugca g 21
<210> 426
   <211> 21
   <212> DNA
   <213> homo sapiens
<400> 426
   uacccggccg auaucuaugg g 21
<210> 427
   <211> 21
   <212> DNA
   <213> homo sapiens
<400> 427
   uucucaauuc cgacuggcct t 21
<210> 428
   <211> 21
   <212> DNA
   <213> homo sapiens
<400> 428
   uauuacagua aaguugauug a 21
<210> 429
   <211> 21
   <212> DNA
   <213> homo sapiens
<400> 429
   uuaacacagg cguauuccgt g 21
<210> 430
   <211> 21
   <212> DNA
   <213> homo sapiens
<400> 430
   aaaugugcuc uguacgccca g 21
<210> 431
   <211> 21
   <212> DNA
   <213> homo sapiens
<400> 431
   uaguugaaau gcuuguccgc t 21
<210> 432
   <211> 21
   <212> DNA
   <213> homo sapiens
<400> 432
   uuggcuccag agcacgccgg g 21
<210> 433
   <211> 21
   <212> DNA
   <213> homo sapiens
<400> 433
   uucucugaca ccucaacucc a 21
<210> 434
   <211> 21
   <212> DNA
   <213> homo sapiens
<400> 434
   uaaggagcuc agaucaaaca g 21
<210> 435
   <211> 21
   <212> DNA
   <213> homo sapiens
<400> 435
   ugaacauuca gucagaucga a 21
<210> 436
   <211> 21
   <212> DNA
   <213> homo sapiens
<400> 436
   uauaguacga gacuccguug t 21
<210> 437
   <211> 21
   <212> DNA
   <213> homo sapiens
<400> 437
   augaauagag aaguguccgg a 21
<210> 438
   <211> 21
   <212> DNA
   <213> homo sapiens
<400> 438
   auaagcacag uaaagguggt a 21
<210> 439
   <211> 21
   <212> DNA
   <213> homo sapiens
<400> 439
   uuaacagcuu aggcguuccc a 21
<210> 440
   <211> 21
   <212> DNA
   <213> homo sapiens
<400> 440
   uuccuuuccc aucguuacct g 21
<210> 441
   <211> 21
   <212> DNA
   <213> homo sapiens
<400> 441
   auugauguag aaaucagggt t 21
<210> 442
   <211> 21
   <212> DNA
   <213> homo sapiens
<400> 442
   auguaguauu uccuccacgt g 21
<210> 443
   <211> 21
   <212> DNA
   <213> homo sapiens
<400> 443
   uuaaggcuuc uuauaggucg a 21
<210> 444
   <211> 21
   <212> DNA
   <213> homo sapiens
<400> 444
   auugaugagg uagugcuccg g 21
<210> 445
   <211> 21
   <212> DNA
   <213> homo sapiens
<400> 445
   aaauauagga ugaaccuccg c 21
<210> 446
   <211> 21
   <212> DNA
   <213> homo sapiens
<400> 446
   uauaggauga accuccgcuc t 21
<210> 447
   <211> 21
   <212> DNA
   <213> homo sapiens
<400> 447
   uugaguauuu guaggacacg t 21
<210> 448
   <211> 21
   <212> DNA
   <213> homo sapiens
<400> 448
   uuguaggaca cguuggaact t 21
<210> 449
   <211> 21
   <212> DNA
   <213> homo sapiens
<400> 449
   aucccuuaua gagcaagcct g 21
<210> 450
   <211> 21
   <212> DNA
   <213> homo sapiens
<400> 450
   ucaaacguga uccuggugga g 21
<210> 451
   <211> 21
   <212> DNA
   <213> homo sapiens
<400> 451
   caguuaacaa guucuuauat t 21
<210> 452
   <211> 21
   <212> DNA
   <213> homo sapiens
<400> 452
   gcaggugcuu gaaaccguat t 21
<210> 453
   <211> 21
   <212> DNA
   <213> homo sapiens
<400> 453
   caaucuugcu gagcauaaat t 21
<210> 454
   <211> 21
   <212> DNA
   <213> homo sapiens
<400> 454
   ccggcuuuca ggaagauaat t 21
<210> 455
   <211> 21
   <212> DNA
   <213> homo sapiens
<400> 455
   cgcauauggu aucccucaat t 21
<210> 456
   <211> 21
   <212> DNA
   <213> homo sapiens
<400> 456
   gcuacucguu aauuaucaat t 21
<210> 457
   <211> 21
   <212> DNA
   <213> homo sapiens
<400> 457
   ccggaaguug uaugguuaat t 21
<210> 458
   <211> 21
   <212> DNA
   <213> homo sapiens
<400> 458
   gccggguuac gucaccuaat t 21
<210> 459
   <211> 21
   <212> DNA
   <213> homo sapiens
<400> 459
   ccaacuaccu caagagcaat t 21
<210> 460
   <211> 21
   <212> DNA
   <213> homo sapiens
<400> 460
   ggcauuugua uaagacaaat t 21
<210> 461
   <211> 21
   <212> DNA
   <213> homo sapiens
<400> 461
   cugccacucu aauugucaat t 21
<210> 462
   <211> 21
   <212> DNA
   <213> homo sapiens
<400> 462
   gacuaccuau caauuauaat t 21
<210> 463
   <211> 21
   <212> DNA
   <213> homo sapiens
<400> 463
   aguggguuua cauacucaat t 21
<210> 464
   <211> 21
   <212> DNA
   <213> homo sapiens
<400> 464
   ggagcccauc acuauggaat t 21
<210> 465
   <211> 21
   <212> DNA
   <213> homo sapiens
<400> 465
   gcggacaguu aauaacagat t 21
<210> 466
   <211> 21
   <212> DNA
   <213> homo sapiens
<400> 466
   gguaaagacu acaucccaat t 21
<210> 467
   <211> 21
   <212> DNA
   <213> homo sapiens
<400> 467
   ggagcugauc acucuaacat t 21
<210> 468
   <211> 21
   <212> DNA
   <213> homo sapiens
<400> 468
   cuuugauccc ugauggaaat t 21
<210> 469
   <211> 21
   <212> DNA
   <213> homo sapiens
<400> 469
   cuugaacacg agaguucaat t 21
<210> 470
   <211> 21
   <212> DNA
   <213> homo sapiens
<400> 470
   ggaaccucgg acaagucuat t 21
<210> 471
   <211> 21
   <212> DNA
   <213> homo sapiens
<400> 471
   gccauacucu uguccucaat t 21
<210> 472
   <211> 21
   <212> DNA
   <213> homo sapiens
<400> 472
   acuugagagu aaccaguaat t 21
<210> 473
   <211> 21
   <212> DNA
   <213> homo sapiens
<400> 473
   cgaccacgcu gagcuggaat t 21
<210> 474
   <211> 21
   <212> DNA
   <213> homo sapiens
<400> 474
   ccauaaucau uccgaagcat t 21
<210> 475
   <211> 21
   <212> DNA
   <213> homo sapiens
<400> 475
   ccugugaagc aacagucaat t 21
<210> 476
   <211> 21
   <212> DNA
   <213> homo sapiens
<400> 476
   caggaguaca aauggaugat t 21
<210> 477
   <211> 21
   <212> DNA
   <213> homo sapiens
<400> 477
   ggaacaaggc aagaaaccat t 21
<210> 478
   <211> 21
   <212> DNA
   <213> homo sapiens
<400> 478
   cacuacacau ggagccuaat t 21
<210> 479
   <211> 21
   <212> DNA
   <213> homo sapiens
<400> 479
   gguuguggcu gacucuagat t 21
<210> 480
   <211> 21
   <212> DNA
   <213> homo sapiens
<400> 480
   cguggcuacu cguuaauuat t 21
<210> 481
   <211> 21
   <212> DNA
   <213> homo sapiens
<400> 481
   gccggaaguu guaugguuat t 21
<210> 482
   <211> 21
   <212> DNA
   <213> homo sapiens
<400> 482
   cucuaauugu caaugugaat t 21
<210> 483
   <211> 21
   <212> DNA
   <213> homo sapiens
<400> 483
   ggaaauagug gguuuacaut t 21
<210> 484
   <211> 21
   <212> DNA
   <213> homo sapiens
<400> 484
   ggacguaacu gaagaggaut t 21
<210> 485
   <211> 21
   <212> DNA
   <213> homo sapiens
<400> 485
   gaugcaggga auuauacaat t 21
<210> 486
   <211> 21
   <212> DNA
   <213> homo sapiens
<400> 486
   cggaaguugu augguuaaat t 21
<210> 487
   <211> 21
   <212> DNA
   <213> homo sapiens
<400> 487
   cagucaaugg gcauuuguat t 21
<210> 488
   <211> 21
   <212> DNA
   <213> homo sapiens
<400> 488
   ggguuacguc accuaacaut t 21
<210> 489
   <211> 21
   <212> DNA
   <213> homo sapiens
<400> 489
   caagagcaaa cgugacuuat t 21
<210> 490
   <211> 21
   <212> DNA
   <213> homo sapiens
<400> 490
   caacuaccuc aagagcaaat t 21
<210> 491
   <211> 21
   <212> DNA
   <213> homo sapiens
<400> 491
   augccauacu gacaggaaat t 21
<210> 492
   <211> 21
   <212> DNA
   <213> homo sapiens
<400> 492
   agaacaaugc acuacaguat t 21
<210> 493
   <211> 21
   <212> DNA
   <213> homo sapiens
<400> 493
   guggcuacuc guuaauuaut t 21
<210> 494
   <211> 21
   <212> DNA
   <213> homo sapiens
<400> 494
   gguaucccuc aaccuacaat t 21
<210> 495
   <211> 21
   <212> DNA
   <213> homo sapiens
<400> 495
   agcauaccuc acuguucaat t 21
<210> 496
   <211> 21
   <212> DNA
   <213> homo sapiens
<400> 496
   uguacaacag gaugguaaat t 21
<210> 497
   <211> 21
   <212> DNA
   <213> homo sapiens
<400> 497
   ucuaauuguc aaugugaaat t 21
<210> 498
   <211> 21
   <212> DNA
   <213> homo sapiens
<400> 498
   gaacaaugca cuacaguaut t 21
<210> 499
   <211> 21
   <212> DNA
   <213> homo sapiens
<400> 499
   caagauugac uugagaguat t 21
<210> 500
   <211> 21
   <212> DNA
   <213> homo sapiens
<400> 500
   gagcaaacgu gacuuauuut t 21
<210> 501
   <211> 21
   <212> DNA
   <213> homo sapiens
<400> 501
   gaaaguuacc agucuauuat t 21
<210> 502
   <211> 21
   <212> DNA
   <213> homo sapiens
<400> 502
   caaugugguc aaccuucuat t 21
<210> 503
   <211> 21
   <212> DNA
   <213> homo sapiens
<400> 503
   ccagcuacau gaucagcuat t 21
<210> 504
   <211> 21
   <212> DNA
   <213> homo sapiens
<400> 504
   gaauuuccug ggacagcaat t 21
<210> 505
   <211> 21
   <212> DNA
   <213> homo sapiens
<400> 505
   agacgaacuu ggaaaucaut t 21
<210> 506
   <211> 21
   <212> DNA
   <213> homo sapiens
<400> 506
   cauggagucg uguacauuat t 21
<210> 507
   <211> 21
   <212> DNA
   <213> homo sapiens
<400> 507
   cgucauggau ccagaugaat t 21
<210> 508
   <211> 21
   <212> DNA
   <213> homo sapiens
<400> 508
   caggaaaccu ggagaaucat t 21
<210> 509
   <211> 21
   <212> DNA
   <213> homo sapiens
<400> 509
   cgcugacaug uacggucuat t 21
<210> 510
   <211> 21
   <212> DNA
   <213> homo sapiens
<400> 510
   ugauuauacu acaccagaat t 21
<210> 511
   <211> 21
   <212> DNA
   <213> homo sapiens
<400> 511
   gggaccuggc ggcacgaaat t 21
<210> 512
   <211> 21
   <212> DNA
   <213> homo sapiens
<400> 512
   cccauacccu ugugaagaat t 21
<210> 513
   <211> 21
   <212> DNA
   <213> homo sapiens
<400> 513
   gauacucuuu ggaaauugat t 21
<210> 514
   <211> 21
   <212> DNA
   <213> homo sapiens
<400> 514
   gucucaugga auugaacuat t 21
<210> 515
   <211> 21
   <212> DNA
   <213> homo sapiens
<400> 515
   cggcggugau ugccauguut t 21
<210> 516
   <211> 21
   <212> DNA
   <213> homo sapiens
<400> 516
   caggcauugu auugaaggat t 21
<210> 517
   <211> 21
   <212> DNA
   <213> homo sapiens
<400> 517
   ucagcauaag aaacuuguat t 21
<210> 518
   <211> 21
   <212> DNA
   <213> homo sapiens
<400> 518
   cauuguucuu ccgauaucat t 21
<210> 519
   <211> 21
   <212> DNA
   <213> homo sapiens
<400> 519
   cauguucucu aauagcacat t 21
<210> 520
   <211> 21
   <212> DNA
   <213> homo sapiens
<400> 520
   gucccucagu gauguagaat t 21
<210> 521
   <211> 21
   <212> DNA
   <213> homo sapiens
<400> 521
   agaucaugug guuuaaagat t 21
<210> 522
   <211> 21
   <212> DNA
   <213> homo sapiens
<400> 522
   cgugucacuu ugugcaagat t 21
<210> 523
   <211> 21
   <212> DNA
   <213> homo sapiens
<400> 523
   ggcuuuggcc caauaaucat t 21
<210> 524
   <211> 21
   <212> DNA
   <213> homo sapiens
<400> 524
   gugucagcuu uguacaaaut t 21
<210> 525
   <211> 21
   <212> DNA
   <213> homo sapiens
<400> 525
   gcuuggcccg ggauauuuat t 21
<210> 526
   <211> 21
   <212> DNA
   <213> homo sapiens
<400> 526
   gacggacagu gguaugguut t 21
<210> 527
   <211> 21
   <212> DNA
   <213> homo sapiens
<400> 527
   ggauaucacu ccgaugacat t 21
<210> 528
   <211> 21
   <212> DNA
   <213> homo sapiens
<400> 528
   gacagaucua cguuugagat t 21
<210> 529
   <211> 21
   <212> DNA
   <213> homo sapiens
<400> 529
   gcucuggauu uguggaggat t 21
<210> 530
   <211> 21
   <212> DNA
   <213> homo sapiens
<400> 530
   acuuggccuc ggucauuuat t 21
<210> 531
   <211> 21
   <212> DNA
   <213> homo sapiens
<400> 531
   cugcgaagua ccuugguuat t 21
<210> 532
   <211> 21
   <212> DNA
   <213> homo sapiens
<400> 532
   gccucugugg guuugccuat t 21
<210> 533
   <211> 21
   <212> DNA
   <213> homo sapiens
<400> 533
   cgacugccuu augaugccat t 21
<210> 534
   <211> 21
   <212> DNA
   <213> homo sapiens
<400> 534
   ccaguacggc accacucaat t 21
<210> 535
   <211> 21
   <212> DNA
   <213> homo sapiens
<400> 535
   cuuggauacu cuuuggaaat t 21
<210> 536
   <211> 21
   <212> DNA
   <213> homo sapiens
<400> 536
   cuggagaauc agacgacaat t 21
<210> 537
   <211> 21
   <212> DNA
   <213> homo sapiens
<400> 537
   ggcacgaaau auccucuuat t 21
<210> 538
   <211> 21
   <212> DNA
   <213> homo sapiens
<400> 538
   ccagucuauu auguacauat t 21
<210> 539
   <211> 21
   <212> DNA
   <213> homo sapiens
<400> 539
   cagcauaaga aacuuguaat t 21
<210> 540
   <211> 21
   <212> DNA
   <213> homo sapiens
<400> 540
   acuuguaaac cgagaccuat t 21
<210> 541
   <211> 21
   <212> DNA
   <213> homo sapiens
<400> 541
   cuuguaaacc gagaccuaat t 21
<210> 542
   <211> 21
   <212> DNA
   <213> homo sapiens
<400> 542
   gaaauauccu cuuaucggat t 21
<210> 543
   <211> 21
   <212> DNA
   <213> homo sapiens
<400> 543
   agcauaagaa acuuguaaat t 21
<210> 544
   <211> 21
   <212> DNA
   <213> homo sapiens
<400> 544
   ggaaaccugg agaaucagat t 21
<210> 545
   <211> 21
   <212> DNA
   <213> homo sapiens
<400> 545
   acucaggcau uguauugaat t 21
<210> 546
   <211> 21
   <212> DNA
   <213> homo sapiens
<400> 546
   augugaagcg gucaacaaat t 21
<210> 547
   <211> 21
   <212> DNA
   <213> homo sapiens
<400> 547
   cagcuacaug aucagcuaut t 21
<210> 548
   <211> 21
   <212> DNA
   <213> homo sapiens
<400> 548
   gcggcuacca guccggauat t 21
<210> 549
   <211> 21
   <212> DNA
   <213> homo sapiens
<400> 549
   acucaaacgc ugacauguat t 21
<210> 550
   <211> 21
   <212> DNA
   <213> homo sapiens
<400> 550
   cuacauuguu cuuccgauat t 21
<210> 551
   <211> 21
   <212> DNA
   <213> homo sapiens
<400> 551
   cgcucuuggu caacaggaat t 21
<210> 552
   <211> 21
   <212> DNA
   <213> homo sapiens
<400> 552
   cgcugauguc ggagcucaat t 21
<210> 553
   <211> 21
   <212> DNA
   <213> homo sapiens
<400> 553
   gcuucaccau cgaauccaat t 21
<210> 554
   <211> 21
   <212> DNA
   <213> homo sapiens
<400> 554
   cgaggucauu gugcaugaat t 21
<210> 555
   <211> 21
   <212> DNA
   <213> homo sapiens
<400> 555
   gccgauuauu ccuugguaat t 21
<210> 556
   <211> 21
   <212> DNA
   <213> homo sapiens
<400> 556
   gcaucgugug guacaaagat t 21
<210> 557
   <211> 21
   <212> DNA
   <213> homo sapiens
<400> 557
   cgcuucugcu cgacugcaat t 21
<210> 558
   <211> 21
   <212> DNA
   <213> homo sapiens
<400> 558
   gcggcuucag guagcugaat t 21
<210> 559
   <211> 21
   <212> DNA
   <213> homo sapiens
<400> 559
   gcaucuucga caagguguat t 21
<210> 560
   <211> 21
   <212> DNA
   <213> homo sapiens
<400> 560
   cgcccgaguu ccagugguat t 21
<210> 561
   <211> 21
   <212> DNA
   <213> homo sapiens
<400> 561
   caggcagcua cgucugcuat t 21
<210> 562
   <211> 21
   <212> DNA
   <213> homo sapiens
<400> 562
   ccgcuuucgg cauccacaat t 21
<210> 563
   <211> 21
   <212> DNA
   <213> homo sapiens
<400> 563
   ggaaagaaua agacugugat t 21
<210> 564
   <211> 21
   <212> DNA
   <213> homo sapiens
<400> 564
   cgaccuugaa caucacggat t 21
<210> 565
   <211> 21
   <212> DNA
   <213> homo sapiens
<400> 565
   cgagcacgag ggccacuaut t 21
<210> 566
   <211> 21
   <212> DNA
   <213> homo sapiens
<400> 566
   cgcuggagau gcagugcuut t 21
<210> 567
   <211> 21
   <212> DNA
   <213> homo sapiens
<400> 567
   caggcaacga gcucuaugat t 21
<210> 568
   <211> 21
   <212> DNA
   <213> homo sapiens
<400> 568
   gcauggagau cgugauccut t 21
<210> 569
   <211> 21
   <212> DNA
   <213> homo sapiens
<400> 569
   cggcucacgc agaacuugat t 21
<210> 570
   <211> 21
   <212> DNA
   <213> homo sapiens
<400> 570
   gagcgacgcu cccaacagat t 21
<210> 571
   <211> 21
   <212> DNA
   <213> homo sapiens
<400> 571
   cagacaucaa gacgggcuat t 21
<210> 572
   <211> 21
   <212> DNA
   <213> homo sapiens
<400> 572
   gugguuccuc caggaugaat t 21
<210> 573
   <211> 21
   <212> DNA
   <213> homo sapiens
<400> 573
   ggcccuacug caagguguut t 21
<210> 574
   <211> 21
   <212> DNA
   <213> homo sapiens
<400> 574
   ggagggcggu gaccacgcat t 21
<210> 575
   <211> 21
   <212> DNA
   <213> homo sapiens
<400> 575
   gcgaauaccu guccuacgat t 21
<210> 576
   <211> 21
   <212> DNA
   <213> homo sapiens
<400> 576
   gcacgaccug ggcucguaut t 21
<210> 577
   <211> 21
   <212> DNA
   <213> homo sapiens
<400> 577
   agcagccauu caucaacaat t 21
<210> 578
   <211> 21
   <212> DNA
   <213> homo sapiens
<400> 578
   gccuugcccg ggacaucuat t 21
<210> 579
   <211> 21
   <212> DNA
   <213> homo sapiens
<400> 579
   gacccaaggg cugcgucaat t 21
<210> 580
   <211> 21
   <212> DNA
   <213> homo sapiens
<400> 580
   uggcggacuc caaccagaat t 21
<210> 581
   <211> 21
   <212> DNA
   <213> homo sapiens
<400> 581
   agaaguaccu gucggugcat t 21
<210> 582
   <211> 21
   <212> DNA
   <213> homo sapiens
<400> 582
   aguacaucaa ggcacgcaut t 21
<210> 583
   <211> 21
   <212> DNA
   <213> homo sapiens
<400> 583
   aagccgacag cuacaaguat t 21
<210> 584
   <211> 21
   <212> DNA
   <213> homo sapiens
<400> 584
   gagcccgcug accauggaat t 21
<210> 585
   <211> 21
   <212> DNA
   <213> homo sapiens
<400> 585
   cagcgucgag uggcucaaat t 21
<210> 586
   <211> 21
   <212> DNA
   <213> homo sapiens
<400> 586
   cgacggcuuc accaucgaat t 21
<210> 587
   <211> 21
   <212> DNA
   <213> homo sapiens
<400> 587
   cgccgauuau uccuugguat t 21
<210> 588
   <211> 21
   <212> DNA
   <213> homo sapiens
<400> 588
   ccgaggucau ugugcaugat t 21
<210> 589
   <211> 21
   <212> DNA
   <213> homo sapiens
<400> 589
   cugaaagcau cuucgacaat t 21
<210> 590
   <211> 21
   <212> DNA
   <213> homo sapiens
<400> 590
   ugguaauaug aguaauacat t 21
<210> 591
   <211> 21
   <212> DNA
   <213> homo sapiens
<400> 591
   gagcaggcau agacaagaat t 21
<210> 592
   <211> 21
   <212> DNA
   <213> homo sapiens
<400> 592
   cgauuauucc uugguaauat t 21
<210> 593
   <211> 21
   <212> DNA
   <213> homo sapiens
<400> 593
   cagcaucgug ugguacaaat t 21
<210> 594
   <211> 21
   <212> DNA
   <213> homo sapiens
<400> 594
   gggagagcac cgaggucaut t 21
<210> 595
   <211> 21
   <212> DNA
   <213> homo sapiens
<400> 595
   cggcccacgc agacaucaat t 21
<210> 596
   <211> 21
   <212> DNA
   <213> homo sapiens
<400> 596
   cccgaguucc agugguacat t 21
<210> 597
   <211> 21
   <212> DNA
   <213> homo sapiens
<400> 597
   gguacaugcc aacgacacat t 21
<210> 598
   <211> 21
   <212> DNA
   <213> homo sapiens
<400> 598
   cgacugcaag aacgugcaut t 21
<210> 599
   <211> 21
   <212> DNA
   <213> homo sapiens
<400> 599
   gagguacaug ccaacgacat t 21
<210> 600
   <211> 21
   <212> DNA
   <213> homo sapiens
<400> 600
   cgagcucuau gacauccagt t 21
<210> 601
   <211> 21
   <212> DNA
   <213> homo sapiens
<400> 601
   gggccagauu guaagcuuat t 21
<210> 602
   <211> 21
   <212> DNA
   <213> homo sapiens
<400> 602
   gcacguugau gugaagauat t 21
<210> 603
   <211> 21
   <212> DNA
   <213> homo sapiens
<400> 603
   gggagauaac gugaacauat t 21
<210> 604
   <211> 21
   <212> DNA
   <213> homo sapiens
<400> 604
   agauauuggu guccuuaaat t 21
<210> 605
   <211> 21
   <212> DNA
   <213> homo sapiens
<400> 605
   ggaaugacau caaauuucat t 21
<210> 606
   <211> 21
   <212> DNA
   <213> homo sapiens
<400> 606
   gccucuaacu uguaaacaat t 21
<210> 607
   <211> 21
   <212> DNA
   <213> homo sapiens
<400> 607
   gggucugcag ugcaaugaat t 21
<210> 608
   <211> 21
   <212> DNA
   <213> homo sapiens
<400> 608
   ugaaguaccu gauauucuat t 21
<210> 609
   <211> 21
   <212> DNA
   <213> homo sapiens
<400> 609
   ugaucauauu gcaauugaat t 21
<210> 610
   <211> 21
   <212> DNA
   <213> homo sapiens
<400> 610
   ggacaauauu ggauggcuat t 21
<210> 611
   <211> 21
   <212> DNA
   <213> homo sapiens
<400> 611
   gccagaucau auagaaguat t 21
<210> 612
   <211> 21
   <212> DNA
   <213> homo sapiens
<400> 612
   cagcccugcu gauaccaaat t 21
<210> 613
   <211> 21
   <212> DNA
   <213> homo sapiens
<400> 613
   caaugacccu gauaguacat t 21
<210> 614
   <211> 21
   <212> DNA
   <213> homo sapiens
<400> 614
   gaaugggcug aaaucagaat t 21
<210> 615
   <211> 21
   <212> DNA
   <213> homo sapiens
<400> 615
   caugaagaug cgucaacaat t 21
<210> 616
   <211> 21
   <212> DNA
   <213> homo sapiens
<400> 616
   ccuuuauguu gaaugcuaut t 21
<210> 617
   <211> 21
   <212> DNA
   <213> homo sapiens
<400> 617
   gcuaauggaa agucacaaat t 21
<210> 618
   <211> 21
   <212> DNA
   <213> homo sapiens
<400> 618
   gccagucccg uuucauuuat t 21
<210> 619
   <211> 21
   <212> DNA
   <213> homo sapiens
<400> 619
   ggcgugagua caauuaguat t 21
<210> 620
   <211> 21
   <212> DNA
   <213> homo sapiens
<400> 620
   ggcuuggcaa cauauucaat t 21
<210> 621
   <211> 21
   <212> DNA
   <213> homo sapiens
<400> 621
   gugguuucau augcaauaat t 21
<210> 622
   <211> 21
   <212> DNA
   <213> homo sapiens
<400> 622
   acgugaauac cacgcuuuat t 21
<210> 623
   <211> 21
   <212> DNA
   <213> homo sapiens
<400> 623
   gggacuuuga agccuuaaut t 21
<210> 624
   <211> 21
   <212> DNA
   <213> homo sapiens
<400> 624
   cgggaugacu ugugcagaat t 21
<210> 625
   <211> 21
   <212> DNA
   <213> homo sapiens
<400> 625
   acccagaucc uacaauuuat t 21
<210> 626
   <211> 21
   <212> DNA
   <213> homo sapiens
<400> 626
   agauuguuac acucaacuat t 21
<210> 627
   <211> 21
   <212> DNA
   <213> homo sapiens
<400> 627
   augggagauu guuagcuuat t 21
<210> 628
   <211> 21
   <212> DNA
   <213> homo sapiens
<400> 628
   acaucagcuc ugagccuuat t 21
<210> 629
   <211> 21
   <212> DNA
   <213> homo sapiens
<400> 629
   cgugauugac acuggacaut t 21
<210> 630
   <211> 21
   <212> DNA
   <213> homo sapiens
<400> 630
   gaagcggccu aggacagaat t 21
<210> 631
   <211> 21
   <212> DNA
   <213> homo sapiens
<400> 631
   ugacuucggu gcuacuuaat t 21
<210> 632
   <211> 21
   <212> DNA
   <213> homo sapiens
<400> 632
   caucaagaag gauggguuat t 21
<210> 633
   <211> 21
   <212> DNA
   <213> homo sapiens
<400> 633
   aauuugaccu ggcaaccaat t 21
<210> 634
   <211> 21
   <212> DNA
   <213> homo sapiens
<400> 634
   ccuuucccau ccuaaucuat t 21
<210> 635
   <211> 21
   <212> DNA
   <213> homo sapiens
<400> 635
   agggcuacag acuggagaat t 21
<210> 636
   <211> 21
   <212> DNA
   <213> homo sapiens
<400> 636
   gaaugacauc aaauuucaat t 21
<210> 637
   <211> 21
   <212> DNA
   <213> homo sapiens
<400> 637
   cagauauugg uguccuuaat t 21
<210> 638
   <211> 21
   <212> DNA
   <213> homo sapiens
<400> 638
   cgugaacaua ucuuucaaat t 21
<210> 639
   <211> 21
   <212> DNA
   <213> homo sapiens
<400> 639
   cagaucauau agaaguaaat t 21
<210> 640
   <211> 21
   <212> DNA
   <213> homo sapiens
<400> 640
   cacguugaug ugaagauaat t 21
<210> 641
   <211> 21
   <212> DNA
   <213> homo sapiens
<400> 641
   cgaugcuaau ggaaagucat t 21
<210> 642
   <211> 21
   <212> DNA
   <213> homo sapiens
<400> 642
   ggaaggcgug aguacaauut t 21
<210> 643
   <211> 21
   <212> DNA
   <213> homo sapiens
<400> 643
   gcgugaguac aauuaguaut t 21
<210> 644
   <211> 21
   <212> DNA
   <213> homo sapiens
<400> 644
   acguugaugu gaagauaaat t 21
<210> 645
   <211> 21
   <212> DNA
   <213> homo sapiens
<400> 645
   cgugaguaca auuaguauat t 21
<210> 646
   <211> 21
   <212> DNA
   <213> homo sapiens
<400> 646
   gcaacauauu caagugacat t 21
<210> 647
   <211> 21
   <212> DNA
   <213> homo sapiens
<400> 647
   ggagauuguu agcuuaggat t 21
<210> 648
   <211> 21
   <212> DNA
   <213> homo sapiens
<400> 648
   ggauacgaac caugaagaut t 21
<210> 649
   <211> 21
   <212> DNA
   <213> homo sapiens
<400> 649
   cauccuaauc uacaaaggat t 21
<210> 650
   <211> 21
   <212> DNA
   <213> homo sapiens
<400> 650
   ggagauaacg ugaacauaut t 21
<210> 651
   <211> 21
   <212> DNA
   <213> homo sapiens
<400> 651
   ggucguuacc agagauuuat t 21
<210> 652
   <211> 21
   <212> DNA
   <213> homo sapiens
<400> 652
   acguggaguu cauguguaat t 21
<210> 653
   <211> 21
   <212> DNA
   <213> homo sapiens
<400> 653
   guugauaaau cugaguauat t 21
<210> 654
   <211> 21
   <212> DNA
   <213> homo sapiens
<400> 654
   gccacacucu gcaccgcuat t 21
<210> 655
   <211> 21
   <212> DNA
   <213> homo sapiens
<400> 655
   gagcgagggu caguuugaat t 21
<210> 656
   <211> 21
   <212> DNA
   <213> homo sapiens
<400> 656
   ccaaccucua acugcagaat t 21
<210> 657
   <211> 21
   <212> DNA
   <213> homo sapiens
<400> 657
   guggcuuauu aauuccgaut t 21
<210> 658
   <211> 21
   <212> DNA
   <213> homo sapiens
<400> 658
   cgcaggaugg ucccuuguat t 21
<210> 659
   <211> 21
   <212> DNA
   <213> homo sapiens
<400> 659
   caggguugcc cgccaacaat t 21
<210> 660
   <211> 21
   <212> DNA
   <213> homo sapiens
<400> 660
   gggaggcauc aguugcuaut t 21
<210> 661
   <211> 21
   <212> DNA
   <213> homo sapiens
<400> 661
   ccgucagcug uaacccucat t 21
<210> 662
   <211> 21
   <212> DNA
   <213> homo sapiens
<400> 662
   gggacauuca ccacaucgat t 21
<210> 663
   <211> 21
   <212> DNA
   <213> homo sapiens
<400> 663
   agaagugcau acaccgagat t 21
<210> 664
   <211> 21
   <212> DNA
   <213> homo sapiens
<400> 664
   gucggacgca acagagaaat t 21
<210> 665
   <211> 21
   <212> DNA
   <213> homo sapiens
<400> 665
   acaccugcau uguggagaat t 21
<210> 666
   <211> 21
   <212> DNA
   <213> homo sapiens
<400> 666
   gcaagauugg cccagacaat t 21
<210> 667
   <211> 21
   <212> DNA
   <213> homo sapiens
<400> 667
   gaggcauuau uugaccggat t 21
<210> 668
   <211> 21
   <212> DNA
   <213> homo sapiens
<400> 668
   ugccaauggc ggacucaaat t 21
<210> 669
   <211> 21
   <212> DNA
   <213> homo sapiens
<400> 669
   cagacugguc uuaggcaaat t 21
<210> 670
   <211> 21
   <212> DNA
   <213> homo sapiens
<400> 670
   gaucuugaag acugcuggat t 21
<210> 671
   <211> 21
   <212> DNA
   <213> homo sapiens
<400> 671
   ggcggguaac ucuaucggat t 21
<210> 672
   <211> 21
   <212> DNA
   <213> homo sapiens
<400> 672
   cuguggugcc cucugacaat t 21
<210> 673
   <211> 21
   <212> DNA
   <213> homo sapiens
<400> 673
   ucguggagua ugccuccaat t 21
<210> 674
   <211> 21
   <212> DNA
   <213> homo sapiens
<400> 674
   cguccggucg ggaccgagat t 21
<210> 675
   <211> 21
   <212> DNA
   <213> homo sapiens
<400> 675
   ccaaaugccu gguaccagat t 21
<210> 676
   <211> 21
   <212> DNA
   <213> homo sapiens
<400> 676
   aggcuaucgg gcuggacaat t 21
<210> 677
   <211> 21
   <212> DNA
   <213> homo sapiens
<400> 677
   cgcacaucca guggcuaaat t 21
<210> 678
   <211> 21
   <212> DNA
   <213> homo sapiens
<400> 678
   cacagaauug gaggcuacat t 21
<210> 679
   <211> 21
   <212> DNA
   <213> homo sapiens
<400> 679
   gcuucgagcc acgauugaat t 21
<210> 680
   <211> 21
   <212> DNA
   <213> homo sapiens
<400> 680
   guggaguauc cauggagaut t 21
<210> 681
   <211> 21
   <212> DNA
   <213> homo sapiens
<400> 681
   cacacugcgc ugguugaaat t 21
<210> 682
   <211> 21
   <212> DNA
   <213> homo sapiens
<400> 682
   cccgacaccc ggagcucuat t 21
<210> 683
   <211> 21
   <212> DNA
   <213> homo sapiens
<400> 683
   gagauuuacc caucgguaat t 21
<210> 684
   <211> 21
   <212> DNA
   <213> homo sapiens
<400> 684
   gggucguuac cagagauuut t 21
<210> 685
   <211> 21
   <212> DNA
   <213> homo sapiens
<400> 685
   ggaggcauca guugcuauat t 21
<210> 686
   <211> 21
   <212> DNA
   <213> homo sapiens
<400> 686
   agcgaggguc aguuugaaat t 21
<210> 687
   <211> 21
   <212> DNA
   <213> homo sapiens
<400> 687
   aguugauaaa ucugaguaut t 21
<210> 688
   <211> 21
   <212> DNA
   <213> homo sapiens
<400> 688
   agagauuuac ccaucgguat t 21
<210> 689
   <211> 21
   <212> DNA
   <213> homo sapiens
<400> 689
   gcaggauggu cccuuguaut t 21
<210> 690
   <211> 21
   <212> DNA
   <213> homo sapiens
<400> 690
   aaguugauaa aucugaguat t 21
<210> 691
   <211> 21
   <212> DNA
   <213> homo sapiens
<400> 691
   caagaagugc auacaccgat t 21
<210> 692
   <211> 21
   <212> DNA
   <213> homo sapiens
<400> 692
   uguaugucau cguggaguat t 21
<210> 693
   <211> 21
   <212> DNA
   <213> homo sapiens
<400> 693
   aguggcuuau uaauuccgat t 21
<210> 694
   <211> 21
   <212> DNA
   <213> homo sapiens
<400> 694
   gcaucauaau ggacucugut t 21
<210> 695
   <211> 21
   <212> DNA
   <213> homo sapiens
<400> 695
   aguuaauacc accgacaaat t 21
<210> 696
   <211> 21
   <212> DNA
   <213> homo sapiens
<400> 696
   ggagcaucau aauggacuct t 21
<210> 697
   <211> 21
   <212> DNA
   <213> homo sapiens
<400> 697
   uggcuuauua auuccgauat t 21
<210> 698
   <211> 21
   <212> DNA
   <213> homo sapiens
<400> 698
   aggcauuauu ugaccggaut t 21
<210> 699
   <211> 21
   <212> DNA
   <213> homo sapiens
<400> 699
   agucggacgc aacagagaat t 21
<210> 700
   <211> 21
   <212> DNA
   <213> homo sapiens
<400> 700
   cacgggacau ucaccacaut t 21
<210> 701
   <211> 21
   <212> DNA
   <213> homo sapiens
<400> 701
   ggcagauguu ccuaauaaat t 21
<210> 702
   <211> 21
   <212> DNA
   <213> homo sapiens
<400> 702
   ggugcuucag uuagaucaat t 21
<210> 703
   <211> 21
   <212> DNA
   <213> homo sapiens
<400> 703
   ggcuuaccau ccaaacaaut t 21
<210> 704
   <211> 21
   <212> DNA
   <213> homo sapiens
<400> 704
   cccacuaacu ggcuaauuat t 21
<210> 705
   <211> 21
   <212> DNA
   <213> homo sapiens
<400> 705
   gcaccuccau ggaaacgaat t 21
<210> 706
   <211> 21
   <212> DNA
   <213> homo sapiens
<400> 706
   cgaaucugac auuagaugat t 21
<210> 707
   <211> 21
   <212> DNA
   <213> homo sapiens
<400> 707
   cugagacacu caacaaguat t 21
<210> 708
   <211> 21
   <212> DNA
   <213> homo sapiens
<400> 708
   ggaguucuug gcacgucaut t 21
<210> 709
   <211> 21
   <212> DNA
   <213> homo sapiens
<400> 709
   gcuaugaggu ccugggaaat t 21
<210> 710
   <211> 21
   <212> DNA
   <213> homo sapiens
<400> 710
   cgaugaagga auaucucuut t 21
<210> 711
   <211> 21
   <212> DNA
   <213> homo sapiens
<400> 711
   cacaggagca ucuccucaat t 21
<210> 712
   <211> 21
   <212> DNA
   <213> homo sapiens
<400> 712
   ccagaucuau gccacaagat t 21
<210> 713
   <211> 21
   <212> DNA
   <213> homo sapiens
<400> 713
   ggcucuggac aggcacuaut t 21
<210> 714
   <211> 21
   <212> DNA
   <213> homo sapiens
<400> 714
   ggcuggaugc ucucguugat t 21
<210> 715
   <211> 21
   <212> DNA
   <213> homo sapiens
<400> 715
   caagauccug gcuaugcaut t 21
<210> 716
   <211> 21
   <212> DNA
   <213> homo sapiens
<400> 716
   gcuguugagg uaccuccaat t 21
<210> 717
   <211> 21
   <212> DNA
   <213> homo sapiens
<400> 717
   agaauaacca acacccugat t 21
<210> 718
   <211> 21
   <212> DNA
   <213> homo sapiens
<400> 718
   caugucaaau auuacagaut t 21
<210> 719
   <211> 21
   <212> DNA
   <213> homo sapiens
<400> 719
   aaguggaacg agacaagcat t 21
<210> 720
   <211> 21
   <212> DNA
   <213> homo sapiens
<400> 720
   gucucuucca accucugaat t 21
<210> 721
   <211> 21
   <212> DNA
   <213> homo sapiens
<400> 721
   cgccaggcuu accauccaat t 21
<210> 722
   <211> 21
   <212> DNA
   <213> homo sapiens
<400> 722
   guugaacguc acaucuuuat t 21
<210> 723
   <211> 21
   <212> DNA
   <213> homo sapiens
<400> 723
   ggccggugcu ucaguuagat t 21
<210> 724
   <211> 21
   <212> DNA
   <213> homo sapiens
<400> 724
   gcaccaucau ucccguugat t 21
<210> 725
   <211> 21
   <212> DNA
   <213> homo sapiens
<400> 725
   aggcuuacca uccaaacaat t 21
<210> 726
   <211> 21
   <212> DNA
   <213> homo sapiens
<400> 726
   gccaggcuua ccauccaaat t 21
<210> 727
   <211> 21
   <212> DNA
   <213> homo sapiens
<400> 727
   gugcuucagu uagaucaaat t 21
<210> 728
   <211> 21
   <212> DNA
   <213> homo sapiens
<400> 728
   caccaucauu cccguugaat t 21
<210> 729
   <211> 21
   <212> DNA
   <213> homo sapiens
<400> 729
   ccuccaugga aacgaagcat t 21
<210> 730
   <211> 21
   <212> DNA
   <213> homo sapiens
<400> 730
   agaggguuug gaagccagat t 21
<210> 731
   <211> 21
   <212> DNA
   <213> homo sapiens
<400> 731
   cuaacuggcu aauuagcaut t 21
<210> 732
   <211> 21
   <212> DNA
   <213> homo sapiens
<400> 732
   caucgaugaa ggaauaucut t 21
<210> 733
   <211> 21
   <212> DNA
   <213> homo sapiens
<400> 733
   aggcagaugu uccuaauaat t 21
<210> 734
   <211> 21
   <212> DNA
   <213> homo sapiens
<400> 734
   ccacuaacug gcuaauuagt t 21
<210> 735
   <211> 21
   <212> DNA
   <213> homo sapiens
<400> 735
   cggugcuuca guuagaucat t 21
<210> 736
   <211> 21
   <212> DNA
   <213> homo sapiens
<400> 736
   gcuuaccauc caaacaauut t 21
<210> 737
   <211> 21
   <212> DNA
   <213> homo sapiens
<400> 737
   ccgccaggcu uaccauccat t 21
<210> 738
   <211> 21
   <212> DNA
   <213> homo sapiens
<400> 738
   ugcauggccu ggucagcaat t 21
<210> 739
   <211> 21
   <212> DNA
   <213> homo sapiens
<400> 739
   gaggguuugg aagccagaut t 21
<210> 740
   <211> 21
   <212> DNA
   <213> homo sapiens
<400> 740
   ucaguuagau caaaccauut t 21
<210> 741
   <211> 21
   <212> DNA
   <213> homo sapiens
<400> 741
   gccuuugcac cuccauggat t 21
<210> 742
   <211> 21
   <212> DNA
   <213> homo sapiens
<400> 742
   ggaaacgaag caccaucaut t 21
<210> 743
   <211> 21
   <212> DNA
   <213> homo sapiens
<400> 743
   caggcuuacc auccaaacat t 21
<210> 744
   <211> 21
   <212> DNA
   <213> homo sapiens
<400> 744
   cagaucuaug ccacaagaat t 21
<210> 745
   <211> 21
   <212> DNA
   <213> homo sapiens
<400> 745
   ggaugcucuc guugacccut t 21
<210> 746
   <211> 21
   <212> DNA
   <213> homo sapiens
<400> 746
   uguugugauc aucgccuaut t 21
<210> 747
   <211> 21
   <212> DNA
   <213> homo sapiens
<400> 747
   ugagacacuc aacaaguaut t 21
<210> 748
   <211> 21
   <212> DNA
   <213> homo sapiens
<400> 748
   gaaacgaagc accaucauut t 21
<210> 749
   <211> 21
   <212> DNA
   <213> homo sapiens
<400> 749
   gcagauguuc cuaauaaagt t 21
<210> 750
   <211> 21
   <212> DNA
   <213> homo sapiens
<400> 750
   gucaucgugu uaccuccuat t 21
<210> 751
   <211> 21
   <212> DNA
   <213> homo sapiens
<400> 751
   ccacuaaauu gacacuuaat t 21
<210> 752
   <211> 21
   <212> DNA
   <213> homo sapiens
<400> 752
   ggaauugacc cacaagaaat t 21
<210> 753
   <211> 21
   <212> DNA
   <213> homo sapiens
<400> 753
   gcuuauucac aagacccaat t 21
<210> 754
   <211> 21
   <212> DNA
   <213> homo sapiens
<400> 754
   cuauguucua agaagugaat t 21
<210> 755
   <211> 21
   <212> DNA
   <213> homo sapiens
<400> 755
   ggaccgucua cucauccaat t 21
<210> 756
   <211> 21
   <212> DNA
   <213> homo sapiens
<400> 756
   aggugaagau cuuaguaaut t 21
<210> 757
   <211> 21
   <212> DNA
   <213> homo sapiens
<400> 757
   ccaaggcuga uuaaaccaat t 21
<210> 758
   <211> 21
   <212> DNA
   <213> homo sapiens
<400> 758
   gcagauaaac aguagugaut t 21
<210> 759
   <211> 21
   <212> DNA
   <213> homo sapiens
<400> 759
   ggcggaugcu guuacggaut t 21
<210> 760
   <211> 21
   <212> DNA
   <213> homo sapiens
<400> 760
   caagauucua gcuauacaut t 21
<210> 761
   <211> 21
   <212> DNA
   <213> homo sapiens
<400> 761
   gggccaugcg ggucaucuut t 21
<210> 762
   <211> 21
   <212> DNA
   <213> homo sapiens
<400> 762
   ccuacaugag guacugagat t 21
<210> 763
   <211> 21
   <212> DNA
   <213> homo sapiens
<400> 763
   gaagcgcuac uuggucaaat t 21
<210> 764
   <211> 21
   <212> DNA
   <213> homo sapiens
<400> 764
   gugauaugua ccuaacgaat t 21
<210> 765
   <211> 21
   <212> DNA
   <213> homo sapiens
<400> 765
   agaggaauga aguacucaut t 21
<210> 766
   <211> 21
   <212> DNA
   <213> homo sapiens
<400> 766
   agcucugacu accacccaat t 21
<210> 767
   <211> 21
   <212> DNA
   <213> homo sapiens
<400> 767
   cccuugcaug guuuagaaat t 21
<210> 768
   <211> 21
   <212> DNA
   <213> homo sapiens
<400> 768
   acauagaauu aacacaugat t 21
<210> 769
   <211> 21
   <212> DNA
   <213> homo sapiens
<400> 769
   guaccgcaau gugacuuaat t 21
<210> 770
   <211> 21
   <212> DNA
   <213> homo sapiens
<400> 770
   ggacaugggc aacaauacat t 21
<210> 771
   <211> 21
   <212> DNA
   <213> homo sapiens
<400> 771
   cuccaauaac agcaggucat t 21
<210> 772
   <211> 21
   <212> DNA
   <213> homo sapiens
<400> 772
   gaaccaccug ccuauauuut t 21
<210> 773
   <211> 21
   <212> DNA
   <213> homo sapiens
<400> 773
   gaaguuucau cgucaaggut t 21
<210> 774
   <211> 21
   <212> DNA
   <213> homo sapiens
<400> 774
   acacggacga accuugaaat t 21
<210> 775
   <211> 21
   <212> DNA
   <213> homo sapiens
<400> 775
   aggauuuaag uuuaccucat t 21
<210> 776
   <211> 21
   <212> DNA
   <213> homo sapiens
<400> 776
   acgccuucau uggccagaat t 21
<210> 777
   <211> 21
   <212> DNA
   <213> homo sapiens
<400> 777
   cacuaaauug acacuuaaat t 21
<210> 778
   <211> 21
   <212> DNA
   <213> homo sapiens
<400> 778
   cauggcacuc uauguucuat t 21
<210> 779
   <211> 21
   <212> DNA
   <213> homo sapiens
<400> 779
   ggugaagauc uuaguaauut t 21
<210> 780
   <211> 21
   <212> DNA
   <213> homo sapiens
<400> 780
   gucuacucau ccaauguuat t 21
<210> 781
   <211> 21
   <212> DNA
   <213> homo sapiens
<400> 781
   ggcacucuau guucuaagat t 21
<210> 782
   <211> 21
   <212> DNA
   <213> homo sapiens
<400> 782
   gcaaugugac uuaaugccat t 21
<210> 783
   <211> 21
   <212> DNA
   <213> homo sapiens
<400> 783
   gcugauuaaa ccaaggcuat t 21
<210> 784
   <211> 21
   <212> DNA
   <213> homo sapiens
<400> 784
   aggaauugac ccacaagaat t 21
<210> 785
   <211> 21
   <212> DNA
   <213> homo sapiens
<400> 785
   cagauaaaca guagugauat t 21
<210> 786
   <211> 21
   <212> DNA
   <213> homo sapiens
<400> 786
   gaaguauccu ucagccugat t 21
<210> 787
   <211> 21
   <212> DNA
   <213> homo sapiens
<400> 787
   cagacauaga auuaacacat t 21
<210> 788
   <211> 21
   <212> DNA
   <213> homo sapiens
<400> 788
   gaggacaugg gcaacaauat t 21
<210> 789
   <211> 21
   <212> DNA
   <213> homo sapiens
<400> 789
   uauaggaauu gacccacaat t 21
<210> 790
   <211> 21
   <212> DNA
   <213> homo sapiens
<400> 790
   gaauugaccc acaagaaaut t 21
<210> 791
   <211> 21
   <212> DNA
   <213> homo sapiens
<400> 791
   gguggccacu ccaauaacat t 21
<210> 792
   <211> 21
   <212> DNA
   <213> homo sapiens
<400> 792
   gagugaacaa auuuacagat t 21
<210> 793
   <211> 21
   <212> DNA
   <213> homo sapiens
<400> 793
   agugauaguu guaccgcaat t 21
<210> 794
   <211> 21
   <212> DNA
   <213> homo sapiens
<400> 794
   cgaaggaccg ucuacucaut t 21
<210> 795
   <211> 21
   <212> DNA
   <213> homo sapiens
<400> 795
   gugaagaucu uaguaauuat t 21
<210> 796
   <211> 21
   <212> DNA
   <213> homo sapiens
<400> 796
   caucgucaag guuguuucat t 21
<210> 797
   <211> 21
   <212> DNA
   <213> homo sapiens
<400> 797
   gcuauacaug gcuugaucat t 21
<210> 798
   <211> 21
   <212> DNA
   <213> homo sapiens
<400> 798
   gaagaucuua guaauuacat t 21
<210> 799
   <211> 21
   <212> DNA
   <213> homo sapiens
<400> 799
   cgcuacuugg ucaaauucat t 21
<210> 800
   <211> 21
   <212> DNA
   <213> homo sapiens
<400> 800
   auauguaccu aacgaaguat t 21
<210> 801
   <211> 21
   <212> DNA
   <213> homo sapiens
<400> 801
   gaggaagacu guuacuacat t 21
<210> 802
   <211> 21
   <212> DNA
   <213> homo sapiens
<400> 802
   guugcuagau uaucguccat t 21
<210> 803
   <211> 21
   <212> DNA
   <213> homo sapiens
<400> 803
   gauggccaau ucugccauat t 21
<210> 804
   <211> 21
   <212> DNA
   <213> homo sapiens
<400> 804
   cccuguccuc caggugaaat t 21
<210> 805
   <211> 21
   <212> DNA
   <213> homo sapiens
<400> 805
   aguguuaaug cccaagugat t 21
<210> 806
   <211> 21
   <212> DNA
   <213> homo sapiens
<400> 806
   ccaagugcaa agaggaagut t 21
<210> 807
   <211> 21
   <212> DNA
   <213> homo sapiens
<400> 807
   aucaaggaau gcacacucat t 21
<210> 808
   <211> 21
   <212> DNA
   <213> homo sapiens
<400> 808
   gugcccugcc aagaagggat t 21
<210> 809
   <211> 21
   <212> DNA
   <213> homo sapiens
<400> 809
   agcuagggac ugcacaguct t 21
<210> 810
   <211> 21
   <212> DNA
   <213> homo sapiens
<400> 810
   ggaaggccug caucaugaut t 21
<210> 811
   <211> 21
   <212> DNA
   <213> homo sapiens
<400> 811
   cuacaguuga gacucagaat t 21
<210> 812
   <211> 21
   <212> DNA
   <213> homo sapiens
<400> 812
   uacgucuguu gcuagauuat t 21
<210> 813
   <211> 21
   <212> DNA
   <213> homo sapiens
<400> 813
   agacuguuac uacaguugat t 21
<210> 814
   <211> 21
   <212> DNA
   <213> homo sapiens
<400> 814
   gacuguuacu acaguugagt t 21
<210> 815
   <211> 21
   <212> DNA
   <213> homo sapiens
<400> 815
   gguucuuacg ucuguugcut t 21
<210> 816
   <211> 21
   <212> DNA
   <213> homo sapiens
<400> 816
   acuacaguug agacucagat t 21
<210> 817
   <211> 21
   <212> DNA
   <213> homo sapiens
<400> 817
   guccuccagg ugaaaggaat t 21
<210> 818
   <211> 21
   <212> DNA
   <213> homo sapiens
<400> 818
   uugcuagauu aucguccaat t 21
<210> 819
   <211> 21
   <212> DNA
   <213> homo sapiens
<400> 819
   ggaagacugu uacuacagut t 21
<210> 820
   <211> 21
   <212> DNA
   <213> homo sapiens
<400> 820
   uggaaggccu gcaucaugat t 21
<210> 821
   <211> 21
   <212> DNA
   <213> homo sapiens
<400> 821
   uccaagggau uggaauugat t 21
<210> 822
   <211> 21
   <212> DNA
   <213> homo sapiens
<400> 822
   ggaaagcuag ggacugcact t 21
<210> 823
   <211> 21
   <212> DNA
   <213> homo sapiens
<400> 823
   guugagacuc agaacuuggt t 21
<210> 824
   <211> 21
   <212> DNA
   <213> homo sapiens
<400> 824
   cgucuguugc uagauuauct t 21
<210> 825
   <211> 21
   <212> DNA
   <213> homo sapiens
<400> 825
   auggccaauu cugccauaat t 21
<210> 826
   <211> 21
   <212> DNA
   <213> homo sapiens
<400> 826
   aguugagacu cagaacuugt t 21
<210> 827
   <211> 21
   <212> DNA
   <213> homo sapiens
<400> 827
   gaaagcuagg gacugcacat t 21
<210> 828
   <211> 21
   <212> DNA
   <213> homo sapiens
<400> 828
   ugacaucaac uccaagggat t 21
<210> 829
   <211> 21
   <212> DNA
   <213> homo sapiens
<400> 829
   ugauggccaa uucugccaut t 21
<210> 830
   <211> 21
   <212> DNA
   <213> homo sapiens
<400> 830
   gccugcauca ugauggccat t 21
<210> 831
   <211> 21
   <212> DNA
   <213> homo sapiens
<400> 831
   guguuaaugc ccaagugact t 21
<210> 832
   <211> 21
   <212> DNA
   <213> homo sapiens
<400> 832
   ggccaauucu gccauaagct t 21
<210> 833
   <211> 21
   <212> DNA
   <213> homo sapiens
<400> 833
   gucuguugcu agauuaucgt t 21
<210> 834
   <211> 21
   <212> DNA
   <213> homo sapiens
<400> 834
   acuguuacua caguugagat t 21
<210> 835
   <211> 21
   <212> DNA
   <213> homo sapiens
<400> 835
   cuuacgucug uugcuagaut t 21
<210> 836
   <211> 21
   <212> DNA
   <213> homo sapiens
<400> 836
   uguuaaugcc caagugacut t 21
<210> 837
   <211> 21
   <212> DNA
   <213> homo sapiens
<400> 837
   caauucugcc auaagcccut t 21
<210> 838
   <211> 21
   <212> DNA
   <213> homo sapiens
<400> 838
   uuacuacagu ugagacucat t 21
<210> 839
   <211> 21
   <212> DNA
   <213> homo sapiens
<400> 839
   acaguugaga cucagaacut t 21
<210> 840
   <211> 21
   <212> DNA
   <213> homo sapiens
<400> 840
   ugcuagauua ucguccaaat t 21
<210> 841
   <211> 21
   <212> DNA
   <213> homo sapiens
<400> 841
   caagggauug gaauugaggt t 21
<210> 842
   <211> 21
   <212> DNA
   <213> homo sapiens
<400> 842
   augauggcca auucugccat t 21
<210> 843
   <211> 21
   <212> DNA
   <213> homo sapiens
<400> 843
   aggaagacug uuacuacagt t 21
<210> 844
   <211> 21
   <212> DNA
   <213> homo sapiens
<400> 844
   ucuuacgucu guugcuagat t 21
<210> 845
   <211> 21
   <212> DNA
   <213> homo sapiens
<400> 845
   ucuguugcua gauuaucgut t 21
<210> 846
   <211> 21
   <212> DNA
   <213> homo sapiens
<400> 846
   uguuacuaca guugagacut t 21
<210> 847
   <211> 21
   <212> DNA
   <213> homo sapiens
<400> 847
   auucugccau aagcccugut t 21
<210> 848
   <211> 21
   <212> DNA
   <213> homo sapiens
<400> 848
   uuacgucugu ugcuagauut t 21
<210> 849
   <211> 21
   <212> DNA
   <213> homo sapiens
<400> 849
   uggccaauuc ugccauaagt t 21
<210> 850
   <211> 21
   <212> DNA
   <213> homo sapiens
<400> 850
   gaaccagacu gcgugcccut t 21
<210> 851
   <211> 21
   <212> DNA
   <213> homo sapiens
<400> 851
   gggugucaua ggugacaaat t 21
<210> 852
   <211> 21
   <212> DNA
   <213> homo sapiens
<400> 852
   gaccaggcuu gcucuauaat t 21
<210> 853
   <211> 21
   <212> DNA
   <213> homo sapiens
<400> 853
   ggaagauacc acaaucagat t 21
<210> 854
   <211> 21
   <212> DNA
   <213> homo sapiens
<400> 854
   gcauaauugu ccugaaauat t 21
<210> 855
   <211> 21
   <212> DNA
   <213> homo sapiens
<400> 855
   ggaggaaaua cuacauuaat t 21
<210> 856
   <211> 21
   <212> DNA
   <213> homo sapiens
<400> 856
   cgcagguaac gaugggaaat t 21
<210> 857
   <211> 21
   <212> DNA
   <213> homo sapiens
<400> 857
   ggaugaucca acugaacuat t 21
<210> 858
   <211> 21
   <212> DNA
   <213> homo sapiens
<400> 858
   gcuuaguguc agaaggcaat t 21
<210> 859
   <211> 21
   <212> DNA
   <213> homo sapiens
<400> 859
   gaggcggcac acccuauaat t 21
<210> 860
   <211> 21
   <212> DNA
   <213> homo sapiens
<400> 860
   ccuauauuuc agauggaaat t 21
<210> 861
   <211> 21
   <212> DNA
   <213> homo sapiens
<400> 861
   gacugaagcu ggugcgcaat t 21
<210> 862
   <211> 21
   <212> DNA
   <213> homo sapiens
<400> 862
   acccugauuu cuacaucaat t 21
<210> 863
   <211> 21
   <212> DNA
   <213> homo sapiens
<400> 863
   gguguucuau ugcugagaat t 21
<210> 864
   <211> 21
   <212> DNA
   <213> homo sapiens
<400> 864
   cgaccuauaa gaagccuuat t 21
<210> 865
   <211> 21
   <212> DNA
   <213> homo sapiens
<400> 865
   gcggcgaaug gaucuuugat t 21
<210> 866
   <211> 21
   <212> DNA
   <213> homo sapiens
<400> 866
   cggagcacua ccucaucaat t 21
<210> 867
   <211> 21
   <212> DNA
   <213> homo sapiens
<400> 867
   agcggaugag cgucauaaat t 21
<210> 868
   <211> 21
   <212> DNA
   <213> homo sapiens
<400> 868
   ccaacguguc cuacaaauat t 21
<210> 869
   <211> 21
   <212> DNA
   <213> homo sapiens
<400> 869
   cgugaaccuc ggcaggguat t 21
<210> 870
   <211> 21
   <212> DNA
   <213> homo sapiens
<400> 870
   guuugagugu gcucagauat t 21
<210> 871
   <211> 21
   <212> DNA
   <213> homo sapiens
<400> 871
   gaauugaccu guacaaagat t 21
<210> 872
   <211> 21
   <212> DNA
   <213> homo sapiens
<400> 872
   cgauaccucu caagucaaat t 21
<210> 873
   <211> 21
   <212> DNA
   <213> homo sapiens
<400> 873
   aauucgucca gaaacacaat t 21
<210> 874
   <211> 21
   <212> DNA
   <213> homo sapiens
<400> 874
   gugaggaaug gaagcucuat t 21
<210> 875
   <211> 21
   <212> DNA
   <213> homo sapiens
<400> 875
   gcagcgagag ccaagugaat t 21
<210> 876
   <211> 21
   <212> DNA
   <213> homo sapiens
<400> 876
   cauagauauc ggccggguat t 21
<210> 877
   <211> 21
   <212> DNA
   <213> homo sapiens
<400> 877
   ggccagucgg aauugagaat t 21
<210> 878
   <211> 21
   <212> DNA
   <213> homo sapiens
<400> 878
   aaucaacuuu acuguaauat t 21
<210> 879
   <211> 21
   <212> DNA
   <213> homo sapiens
<400> 879
   cggaauacgc cuguguuaat t 21
<210> 880
   <211> 21
   <212> DNA
   <213> homo sapiens
<400> 880
   gggcguacag agcacauuut t 21
<210> 881
   <211> 21
   <212> DNA
   <213> homo sapiens
<400> 881
   cggacaagca uuucaacuat t 21
<210> 882
   <211> 21
   <212> DNA
   <213> homo sapiens
<400> 882
   cggcgugcuc uggagccaat t 21
<210> 883
   <211> 21
   <212> DNA
   <213> homo sapiens
<400> 883
   gaguugaggu gucagagaat t 21
<210> 884
   <211> 21
   <212> DNA
   <213> homo sapiens
<400> 884
   guuugaucug agcuccuuat t 21
<210> 885
   <211> 21
   <212> DNA
   <213> homo sapiens
<400> 885
   cgaucugacu gaauguucat t 21
<210> 886
   <211> 21
   <212> DNA
   <213> homo sapiens
<400> 886
   aacggagucu cguacuauat t 21
<210> 887
   <211> 21
   <212> DNA
   <213> homo sapiens
<400> 887
   cggacacuuc ucuauucaut t 21
<210> 888
   <211> 21
   <212> DNA
   <213> homo sapiens
<400> 888
   ccaccuuuac ugugcuuaut t 21
<210> 889
   <211> 21
   <212> DNA
   <213> homo sapiens
<400> 889
   ggaacgccua agcuguuaat t 21
<210> 890
   <211> 21
   <212> DNA
   <213> homo sapiens
<400> 890
   gguaacgaug ggaaaggaat t 21
<210> 891
   <211> 21
   <212> DNA
   <213> homo sapiens
<400> 891
   cccugauuuc uacaucaaut t 21
<210> 892
   <211> 21
   <212> DNA
   <213> homo sapiens
<400> 892
   cguggaggaa auacuacaut t 21
<210> 893
   <211> 21
   <212> DNA
   <213> homo sapiens
<400> 893
   gaccuauaag aagccuuaat t 21
<210> 894
   <211> 21
   <212> DNA
   <213> homo sapiens
<400> 894
   ggagcacuac cucaucaaut t 21
<210> 895
   <211> 21
   <212> DNA
   <213> homo sapiens
<400> 895
   ggagguucau ccuauauuut t 21
<210> 896
   <211> 21
   <212> DNA
   <213> homo sapiens
<400> 896
   agcggagguu cauccuauat t 21
<210> 897
   <211> 21
   <212> DNA
   <213> homo sapiens
<400> 897
   guguccuaca aauacucaat t 21
<210> 898
   <211> 21
   <212> DNA
   <213> homo sapiens
<400> 898
   guuccaacgu guccuacaat t 21
<210> 899
   <211> 21
   <212> DNA
   <213> homo sapiens
<400> 899
   ggcuugcucu auaagggaut t 21
<210> 900
   <211> 21
   <212> DNA
   <213> homo sapiens
<400> 900
   ccaccaggau cacguuugat t 21
<210> 901
   <211> 21
   <212> DNA
   <213> homo sapiens
<400> 901
   ucgaaguacu cagcguaagt t 21
<210> 902
   <211> 21
   <212> DNA
   <213> homo sapiens
<400> 902
   cuuacgcuga guacuucgat t 21
<210> 903
   <211> 21
   <212> DNA
   <213> homo sapiens
<400> 903
   cuuacgcuga guacuucgat t 21
<210> 904
   <211> 21
   <212> DNA
   <213> homo sapiens
<400> 904
   ucgaaguacu cagcguaagt t 21
<210> 905
   <211> 19
   <212> DNA
   <213> homo sapiens
<400> 905
   ucgaaguacu cagcguaag 19
<210> 906
   <211> 19
   <212> DNA
   <213> homo sapiens
<400> 906
   cuuacgcuga guacuucga 19
<210> 907
   <211> 19
   <212> DNA
   <213> homo sapiens
<400> 907
   ucgaaguacu cagcguaag 19
<210> 908
   <211> 19
   <212> DNA
   <213> homo sapiens
<400> 908
   cuuacgcuga guacuucga 19
<210> 909
   <211> 21
   <212> DNA
   <213> homo sapiens
<400> 909
   uugagguuug aaaucgaccc t 21
<210> 910
   <211> 21
   <212> DNA
   <213> homo sapiens
<400> 910
   ggucgauuuc aaaccucaat t 21
<210> 911
   <211> 23
   <212> DNA
   <213> homo sapiens
<400> 911
   uaauuuguuc cugucuuccd adg 23
<210> 912
   <211> 21
   <212> DNA
   <213> homo sapiens
<400> 912
   ggaagacagg aacaaauuat t 21
<210> 913
   <211> 21
   <212> DNA
   <213> homo sapiens
<400> 913
   acgugacacg uucggagaat t 21
<210> 914
   <211> 21
   <212> DNA
   <213> homo sapiens
<400> 914
   uucuccgaac gugucacgut t 21
<210> 915
   <211> 19
   <212> DNA
   <213> homo sapiens
<400> 915
   uugagguuug aaaucgacc 19
<210> 916
   <211> 19
   <212> DNA
   <213> homo sapiens
<400> 916
   ggucgauuuc aaaccucaa 19
<210> 917
   <211> 19
   <212> DNA
   <213> homo sapiens
<400> 917
   uaauuuguuc cugucuucc 19
<210> 918
   <211> 19
   <212> DNA
   <213> homo sapiens
<400> 918
   ggaagacagg aacaaauua 19
<210> 919
   <211> 19
   <212> DNA
   <213> homo sapiens
<400> 919
   acgugacacg uucggagaa 19
<210> 920
   <211> 19
   <212> DNA
   <213> homo sapiens
<400> 920
   uucuccgaac gugucacgu 19
<210> 921
   <211> 19
   <212> DNA
   <213> homo sapiens
<400> 921
   uugagguuug aaaucgacc 19
<210> 922
   <211> 19
   <212> DNA
   <213> homo sapiens
<400> 922
   ggucgauuuc aaaccucaa 19
<210> 923
   <211> 19
   <212> DNA
   <213> homo sapiens
<400> 923
   uaauuuguuc cugucuucc 19
<210> 924
   <211> 19
   <212> DNA
   <213> homo sapiens
<400> 924
   ggaagacagg aacaaauua 19
<210> 925
   <211> 19
   <212> DNA
   <213> homo sapiens
<400> 925
   uucuucuuua auuaacacc 19
<210> 926
   <211> 19
   <212> DNA
   <213> homo sapiens
<400> 926
   gguguuaauu aaagaagaa 19
<210> 927
   <211> 19
   <212> DNA
   <213> homo sapiens
<400> 927
   ucugaguuug uaaauaucg 19
<210> 928
   <211> 19
   <212> DNA
   <213> homo sapiens
<400> 928
   cgauauuuac aaacucaga 19
<210> 929
   <211> 19
   <212> DNA
   <213> homo sapiens
<400> 929
   acgugacacg uucggagaa 19
<210> 930
   <211> 19
   <212> DNA
   <213> homo sapiens
<400> 930
   uucuccgaac gugucacgt 19

The application further provides the following numbered clauses:
1. Short interfering ribonucleic acid (siRNA) for oral administration, said siRNA comprising two separate RNA strands that are complementary to each other over at least 15 nucleotides, wherein each strand is 49 nucleotides or less, and wherein at least one of which strands contains at least one chemical modification.
2. siRNA according to clause 1, comprising at least one modified nucleotide.
3. siRNA according to clause 1 or 2 wherein said siRNA comprises at least one 3' end cap.
4. siRNA according to clause 2 or 3, wherein said modified nucleotide is selected from among 2' alkoxyribonucleotide, 2' alkoxyalkoxy ribonucleotide, a locked nucleic acid ribonucleotide (LNA), 2'-fluoro ribonucleotide, morpholino nucleotide.
5. siRNA according to any one of clauses 2-4 wherein said modified nucleotide is selected from among nucleotides having a modified internucleoside linkage selected from among phosphorothioate, phosphorodithioate, phosphoramidate, boranophosphonoate, and amide linkages.
6. siRNA according to any one of clauses 1-5 wherein said two RNA strands are fully complementary to each other.
7. siRNA according to clause 1-6 comprising a 1 to 6 nucleotide overhang on at least one of the 5' end or 3' end.
8. siRNA according to any one of clauses 3-7 wherein said at least one 3' cap, when present, is a chemical moiety conjugated to the 3' end via the 3' carbon and is selected from among compounds of Formula I: 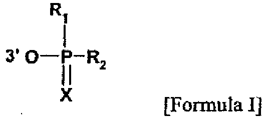 wherein
   X is O or S
   R₁ and R₂ are independently OH, NH₂, SH, alkyl, aryl, alkyl-aryl, aryl-alkyl, where alkyl, aryl, alkyl-aryl, aryl-alkyl can be substituted by additional heteroatoms and functional groups, preferably a heteroatom selected from the group of N, O, or S or a functional group selected from the group OH, NH₂, SH, carboxylic acid or ester;
   or R₁ and R₂ may be of formula Y-Z where Y is O, N, S and Z is H, alkyl, aryl, alkyl-aryl, aryl-alkyl, where alkyl, aryl, alkyl-aryl, aryl-alkyl can be substituted by additional heteroatoms, preferably a heteroatom selected from the group of N, O, or S.
9. siRNA according to any one of clauses 1-8 wherein at least one strand is complementary over at least 15 nucleotides to the mRNA or pre-mRNA of VEGFR-1, VEGFR-2, VEGFR3, Tie2, bFGFR, IL8RA, IL8RB, Fas, or IGF2R.
10. siRNA according to any one of clauses 1-9 wherein at least one strand comprises a sequence selected from SEQ ID NO 1-900.
11. siRNA chosen from the group consisting of SEQ ID NO 901-930.
12. siRNA according to any one of clauses 1 to 11 having stability in a standard gastric acid assay that is greater than an unmodified siRNA with the same nucleotide sequence.
13. siRNA according to any one of clauses 1 to 11 having stability in a standard gastric acid assay that is greater than or equal to 50% after 30 minutes exposure.
14. siRNA according to any one of clauses 1 to 11 having stability in a standard serum assay is greater than unmodified siRNA.
15. siRNA according to any one of clauses 1 to 11 having stability in a standard serum assay is greater than or equal to 50% after 30 minutes exposure.
16. siRNA of clause 1 to 11 having stability in a standard intestinal lavage assay that is greater than unmodified siRNA.
17. siRNA according to any one of clauses 1 to 11 having an enhanced oral bioavailability compared to an unmodified siRNA of the same nucleotide sequence.
18. Pharmaceutical composition comprising an siRNA according to any one of clauses 1-17.
19. siRNA according to any one of clauses 1 to 17 for use as a medicament.
20. Use of an siRNA according to any one of clauses 1 to 17 in the preparation of a medicament for treating an angiogenic disorder.
21. Use according to clause 20, wherein the gene targeted by the siRNA is expressed on endothelial cells.
22. Use according to clause 21, wherein the gene targeted by the siRNA is chosen from the group consisting of; VEGFR-1 (GenBank Accession # AF06365); VEGFR-2 (GenBank Accession # AF063658); VEGFR-3 (GenBank Accession # (NM_002020); Tie2 (TEK) (GenBank Accession # NM_000459); bFGFR (GenBank Accession # M60485); IL8RA (GenBank Accession # L19591); IL8RB (GenBank Accession # L19593); Fas (GenBank Accession # X89101); IGF2R (GenBank Accession # NM_000876).
23. Use of an siRNA according to any one of clauses 1 to 22 to inhibit an angiogenic process *in vitro.*

## Claims

1. A short interfering ribonucleic acid (siRNA), said siRNA comprising two RNA strands that are complementary to each other over at least 15 nucleotides, wherein each strand is 49 nucleotides or less, and wherein the 3'-terminus of at least one strand comprises a modification at the 3' carbon, wherein the modification is:

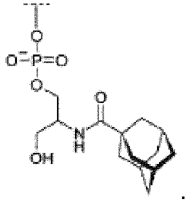

2. The siRNA according to claim 1, wherein both strands comprise a modification at the 3' carbon, wherein the modification is:

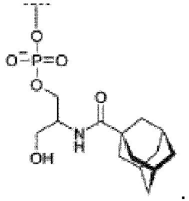

3. The siRNA according to claim 1, wherein each strand is 19 nucleotides.

4. The siRNA according to claim 3, wherein the first two base-pairing nucleotides at the 3' end of each strand are modified.

5. The siRNA according to claim 3, wherein the first two base-pairing nucleotides at the 3' end of each strand are modified, wherein each modified nucleotide is selected from among nucleotides having a modified internucleoside linkage selected from among phosphorothioate, phosphorodithioate, boranophosphonoate, and amide linkages.

6. The siRNA according to claim 3, wherein the first two base-pairing nucleotides at the 3' end of each strand are 2'-methoxyethyl ribonucleotides residues.

7. The siRNA according to claim 3, wherein the two strands are complementary to each other over 19 nucleotides.

8. The siRNA according to claim 1, wherein one end of the siRNA is blunt-ended.

9. The siRNA according to claim 1, wherein both ends of the siRNA are blunt-ended.

10. The siRNA according to claim 1, wherein the two strands are fully complementary to each other over 19 nucleotides and wherein the siRNA is blunt-ended.

11. The siRNA according to claim 1, wherein at least one additional nucleotide is modified.

12. The siRNA according to claim 1, comprising a 1- to 6-nucleotide overhang on at least one of the 5' end or 3' end.

13. Pharmaceutical composition comprising the siRNA according to claim 1, and a pharmaceutically acceptable carrier.

14. The siRNA according to claim 1, for use as a medicament.

15. The siRNA according to claim 1, for use as a medicament to be administered orally, topically, parenterally, by inhalation or spray, or rectally, or by percutaneous, subcutaneous, intravascular, intramuscular, intraperitoneal, intrathecal, or infusion technique.

## Patentansprüche

1. Short-Interfering-Ribonukleinsäure (siRNA), wobei die siRNA zwei RNA-Stränge umfasst, die über mindestens 15 Nukleotide komplementär zueinander sind, wobei die Stränge jeweils eine Länge von 49 Nukleotiden oder weniger aufweisen, und wobei der 3'-Terminus mindestens eines Strangs eine Modifikation am 3'-Kohlenstoff umfasst, wobei es sich bei der Modifikation um: 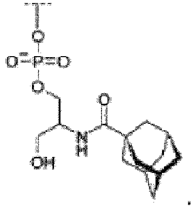 handelt.

2. siRNA nach Anspruch 1, wobei beide Stränge eine Modifikation am 3'-Kohlenstoff umfassen, wobei es sich bei der Modifikation um: 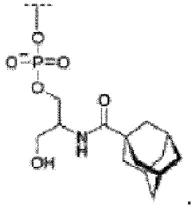 handelt.

3. siRNA nach Anspruch 1, wobei jeder Strang eine Länge von 19 Nukleotiden aufweist.

4. siRNA nach Anspruch 3, wobei die ersten beiden basengepaarten Nukleotide am 3'-Ende jedes Strangs modifiziert sind.

5. siRNA nach Anspruch 3, wobei die ersten beiden basengepaarten Nukleotide am 3'-Ende jedes Strangs modifiziert sind, wobei die modifizierten Nukleotide jeweils aus Nukleotiden mit einer modifizierten Internukleosidbindung ausgewählt aus Phosphorothioat-, Phosphorodithioat-, Boranophosphonoat- und Amidbindungen ausgewählt sind.

6. siRNA nach Anspruch 3, wobei die ersten beiden basengepaarten Nukleotide am 3'-Ende jedes Strangs 2'-Methoxyethylribonukleotidreste sind.

7. siRNA nach Anspruch 3, wobei die beiden Stränge über 19 Nukleotide komplementär zueinander sind.

8. siRNA nach Anspruch 1, wobei ein Ende der siRNA stumpf ist.

9. siRNA nach Anspruch 1, wobei beide Enden der siRNA stumpf sind.

10. siRNA nach Anspruch 1, wobei die beiden Stränge über 19 Nukleotide voll komplementär zueinander sind und wobei die siRNA stumpf ist.

11. siRNA nach Anspruch 1, wobei mindestens ein weiteres Nukleotid modifiziert ist.

12. siRNA nach Anspruch 1, welche 1 bis 6 Nukleotide umfasst, die mindestens an einem vom 5'-Ende oder vom 3'-Ende überhängen.

13. Pharmazeutische Zusammensetzung, umfassend die siRNA nach Anspruch 1 und einen pharmazeutisch unbedenklichen Träger.

14. siRNA nach Anspruch 1 zur Verwendung als Medikament.

15. siRNA nach Anspruch 1 zur Verwendung als Medikament, welches oral, topisch, parenteral, durch Inhalation oder als Spray oder rektal oder mittels eines perkutanen, subkutanen, intravaskulären, intramuskulären, intraperitonealen, intrathekalen oder Infusionsverfahrens verabreicht wird.

## Revendications

1. Petit acide ribonucléique interférent (siARN), ledit siARN comprenant deux brins d'ARN séparés qui sont complémentaires l'un de l'autre sur au moins 15 nucléotides, où chaque brin mesure 49 nucléotides ou moins, et où l'extrémité 3' d'au moins un brin comprend une modification au niveau du carbone 3', où la modification est :

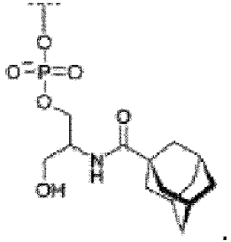

2. siARN selon la revendication 1, où les deux brins comprennent une modification au niveau du carbone 3', où la modification est :

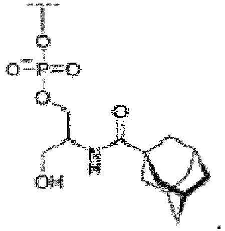

3. siARN selon la revendication 1, où chaque brin mesure 19 nucléotides.

4. siARN selon la revendication 3, où les deux premiers nucléotides d'appariement de base au niveau de l'extrémité 3' de chaque brin sont modifiés.

5. siARN selon la revendication 3, où les deux premiers nucléotides d'appariement de base au niveau de l'extrémité 3' sont modifiés, où chaque nucléotide modifié est choisi parmi les nucléotides présentant une liaison internucléosidique modifiée choisie parmi les liaisons phosphorothioate, phosphorodithioate, boranophosphonoate et amide.

6. siARN selon la revendication 3, où les nucléotides des deux premières paires de base au niveau de l'extrémité 3' de chaque brin sont des résidus de 2'-méthoxyéthylribonucléotides.

7. siARN selon la revendication 3, où les deux brins sont complémentaires l'un de l'autre sur au moins 19 nucléotides.

8. siARN selon la revendication 1, où l'une des extrémités du siARN est émoussée.

9. siARN selon la revendication 1, où les deux extrémités du siARN sont émoussées.

10. siARN selon la revendication 1, où les deux brins sont entièrement complémentaires l'un de l'autre sur 19 nucléotides et où l'extrémité du siARN est émoussée.

11. siARN selon la revendication 1, où au moins un nucléotide supplémentaire est modifié.

12. siARN selon la revendication 1, comprenant une corniche de 1 à 6 nucléotides sur au moins l'une des extrémités 5' et 3'.

13. Composition pharmaceutique comprenant le siARN selon la revendication 1, ainsi qu'un vecteur pharmaceutiquement acceptable.

14. siARN selon la revendication 1, pour utilisation en tant que médicament.

15. siARN selon la revendication 1, pour utilisation en tant que médicament administré par voie orale, topique, parentérale, par inhalation ou pulvérisation, ou rectale, ou par une technique percutanée, sous-cutanée, intravasculaire, intramusculaire, intrapéritonéale, intrathécale ou de perfusion.
